(19)

Europäisches
Patentamt
European
Patent Office
Office européen
des brevets

(11)  **EP 4 516 812 A1**

(12)  **EUROPEAN PATENT APPLICATION**
published in accordance with Art. 153(4) EPC

(43) Date of publication:
 **05.03.2025  Bulletin 2025/10**

(21) Application number: **23796871.4**

(22) Date of filing: **27.04.2023**

(51) International Patent Classification (IPC):
 *C07K 16/40* (2006.01)   *A61P 29/00* (2006.01)
 *A61K 39/00* (2006.01)

(52) Cooperative Patent Classification (CPC):
 **A61K 39/00; A61P 29/00; C07K 16/40**

(86) International application number:
 **PCT/KR2023/005828**

(87) International publication number:
 **WO 2023/211229 (02.11.2023 Gazette 2023/44)**

(84) Designated Contracting States:
 **AL AT BE BG CH CY CZ DE DK EE ES FI FR GB
 GR HR HU IE IS IT LI LT LU LV MC ME MK MT NL
 NO PL PT RO RS SE SI SK SM TR**
 Designated Extension States:
 **BA**
 Designated Validation States:
 **KH MA MD TN**

(30) Priority:  **27.04.2022  KR 20220052421**

(71) Applicant: **MirimGENE Co., Ltd.
 21999 Incheon (KR)**

(72) Inventors:
 • **JIN, Mirim
  Seoul 05649 (KR)**
 • **KANG, Byoung Cheol
  Seoul 01725 (KR)**
 • **CHOI, Yun Hui
  Incheon 21910 (KR)**

 • **JANG, Jinah
  Suwon-si Gyeonggi-do 16295 (KR)**
 • **YOON, Heekyeong
  Incheon 21110 (KR)**
 • **JO, Lee Seul
  Incheon 22009 (KR)**
 • **SEO, Rira
  Incheon 21982 (KR)**
 • **NGUYEN, Thuy Thuy Tram
  Incheon 21404 (KR)**

(74) Representative: **J A Kemp LLP
 80 Turnmill Street
 London EC1M 5QU (GB)**

Remarks:
 The complete document including Reference
 Table(s) and the Sequence Listing(s) can be
 downloaded from the EPO website

(54)  **ANTI-WARS1 ANTIBODY AND USE THEREOF**

(57)   The present invention relates to an anti-WARS1 antibody, and a pharmaceutical composition comprising same as an active ingredient. The anti-WARS1 antibody according to the present invention specifically binds to WARS1 and can inhibit the secretion of TNF-α induced by WARS1. In addition, the anti-WARS1 antibody according to the present invention can be effectively used to prevent or treat inflammatory diseases including sepsis, chronic obstructive pulmonary disease, inflammatory bowel disease, rheumatoid arthritis, atopic dermatitis, macrophage activating syndrome, and the like.

Figure 10

EP 4 516 812 A1

## Description

## Technical Field

The present invention relates to an antibody that specifically bind to a WARS 1 protein and a use thereof.

## Background Art

Aminoacyl-tRNA synthetase (ARS) is an enzyme that mediates the reaction of specifically binding amino acids to tRNA and plays a central role in protein production. Recently, it has been known that ARS is involved in various life phenomena in addition to protein production, such as apoptosis, angiogenesis, inflammatory response, and the like. Among ARSs, human tryptophanyl-tRNA synthetase 1 (WARS1) is an aminoacyl-tRNA synthetase that links tryptophan with the tRNA that recognizes tryptophan and there has recently been an increasing interest in the role of WARS 1 in a host defense mechanism against infection. The present inventors have previously reported that monocytes secrete intracellular WARS1 out of the cell without *de novo* synthesis within minutes after bacterial infection (Young Ha Ahn et al., Nature Microbiology (2016) 2:16191).

Secreted WARS1 functions as an endogenous ligand for TLR4 and TLR2 to activate macrophages, which induces the activation of innate immunity. In addition, WARS1 initiates the production of proinflammatory cytokines and chemokines including TNF-$\alpha$, IL-6, MIP-1$\alpha$, and IL-8 and induces the infiltration of neutrophils. Afterwards, the expression of the WARS 1 gene is induced by IFN-y to maintain continuous secretion of WARS1. Consistent with these findings, WARS1 was observed at high levels in the blood of sepsis patients.

Reflecting this, a strategy to develop anti-WARS1 antibodies for the treatment of infectious and/or inflammatory diseases caused by bacteria, viruses, and/or fungi may be considered. However, ARSs including WARS1 have many similarities in their protein structures, thus there are many cases in which antibodies obtained through immune reactions do not produce highly sensitive antibodies by displaying cross-reactivity with other ARSs, and the like.

Therefore, there is an urgent need for the development of antibodies that can effectively neutralize WARS1 by specifically binding only to WARS1 without cross-reacting with other ARSs.

## Detailed Description of Invention

## Technical Problem

The present inventors have made efforts to discover antibodies or a fragment thereof that specifically bind to WARS1 or a fragment thereof, which would significantly increase the level of expression in patients with infectious diseases, such as sepsis. As a result, the present invention was completed by producing a novel anti-WARS1 antibody that specifically binds to WARS1 and suppresses the inflammatory response in immune cells, and by identifying the pharmacological activity thereof.

## Solution to Problem

In order to achieve the above object, in one aspect of the present invention, there is provided anti-WARS1 antibody consisting of: a heavy chain variable region comprising H-CDR1 comprising the amino acid sequence of SEQ ID NO: 3, H-CDR2 comprising the amino acid sequence of SEQ ID NO: 5, and H-CDR3 comprising the amino acid sequence of SEQ ID NO: 7; and a light chain variable region comprising L-CDR1 comprising the amino acid sequence of SEQ ID NO: 12, L-CDR2 comprising the amino acid sequences of SEQ ID NO: 95, and L-CDR3 comprising the amino acid sequence of SEQ ID NO: 96, or a fragment thereof:

N terminus-Ala Asn Xaa1 Xaa2 His Arg Pro Ser-C terminus (SEQ ID NO: 95),
wherein Xaa1 is Ala, Val, Ile, Leu, Met, Phe, Tyr, Trp, Lys, Arg, His, Asp, Ser, Thr, Glu, Asn, Gln, Cys, Gly, or Pro,
Xaa2 is Ala, Val, Ile, Leu, Met, Phe, Tyr, Trp, Lys, Arg, His, Asp, Ser, Thr, Glu, Asn, Gln, Cys, Gly, or Pro,
when Xaa1 is Ser, Xaa2 is not His,
N terminus-Gly Ala Trp Asp Asp Ser Xaa3 Ser Ala Tyr Val-C terminus (SEQ ID NO: 96),
wherein Xaa3 is Ala, Val, Ile, Leu, Met, Phe, Tyr, Trp, Lys, Arg, His, Asp, Ser, Thr, Glu, Asn, Gln, Cys, Gly, or Pro.

In another aspect of the present invention, there is provided a polynucleotide encoding the antibody or a fragment thereof, an expression vector comprising the polynucleotide, and a transformed cell into which the expression vector has been introduced.

In another aspect of the present invention, there is provided a method for producing an antibody or a fragment thereof, comprising culturing the transformed cell and obtaining the antibody or a fragment thereof.

In another aspect of the present invention, there is provided a pharmaceutical composition for preventing or treating an inflammatory disease comprising the antibody or a fragment thereof as an active ingredient.

In another aspect of the present invention, there is provided a use for preventing or treating an inflammatory disease of the antibody or a fragment thereof.

In another aspect of the present invention, there is provided a method of preventing or treating an inflammatory disease comprising administering the antibody or a fragment thereof to an individual.

**Effects of Invention**

The anti-WARS 1 antibody according to the present invention was specifically bonded to WARS1 and suppressed the secretion of TNF-$\alpha$ induced by WARS1 in immune cells. In addition, as a result of administering the anti-WARS1 antibody of the present invention to a mouse model of severe sepsis, a mouse model of chronic obstructive pulmonary disease, a mouse model of inflammatory bowel disease, a mouse model of rheumatoid arthritis, a mouse model of atopic dermatitis, and a model of macrophage activation syndrome, the mouse models of each disease showed an effect of improvement in all diseases. Therefore, the anti-WARS1 antibody according to the present invention can be effectively used for the treatment of inflammatory diseases.

**Brief Description of Drawings**

Figure 1 is a schematic diagram of a vector for expressing the heavy chain of the anti-WARS1 antibody.

Figure 2 is a schematic diagram of a vector for expressing the light chain of the anti-WARS1 antibody.

Figures 3a and 3b are diagrams showing the result of identifying the anti-WARS1 antibody using SDS-PAGE.

Figures 4a and 4b are graphs showing the result of identifying the binding affinity of the anti-WARS 1 and WARS 1 (derived from human, marmoset (NHP), mouse, rat, or pig) using a direct ELISA.

Figure 5 is a graph showing the result of identifying the binding affinity of the anti-WARS1 antibody and WARS1 (derived from human, marmoset (NHP), mouse, rat, or pig) through a direct ELISA measurement method as Kd (dissociation constant).

Figures 6a to 6c are graphs showing the result of identifying the anti-WARS 1 antibody and WARS1 (derived from human, marmoset (NHP), mouse, rat, or pig) through a sandwich ELISA method.

Figure 7 is a diagram showing the result of identifying the binding specificity of the anti-WARS1 antibody to a recombinant WARS1 protein and secreted WARS1 using western blot. hu: human, Mar: marmoset (NHP), P: pig, R: rat, M: mouse.

Figure 8 shows the result of epitope mapping using peptide production and antibody binding specificity of the human WARS1 sequence.

Figures 9a to 9c are diagrams showing the production and result of the alanine (Ala) mutations of human WARS1 recombinant protein for epitope mapping.

Figure 10 is a graph showing the result of measuring the concentration of TNF-$\alpha$ in the medium after treating the recombinant WARS1 protein and the IgG or anti-WARS1 antibody in a mouse macrophage cell line (J774.1A).

Figure 11 is a diagram showing the experimental schedule for identifying the anti-inflammatory effect of the anti-WARS 1 antibody using a sepsis mouse model.

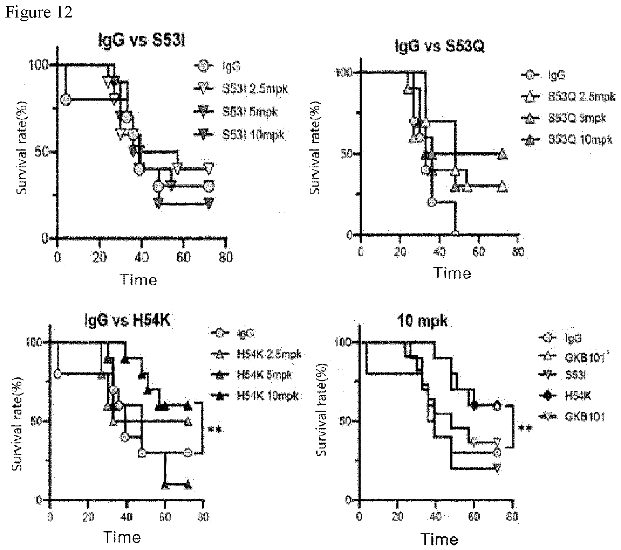

Figure 12 is a graph showing the result of identifying the survival rate of mice after administering the IgG1 or anti-WARS1 antibody to a severe sepsis mouse model.

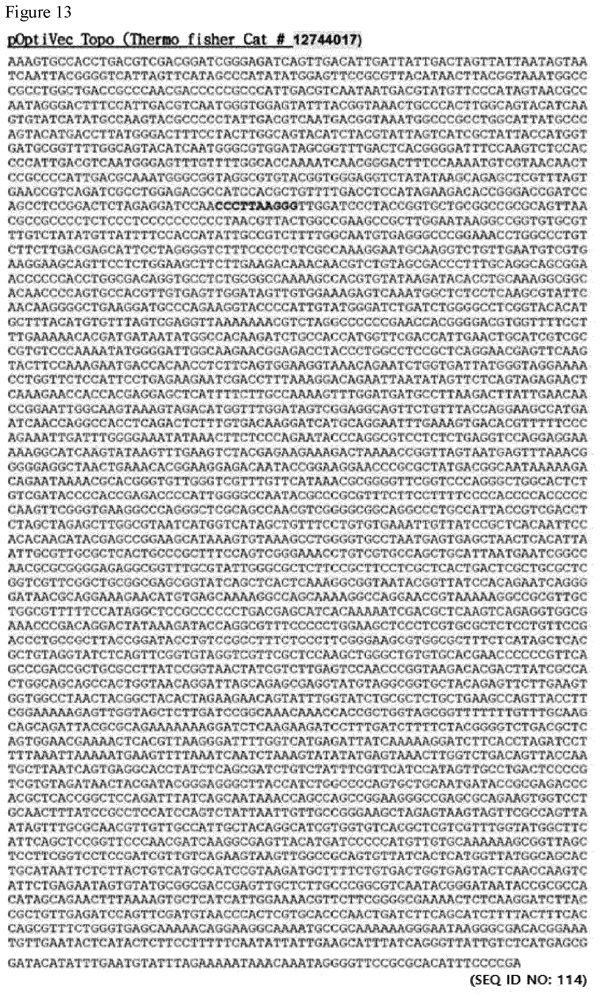

Figure 13 is a diagram showing the nucleotide sequence of a pOptiVec Topo vector.

Figure 14 is a graph showing the result of identifying the anti-inflammatory activity of the anti-WARS1 antibody (GKB101, H54K) in human lung fibroblasts (HLF). *p<0.05, **p<0.01, ***p<0.001 vs. PBS.

Figure 15 is a diagram showing the experimental schedule for verifying the efficacy of the anti-WARS 1 antibody (H54K) in suppressing respiratory damage using a COPD mouse model.

Figures 16a and 16b are diagrams showing the result of identifying the immune cells infiltrated in the lung tissue, collagen deposition, and mucus secretion in the airway after administering Normal, Isotype IgG1, or the anti-WARS 1 antibody (H54K) in a COPD mouse model through H&E staining, Massone trichrome staining, and Periodic acid-Schiff-Alcian Blue (PAS-AB) staining, respectively.

Figures 17a and 17b are graphs showing the result of identifying the expressions of inflammatory cytokines and chemokines in alveolar lavage fluid (17a) and lung tissue (17b) after administering PBS (CON), Isotype IgG1, or the anti-WARS1 antibody (H54K) in a COPD mouse model. *p<0.05, **p<0.001.

Figure 17c is a graph showing the result of identifying the expressions of cough-inducing genes in lung tissue through q-PCR after administering PBS (CON), Isotype IgG1, or the anti-WARS1 antibody (H54K) in a COPD mouse model.

Figures 18a and 18b are graphs showing the result of analyzing the immune cells in lung tissue (18b) and alveolar lavage fluid (18a) after administering PBS (CON), Isotype IgG1, or the anti-WARS 1 antibody (H54K) in a COPD mouse model.

Figure 19 is a graph showing the result of analyzing the immune cells in mediastinal lymph nodes through flow cytometry after administering PBS (CON), Isotype IgG1, or the anti-WARS 1 antibody (H54K) in a COPD mouse model.

Figure 20 is a graph showing the result of analyzing the immune cells in blood through flow cytometry after administering PBS (CON), Isotype IgG1, or the anti-WARS1 antibody (H54K) in a COPD mouse model.

Figure 21 is a diagram showing the experimental schedule for identifying the effect of the anti-WARS1 antibody in improving inflammatory bowel disease using a DSS (dextran sulfate sodium-induced colitis) mouse model.

Figure 22a is a diagram showing the criteria for determining the clinical index for assigning the disease activity index (DAI) to evaluate the effect of the anti-WARS 1 antibody (GKB101, H54K) in improving an inflammatory bowel disease using a DSS mouse model.

Figure 22b is a graph showing the result of evaluating the disease activity index of each administration group after administering a DSS mouse model with saline solution, Isotype IgG1, or the anti-WARS1 antibody (GKB101, H54K). At this time, control is the untreated group.

Figure 23 is a graph showing the result of evaluating the rectal bleeding score of each administration group after administering a DSS mouse model with saline solution, Isotype IgG1, or the anti-WARS1 antibody (GKB101, H54K). At this time, control is the untreated group.

Figure 24 is a graph showing the result of evaluating the colon stool consistency score of each administration group after administering a DSS mouse model with saline solution, Isotype IgG1, or the anti-WARS1 antibody (GKB101, H54K). At this time, control is the untreated group.

Figure 25 is a graph showing the result of measuring the intestinal length of each administration group after administering a DSS mouse model with saline solution, Isotype IgG1, or the anti-WARS1 antibody (GKB101, H54K). At this time, control is the untreated group.

Figure 26 is a graph showing the result of measuring the spleen weight of each administration group after administering a DSS mouse model with saline solution, Isotype IgG1, or the anti-WARS1 antibody (GKB101, H54K). At this time, control is the untreated group.

Figure 27 is a graph showing the result of measuring the immune factors in each region of the intestinal tissue after administering a DSS mouse model with saline solution, Isotype IgG1, or the anti-WARS1 antibody (GKB101, H54K, *p<0.05, **p<0.001, ****p<0.0001 vs. saline solution-administered group.

Figure 28 is a graph showing the result of identifying the gene expression of immune factors in each region of the intestinal tissue using qPCR after administering a DSS mouse model with saline solution, Isotype IgG1, or the anti-WARS1 antibody (GKB101, H54K). *p<0.05, **p<0.01, ***p<0.001 vs. saline solution-administered group.

Figure 29 is a diagram showing the result of identifying the degree of crypt damage in colon tissue of each administration group through H&E staining after administering a DSS mouse model with saline solution, Isotype IgG1, or the anti-WARS1 antibody (GKB101, H54K).

Figure 30 is a graph showing the result of identifying the anti-inflammatory activity of the anti-WARS1 antibody (H54K) in osteoclasts differentiated from J774A.1 cells, human fibroblast-like synoviocytes (FLS), and RAW264.7 cells. *p<0.05, **p<0.01, ***p<0.001.

Figure 31 is a diagram showing the experimental schedule for verifying the effect of the anti-WARS1 antibody in improving rheumatoid arthritis in a CIA (collagen-induced arthritis) mouse model.

Figure 32a is a diagram showing the standards of the clinical index for evaluating the clinical arthritis index by observing the paw of each administration group after administering a CIA mouse model with Isotype IgG1 or the anti-WARS 1 antibody (GKB101, H54K).

Figure 32b is a graph showing the clinical arthritis index and the result of analyzing the clinical arthritis index, and the AUC thereof by observing the paw of each administration group after administering a CIA mouse model with Isotype IgG1 or the anti-WARS 1 antibody (GKB101, H54K). *p<0.05, **p<0.01, vs. H54K antibody-administered group.

Figures 33a to 33d are graphs showing the result of identifying the expression of inflammatory factors in the plasma and paw joints of each administration group after administering a CIA mouse model with Isotype IgG1 or the anti-WARS 1 antibody (GKB101, H54K). *p<0.05, **p<0.01, ***p<0.001, ****p<0.0001 vs. anti-WARS1 antibody-administered group.

Figures 34a to 34c are graphs and diagrams showing the result of identifying the anti-inflammatory activity and the effect of suppressing cell migration and proliferation of the anti-WARS1 antibody (GKB101, H54K) in adult human dermal fibroblasts (HDFa) or human keratinocytes treated with *Staphylococcus aureus.* *p<0.05, ***p<0.001, ****p<0.0001 vs. PBS.

Figure 35 is a diagram showing the experimental schedule for identifying the effect of the anti-WARS 1 antibody in improving atopic dermatitis in an atopic mouse model.

Figure 36 is a diagram showing the result of evaluating the clinical index of each administration group after administering an atopic mouse model with Isotype IgG1, dexamethasone, or the anti-WARS 1 antibody (GKB101, H54K).

Figure 37 is a graph showing the result of identifying the improvement of the tight junction in a human colon cancer cell line (Caco-2) by the anti-WARS1 antibody (GKB101, H54K). *p<0.05, **p<0.01, ***p<0.001 vs. PBS.

Figure 38 is a diagram showing an experimental schedule to identify the anti-inflammatory effect of the anti-WARS1 antibody (GKB101, H54K) in the tissue in a macrophage activation syndrome model using CpG.

Figure 39 is a diagram showing the result of evaluating Ferritin, which is one of the clinical indices, after administering the anti-WARS1 antibody (GKB101, H54K) in a macrophage activation syndrome model using CpG.

Figures 40a and 40b are graphs showing the result of measuring the expression of WARS1 and inflammatory cytokines in liver tissue of mice after administering the anti-WARS 1 antibody (GKB101, H54K) in a macrophage activation syndrome model using CpG.

Figures 41a and 41b are graphs showing the result of analyzing the distribution and infiltration of immune cells in the spleen and liver tissues of mice after administering the anti-WARS1 antibody (GKB101, H54K) in a macrophage activation syndrome model using CpG.

**Best Mode for Carrying out the Invention**

**Anti-WARS1 antibody**

The present invention provides an antibody that specifically binds to WARS1 or a fragment thereof.

At this time, the antibody that specifically binds to the WARS1 or a fragment thereof may be an antibody or a fragment thereof that specifically binds to R20, R24, K27, I36, I43, and K47. The WAR1 may be a human WARS1 comprising the amino acid sequence of SEQ ID NO: 94.

As used herein, the term "antibody" refers to an immunoglobulin molecule that immunologically reacts with a specific antigen, and a protein molecule that specifically recognizes the antigen. The heavy chain and the light chain of the immunoglobulins may include a constant region (C) and a variable region (V). The light chain and heavy chain variable regions of immunoglobulins comprise three variable regions called complementarily determining regions (CDRs) and four framework regions (FRs). The CDR is an antigen-binding site that mainly binds to the epitope of the antigen.

As used herein, the term "WARS" refers to tryptophanyl-tRNA synthetase, and is also known as tryptophan-tRNA ligase, TrpRS, WRS, and so on. WARS is an enzyme that mediates the aminoacylation reaction between tryptophan as the amino acid and tRNA. WARS is encoded by the WARS gene in humans and the amino acid sequence of protein, and the nucleotide sequence of mRNA are disclosed in Genbank accession number NP_004175.2 (protein), GenBank accession number NM_004184.3 (mRNA nucleotide sequence), and the like. WARS has two isoforms: a cytoplasmic form (WARS 1 or tryptophanyl-tRNA synthetase 1, cytoplasmic) and a mitochondrial form (WARS2 or tryptophanyl-tRNA synthetase 2, mitochondrial). WARS in the present invention may be WARS1. The WARS1 is not limited, but is preferably human WARS1 comprising the amino acid sequence of SEQ ID NO: 94.

The anti-WARS 1 antibody of the present invention may be an antibody or a fragment thereof that specifically binds to the WARS1. In addition, the fragment of the antibody of the present invention may be Fab, F(ab')$_2$, Fd, sdFv, Fv, dAb, scFv, sdAb, tetramer, and so on, and may be included in any form as long as it includes an antigen-binding site that can specifically bind to WARS1.

In one aspect of the present invention, there is provided an anti-WARS1 antibody consisting of: a heavy chain variable region comprising H-CDR1 comprising the amino acid sequence of SEQ ID NO: 3, H-CDR2 comprising the amino acid sequence of SEQ ID NO: 5, and H-CDR3 comprising the amino acid sequence of SEQ ID NO: 7; and a light chain variable region comprising L-CDR1 comprising the amino acid sequence of SEQ ID NO: 12, L-CDR2 comprising the amino acid sequences of SEQ ID NO: 95, and L-CDR3 comprising the amino acid sequence of SEQ ID NO: 96, or a fragment thereof:

N terminus-Ala Asn Xaa1 Xaa2 His Arg Pro Ser-C terminus (SEQ ID NO: 95),
wherein Xaa1 is Ala, Val, Ile, Leu, Met, Phe, Tyr, Trp, Lys, Arg, His, Asp, Ser, Thr, Glu, Asn, Gln, Cys, Gly, or Pro,
Xaa2 is Ala, Val, Ile, Leu, Met, Phe, Tyr, Trp, Lys, Arg, His, Asp, Ser, Thr, Glu, Asn, Gln, Cys, Gly, or Pro,
when Xaa1 is Ser, Xaa2 is not His,
N terminus-Gly Ala Trp Asp Asp Ser Xaa3 Ser Ala Tyr Val-C terminus (SEQ ID NO: 96),
wherein Xaa3 is Ala, Val, Ile, Leu, Met, Phe, Tyr, Trp, Lys, Arg, His, Asp, Ser, Thr, Glu, Asn, Gln, Cys, Gly, or Pro.

Specifically, in the L-CDR2 of the light chain variable region, Xaa1 may be any one selected from the group consisting of Ile, Leu, Thr, Val, and Gln, and at this time, Xaa2 may be His or Leu. In addition, when Xaa1 is Ser, Xaa2 may be one selected from the group consisting of Ile, Leu, Lys, Glu, and Phe.

Specifically, in the L-CDR3 of the light chain variable region, Xaa3 may be Leu or Asn.

More specifically, the L-CDR1 of the light chain variable region may include the amino acid sequence of SEQ ID NO: 12, the L-CDR2 may include the amino acid sequence of any one selected from the group selected of SEQ ID NO: 20, 22, 24, 26, 28, 30, 32, 34, 36, 38, 40, 42, and 44, and the L-CDR3 may include the amino acid sequence of SEQ ID NO: 16 or SEQ ID NO: 46.

Preferably, the heavy chain variable region may include the amino acid sequence of SEQ ID NO: 9. In addition, the light chain variable region may include the amino acid sequence of any one selected from the group consisting of SEQ ID NO: 21, 23, 25, 27, 29, 31, 33, 35, 37, 39, 41, 43, 45, 47, 48, and 49.

In one embodiment of the present invention, the antibody or a fragment thereof may include a heavy chain variable region comprising the amino acid sequence of SEQ ID NO: 9 and a light chain variable region comprising SEQ ID NO: 21.

In one embodiment, the antibody or a fragment thereof may include a heavy chain variable region comprising the amino acid sequence of SEQ ID NO: 9 and a light chain variable region comprising SEQ ID NO: 23.

In one embodiment, the antibody or a fragment thereof may include a heavy chain variable region comprising the amino acid sequence of SEQ ID NO: 9 and a light chain variable region comprising SEQ ID NO: 25.

In one embodiment, the antibody or a fragment thereof may include a heavy chain variable region comprising the amino acid sequence of SEQ ID NO: 9 and a light chain variable region comprising SEQ ID NO: 27.

In one embodiment, the antibody or a fragment thereof may include a heavy chain variable region comprising the amino acid sequence of SEQ ID NO: 9 and a light chain variable region comprising SEQ ID NO: 29.

In one embodiment, the antibody or a fragment thereof may include a heavy chain variable region comprising the amino acid sequence of SEQ ID NO: 9 and a light chain variable region comprising SEQ ID NO: 31.

In one embodiment, the antibody or a fragment thereof may include a heavy chain variable region comprising the amino acid sequence of SEQ ID NO: 9 and a light chain variable region comprising SEQ ID NO: 33.

In one embodiment, the antibody or a fragment thereof may include a heavy chain variable region comprising the amino acid sequence of SEQ ID NO: 9 and a light chain variable region comprising SEQ ID NO: 35.

In one embodiment, the antibody or a fragment thereof may include a heavy chain variable region comprising the amino acid sequence of SEQ ID NO: 9 and a light chain variable region comprising SEQ ID NO: 37.

In one embodiment, the antibody or a fragment thereof may include a heavy chain variable region comprising the amino acid sequence of SEQ ID NO: 9 and a light chain variable region comprising SEQ ID NO: 39.

In one embodiment, the antibody or a fragment thereof may include a heavy chain variable region comprising the amino acid sequence of SEQ ID NO: 9 and a light chain variable region comprising SEQ ID NO: 41.

In one embodiment, the antibody or a fragment thereof may include a heavy chain variable region comprising the amino acid sequence of SEQ ID NO: 9 and a light chain variable region comprising SEQ ID NO: 43.

In one embodiment, the antibody or a fragment thereof may include a heavy chain variable region comprising the amino acid sequence of SEQ ID NO: 9 and a light chain variable region comprising SEQ ID NO: 45.

In one embodiment, the antibody or a fragment thereof may include a heavy chain variable region comprising the amino acid sequence of SEQ ID NO: 9 and a light chain variable region comprising SEQ ID NO: 47.

In one embodiment, the antibody or a fragment thereof may include a heavy chain variable region comprising the amino acid sequence of SEQ ID NO: 9 and a light chain variable region comprising SEQ ID NO: 48.

In one embodiment, the antibody or a fragment thereof may include a heavy chain variable region comprising the amino acid sequence of SEQ ID NO: 9 and a light chain variable region comprising SEQ ID NO: 49.

The antibody or a fragment thereof of the present invention may include an immunoglobulin Fc region. At this time, the Fc region of the immunoglobulin refers to a protein that includes a heavy chain constant region 2 (CH2) and a heavy chain constant region 3 (CH3), and does not include heavy chain variable region and light chain variable region, and a light chain constant region (CL) of the immunoglobulin. The immunoglobulin Fc region may be derived from IgG, IgA, IgE, IgD, or IgM.

In addition, the Fc region of the immunoglobulin may be not only a wild-type Fc region, but also a Fc region variant. In addition, as used herein, "Fc region variant" may have a glycosylation pattern different to that of the wild-type Fc region, or an increased or decreased sugar chain compared to the wild-type Fc region, or deglycosylated form. In addition, an aglycosylated Fc region may be included. The Fc region or variant may have an adjusted number of sialic acids, fucosylation, and glycosylation through culture conditions or genetic manipulation of the host.

In addition, the sugar chain of the Fc region of the immunoglobulin may be modified by conventional methods, such as chemical methods, enzymatic methods, and genetic engineering methods using microorganisms. In addition, the Fc region variant may be a mixture of the Fc regions of immunoglobulins IgG, IgA, IgE, IgD, or IgM. In addition, the Fc region variant may have a form in which some of the amino acids of the Fc region are substituted with other amino acids.

In one embodiment of the present invention, the Fc region may include the amino acid sequence of SEQ ID NO: 51.

The antibody of the present invention may include a fusion protein comprising a light chain variable region (VL) and a light chain constant region (CL) of the anti-WARS 1 antibody and a fusion protein comprising a heavy chain variable region (VH), a heavy chain constant region 1 (CH1), and the Fc region of the anti-WARS 1 antibody.

Specifically, the anti-WARS 1 antibody may include (I) and (II).

N'-X'[linker]o-Fc region fragment or variant-C' thereof (I); and

N'-X"-C'            (II)

At this time, in the structural formulas (I) and (II),

N' is the N terminus,
C" is the C terminus,
X is an antigen-binding site of the anti-WARS1 antibody or a fragment thereof and consists of X' and X",
X' is an antigen-binding site of a heavy chain of the anti-WARS1 antibody or a fragment thereof and comprises a variable region (VH) and a CH1 region,
X" is an antigen-binding site of a light chain of the anti-WARS 1 antibody or a fragment thereof and comprises a variable region (VL) and a constant region (CL),
the linker is a peptide linker, and
o is 0 or 1.

At this time, the anti-WARS 1 antibody or a fragment thereof, the antigen-binding site, the variable region, and the constant region are as described above.

The peptide linker may consist of 1 to 50 consecutive amino acids or 3 to 30 consecutive amino acids. In one embodiment, the peptide linker may consist of 23 amino acids. In addition, the peptide linker may include at least one cysteine. Specifically, it may include one, two, or three cysteines. In addition, the peptide linker may be derived from the hinge of immunoglobulin. For example, the hinge may be selected from the hinge region of various IgG1 subtype antibodies. In addition, the hinge may be in the form in which some amino acids of the hinge region derived from immunoglobulin may be substituted with other amino acids, or of a sequence in which some amino acids are added. In one embodiment, the peptide linker may be a peptide linker consisting of the amino acid sequence of SEQ ID NO: 50.

**Polynucleotide for encoding the anti-WARS1 antibody**

In another aspect of the present invention, there is provided a polynucleotide for encoding the anti-WARS1 antibody or a fragment thereof. The anti-WARS1 antibody or a fragment thereof is the same as described above.

The polynucleotide may include a nucleotide sequence of SEQ ID NO: 52, which encodes the heavy chain region of the anti-WARS1 antibody or a fragment thereof. The polynucleotide may include any one nucleotide sequence selected from the group consisting of SEQ ID NO: 54 to SEQ ID NO: 69, which encodes the light chain region of the anti-WARS 1 antibody or a fragment thereof.

In addition, if the polynucleotide encodes the same polypeptides, one or more nucleotides may be mutated by substitution, deletion, insertion, or a combination thereof. When preparing a polynucleotide sequence by chemical synthesis, synthesis methods commonly known in the art, for example, methods disclosed in literatures (Engels and Uhlmann, Angew Chem IntEd Engl., 37:73-127, 1988), may be used, which may include triester, phosphite, phosphoramidite and H-phosphate methods, PCR and other autoprimer methods, and oligonucleotide synthesis methods on solid supports.

According to one embodiment, the polynucleotide may include a nucleotide sequence of SEQ ID NO: 52, and SEQ ID NO: 54 to SEQ ID NO: 69, and a nucleotide sequence having at least about 70%, at least about 75%, at least about 80%, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, at least about 99%, or at least about 100%.

The polynucleotide may additionally include a signal sequence or a leader sequence. As used herein, the term "signal sequence" refers to a nucleic acid encoding the signal peptide that directs the secretion of the target protein. The signal peptide is cleaved after translation of the host cell. Specifically, the signal sequence of the present invention is a nucleotide encoding an amino acid sequence that initiates the movement of a protein across the ER (endoplasmic reticulum) membrane.

The characteristics of signal sequences are well known in the art, and typically contain 16 to 30 amino acid residues, but may contain more or fewer amino acid residues. A typical signal peptide consists of three regions: a basic N-terminal region, a central hydrophobic region, and a more polar C-terminal region. The central hydrophobic region contains 4 to 12 hydrophobic residues that anchor the signal sequence throughout the membrane lipid bilayer while the immature polypeptide moves.

After initiation, the signal sequence is cleaved within the lumen of the ER by cellular enzymes commonly known as signal peptidases. At this time, the signal sequence may be tPa (tissue Plasminogen Activation), HSV gDs (signal sequence of Herpes simplex virus glycoprotein D), IgG signal sequence, or a growth hormone secretion signal sequence. Preferably, a secretion signal sequence used in higher eukaryotic cells including mammals may be used. The signal sequences useful in the present invention include antibody light chain signal sequences, for example, antibody 14.18 (Gillies et al., J. Immunol. Meth 1989, 125:191-202), antibody heavy chain signal sequences, for example, MPOC141 antibody heavy chain signal sequences (Sakano et al., Nature, 1980. 286: 676-683), and other signal sequences known in the art (e.g., Watson et al., Nucleic Acid Research, 1984. 12:5145-5164). In one embodiment, the signal sequence may include the amino acid sequence of SEQ ID NO: 1.

### Vector loaded with a polynucleotide

In another aspect of the present invention, there is provided a vector loaded with a polynucleotide encoding the anti-WARS1 antibody or a fragment thereof. At this time, the polynucleotide may include a heavy chain region comprising the nucleotide sequence of SEQ ID NO: 52 and any one nucleotide sequence selected from the group consisting of SEQ ID NO: 54 to SEQ ID NO: 69.

As used herein, a "vector" may be introduced into a host cell to be recombined and inserted into the host cell genome. Alternatively, the vector may be understood as a nucleic acid vehicle containing a nucleotide sequence capable of spontaneous replication as an episome. The vector includes linear nucleic acids, plasmids, phagemids, cosmids, RNA vectors, viral vectors, mini-chromosomes, and analogs thereof. Examples of virus vectors include retroviruses, adenoviruses, and adeno-associated viruses, but are not limited thereto.

Specifically, the vector may be plasmid DNA, phage DNA, and the like, and may be commercially developed plasmids (pUC18, pBAD, pIDTSAMRT-AMP, etc.), *E. coli*-derived plasmids (pYG601BR322, pBR325, pUC118, pUC119, etc.), Bacillus subtilis-derived plasmids (pUB110, pTP5, etc.), yeast-derived plasmids (Yep13, Yep24, YCp50, etc.), phage DNA (Charon4A, Charon21A, EMBL3, EMBL4, λgt10, λgt11, λZAP, etc.), animal virus vectors (retroviruses, adenoviruses, vaccinia viruses, etc.), insect virus vectors (baculoviruses, etc.), and the like. The expression level and modification of the protein of the vector may vary depending on the host cell, thus selecting and using the host cell most suitable for the purpose would be preferable.

In addition, the plasmid may include a selection marker, such as an antibiotic resistance gene, and host cells maintaining the plasmid may be cultured under selective conditions.

As used herein, the term "gene expression" or "expression" of the target protein is understood to refer to the transcription of a DNA sequence, the translation of an mRNA transcript, and secretion of an antibody product or a fragment thereof. A useful expression may be RcCMV (Invitrogen, Carlsbad) or a variant thereof. The expression vector may include a human CMV (cytomegalovirus) promoter for promoting continuous transcription of the target gene in mammalian cells and a bovine growth hormone polyadenylation signal sequence for increasing the steady-state level of RNA after transcription.

### Transformed cells

In another aspect of the present invention, there is provided a transformed cell into which an expression vector loaded with a polynucleotide encoding the anti-WARS 1 antibody or a fragment thereof has been introduced. At this time, the anti-WARS 1 antibody or a fragment thereof is as described above.

As used herein, the term "transformed cell" refers to prokaryotic cells and eukaryotic cells into which a recombinant expression vector can be introduced. The transformed cell may be produced by introducing a vector into a host cell and transforming it. In addition, the antibody or a fragment thereof of the present invention may be produced by expressing a polynucleotide including the vector.

The transformation may be performed by various methods. There is no particular limitation thereto as long as the antibody of the present invention can be produced. Specifically, the transformation method may be a $CaCl_2$ precipitation method, a Hanahan method that increases efficiency by using a reducing substance called DMSO (dimethyl sulfoxide) in the $CaCl_2$ precipitation method, an electroporation method, a calcium phosphate precipitation method, a protoplast fusion method, a stirring method using silicon carbide fiber, an agrobacteria-mediated transformation method, a transformation method using PEG, dextran sulfate, lipofectamine, and a drying/suppression-mediated transformation method, and the like. In addition, the target may be delivered into the cell using virus particles using infection as the vehicle. In addition, the vector may be introduced into the host cell by gene bombardment, and the like.

In addition, the host cell used in producing the transformed cell is also not particularly limited as long as it can produce the antibody of the present invention. Specifically, the host cell may include cells of a prokaryotic, eukaryotic, mammalian, plant, insect, fungal, or cellular origin, but it not limited thereto. As an example of the prokaryotic cell, *E. coli* may be used. In addition, as an example of the eukaryotic cell, yeast may be used. In addition, as mammalian animal cells, CHO cells, F2N cells, COS cells, BHK cells, Bowes melanoma cells, HeLa cells, 911 cells, AT1080 cells, A549 cells, SP2/0 cells, human lymphoblastoids, NSO cells, HT-1080 cells, PERC.6 cells, HEK 293 cells, or HEK293T cells may be used, but are not limited thereto, and any cells that can be used as mammalian animal host cells known to those skilled in the art may be used.

In addition, to optimize the characteristics of the antibody as a therapeutic agent or for another purpose, the glycosylation-related genes of the host cell may be manipulated using a method known to those skilled in the art to adjust the sugar chain pattern (for example, sialic acid, fucosylation, glycosylation) of the antibody.

**Method for producing the anti-WARS1 antibody or a fragment thereof**

In another aspect of the present invention, there is provided a method for producing the anti-WARS 1 antibody or a fragment thereof.

The method for production may include i) culturing the transformed cells; and ii) obtaining the anti-WARS 1 antibody or a fragment thereof.

As used herein, the term "culture" refers to a method of growing microorganisms under environment conditions that are reasonably controlled in an artificial manner.

The method of culturing the transformed cells may be performed using a commonly known method in the art. Specifically, the culture is not particularly limited as long as the antibody of the present invention may be expressed for production. Specifically, the culture may be continuously cultured in a batch process or fed batch or repeated fed batch process.

In addition, the step of obtaining the antibody from the culture may be performed by methods known in the art. Specifically, the obtaining method is not particularly limited as long as the produced antibody or a fragment thereof of the present invention may be obtained. Preferably, the obtaining method may be centrifugation, filtration, extraction, spraying, drying, evaporation, precipitation, crystallization, electrophoresis, fractional dissolution (for example, ammonium sulfate precipitation), chromatography (for example, ion exchange, affinity, hydrophobicity, and size exclusion), and the like.

**A pharmaceutical composition comprising the anti-WAR1 antibody or a fragment thereof**

In another aspect of the present invention, there is provided a pharmaceutical composition for preventing or treating an inflammatory disease comprising the anti-WARS1 antibody or a fragment thereof as an active ingredient. Herein, the anti-WARS 1 antibody or a fragment thereof is as described above.

As used herein, the term "inflammation" includes a series of processes of suppressing cell damage, removing damaged tissue and necrotic cells, and regenerating tissue by involving immune cells, blood vessels and inflammatory mediators as a response to protect the living body against harmful factors. "Inflammatory disease" is a general term for diseases whose main lesion is inflammation.

The inflammatory disease may be one selected from the group consisting of peritonitis, osteomyelitis, cellulitis, meningitis, encephalitis, pancreatitis, trauma-related shock, cystic fibrosis, stroke, Lyme disease, polyarteritis nodosa, hypersensitivity angiitis, Lou Gehrig's granulomatosis, articular cell arteritis, bursitis, tenosynovitis, epicondylitis (Tennis elbow), Henoch-Schonlein purpura, multicentric reticulohistiocytoma, surcoilosis, hemochromatosis, sickle cell disease and other hemoglobinpathies, hyperlipoproteineimia, hypogammaglobulinemia, familial Mediterranean fever, relapsing fever, sepsis, septic shock, multi-organ dysfunction syndrome, broncho-pulmonary dysplasia, inflammatory bowel disease, chronic obstructive pulmonary disease (COPD), macrophage activation syndrome (MAS), chronic systemic lupus erythematosus, scleroderma, atopic dermatitis, psoriasis, anaphylaxis, dermatitis, diabetic retinopathy, retinitis, macular degeneration, uveitis, conjunctivitis, arthritis, rheumatoid arthritis, ankylosing spondylitis, osteoarthritis, osteoporosis, allergy, diabetes, diabetic nephropathy, pyelonephritis, nephritis, Sjögren's syndrome, autoimmune pancreatitis, periodontal disease, asthma, graft versus host disease, chronic pelvic inflammatory disease, endometritis, rhinitis, transplant rejection, and chronic prostatitis.

The preferred dosage of the pharmaceutical composition varies depending on the state and weight of the patient, degree of disease, drug form, administration route and period, but may be appropriately chosen by those skilled in the art. As long as the antibody or a fragment thereof in the pharmaceutical composition for preventing or treating an inflammatory disease of the present invention may exhibit an anti-inflammatory activity, or in particular, an effect of treating an inflammatory disease, any amount (effective amount) may be included based on the use, formulation, purpose of compounding, and the like, and the common effective amount shall be determined within a range of 0.001 wt% to 20.0 wt% based on the total weight of the composition. Herein, an "effective amount" refers to the amount of active ingredient that may lead to an effect of improving or treating the condition of a disease, especially, that may induce an effect of improving or treating the condition of a disease. Such effective amount may be determined experimentally within the scope of the ordinary ability of those skilled in the art.

As used herein, the term "treatment" may be used to include both a therapeutic treatment and a preventative treatment, and includes all applications or any form of administration to treat diseases in mammals including humans. In addition, the term includes inhibiting or slowing the progression of a disease; partially or completely alleviating a disease by recovering or repairing damaged or missing function; or stimulating inefficient processes; or alleviating severe diseases. Herein, "prevention" may be used to refer to alleviating or reducing the pathological condition or disease of an individual.

Pharmacokinetic parameters, such as bioavailability, and underlying parameters, such as the clearance rate may also affect efficacy. Therefore, "enhanced efficacy" (for example, the improvement of efficacy) may cause enhanced pharmacokinetic parameters and enhanced efficacy, and may be measured by comparing the parameters, such as the clearance rate in test animals or human subjects, and the treatment and improvement of a disease.

Meanwhile, the pharmaceutical composition of the present invention is administered in a "therapeutically effective amount."

As used herein, the term "administration" refers to introducing a specific substance to an individual using an appropriate method, and the administration route of the composition may involve any standard route as long as it may reach the target tissue. It may be administered intraperitoneally, intravenously, intramuscularly, subcutaneously, intradermally, orally, topically, intranasally, intrapulmonaryly, or rectally, but is not limited thereto.

As used herein, the term "therapeutically effective amount" or "pharmaceutically effective amount" refers to an amount of a compound or a composition effective in preventing or treating a target disease and refers to an amount that is sufficient in treating a disease at a reasonable benefit/risk ratio applicable to medical treatment and would not cause side effects. The level of an effective amount may be determined by factors including the health status of the patient, type and severity of the disease, activity of the drug, sensitivity to the drug, method of administration, time of administration, administration route and excretion rate, duration of treatment, combined or simultaneously used drugs, and other factors well known in the medical field. In one embodiment, a therapeutically effective amount refers to an amount of drug that is effective in treating a disease.

At this time, the pharmaceutical composition may further include a pharmaceutically acceptable carrier. The pharmaceutically acceptable carrier may be any carrier that is a nontoxic material suitable for delivery to a patient. Distilled water, alcohol, fats, waxes, and inert solids may be included as carriers. Pharmacologically acceptable adjuvants (buffers, dispersants) may also be included in the pharmaceutical composition.

Specifically, the pharmaceutical composition may be prepared into a parenteral formulation according to the administration route using a conventional method known in the art including a pharmaceutically acceptable carrier. Herein, the meaning of "pharmaceutically acceptable" is that the activity of the active substance is not suppressed, while the subject of application (prescription) does not have toxicity beyond what is acceptable.

When the pharmaceutical composition is prepared as a parenteral formulation, it may be formulated in the form of injections, transdermal administration, nasal inhalation, and suppositories according to methods known in the art along with a suitable carrier. When formulating as an injection, sterilized water, ethanol, polyols, such as glycerol or propylene glycol, or combinations thereof, and preferably Ringer's solution, PBS (phosphate buffered saline) containing triethanol amine or sterile water for injections, and isotonic solutions, such as 5% dextrose, may be used as a suitable carrier. Regarding the formulation of the pharmaceutical composition, it is known in the art, and specific literatures [Remington's Pharmaceutical Sciences (19th ed., 1995)] and the like may be referred. The literature is considered a part of the present specification.

The preferred dosage of the pharmaceutical composition is in the range of 0.01 μg/kg to 10 g/kg, or 0.01 mg/kg to 1 g/kg per day depending on the status, weight, sex, and age of the patient, the severity of the patient, and the administration route. Administration may be performed once to several times a day or once a month to once every two weeks. Such dosages should not be construed as limiting the scope of the present invention in any respect.

The subjects to which the pharmaceutical composition may be applied (prescribed) are mammals and humans, and it is particularly preferable when they are human. The pharmaceutical composition of the present invention may further include any compounds or natural extracts known to have an effect in treating an inflammatory disease.

In another aspect of the present invention, there is provided a use of the anti-WARS 1 antibody or a fragment thereof for preparing a medicament for preventing or treating an inflammatory disease. At this time, the inflammatory disease, prevention, treatment, anti-WARS1 antibody, or fragment thereof are as described above.

In another aspect of the present invention, there is provided a use of the anti-WARS 1 antibody or a fragment thereof for preventing or treating an inflammatory disease. At this time, the inflammatory disease, prevention, treatment, anti-WARS 1 antibody, or fragment thereof are as described above.

In another aspect of the present invention, there is provided a method of preventing or treating an inflammatory disease comprising administering the anti-WARS1 antibody or a fragment thereof to an individual. At this time, the inflammatory disease, prevention, treatment, anti-WARS1 antibody or fragment thereof, and administration are as described above. The individual may be a mammal, and preferably a human. In addition, the individual may be a patient suffering from an inflammatory disease or an individual highly likely to suffer from an inflammatory disease.

The administration route, dosage, and frequency of administration of the antibody or a fragment thereof may be applied to a subject in various methods and amounts depending on the status of the patient and the presence of side effects, and the optimum administration method, dosage, and frequency of administration may be selected by those skilled in the art to a suitable range. The preferred dosage of the anti-WARS 1 antibody or a fragment thereof may be in the range of 0.01 μg/kg to 10 g/kg, or 0.01 mg/kg to 1 g/kg per day depending on the status, weight, sex, and age of the patient, the severity of the patient, and the administration route. Administration may be performed once to many times a day or once a month to once every two weeks. Such dosages should not be construed as limiting the scope of the present invention in any respect.

In addition, the antibody or a fragment thereof may be administered in combination with any compound or natural extract known to have an effect of treating an inflammatory disease, or formulated in the form of a combination formulation with other drugs.

**Mode for Carrying out the Invention**

Hereinafter, the present invention will be described in more detail by way of the following examples. However, the following examples are only for illustrating the present invention, and the scope of the present invention is not limited only to these examples.

**Example 1. Preparation of GKB101**

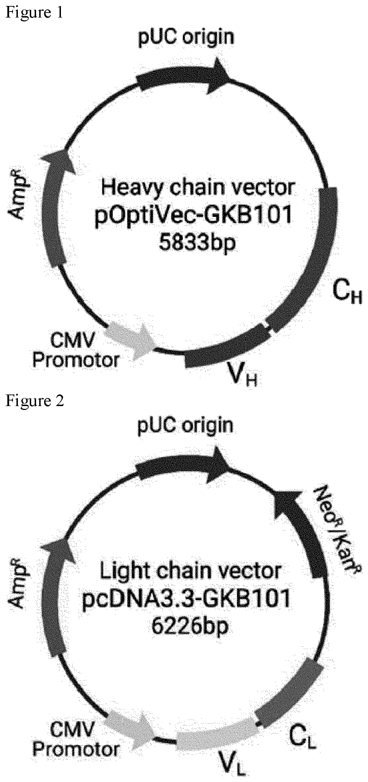

To produce an antibody that specifically binds to WARS1 (SEQ ID NO: 94), a polynucleotide (SEQ ID NO: 52) encoding a polypeptide comprising a signal peptide (SEQ ID NO: 1), a variable region (SEQ ID NO: 9) and a constant region (SEQ ID NO: 10) of an anti-WARS1 antibody heavy chain, a linker (SEQ ID NO: 50), and a Fc region (SEQ ID NO: 51); and a polynucleotide (SEQ ID NO: 53) of a polypeptide comprising a signal peptide (SEQ ID NO: 1), a variable region (SEQ ID NO: 18) and a constant region (SEQ ID NO: 19) of an anti-WARS1 antibody light chain were each cloned in pOptiVEC (Thermo Fisher, Figure 1) and a pcDNA 3.3 vector (Thermo Fisher, Figure 2). The information of the antibody used herein are as shown in Tables 2 and 3 below.

The vector was introduced into Expi293 cells using an Expifectamine 293 reagent (Thermo Fisher) at a ratio of 1:1 to be expressed with the heavy chain and the light chain in the same cell. After introducing the vector, the culture was shaken for about 5 days, then the cell culture was centrifuged, and the supernatant was recovered to separate and purify the antibody.

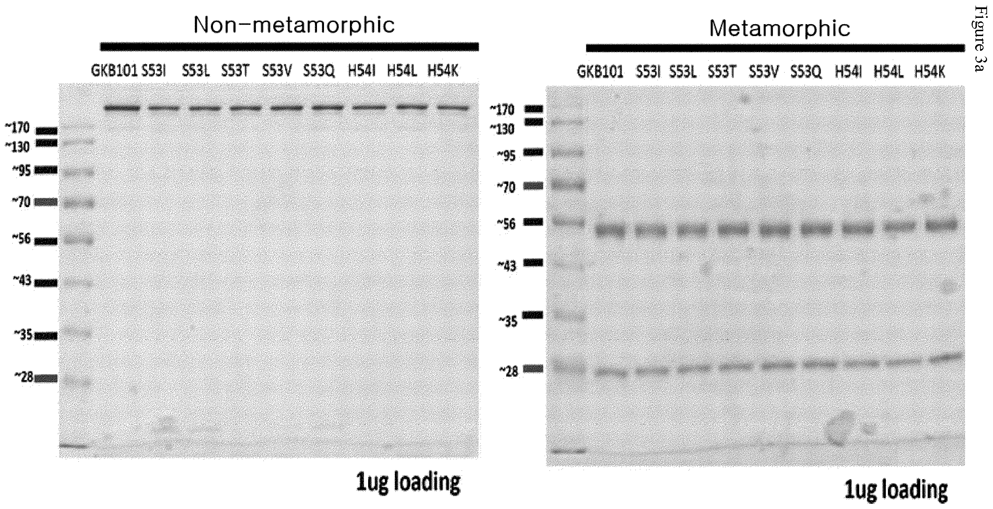

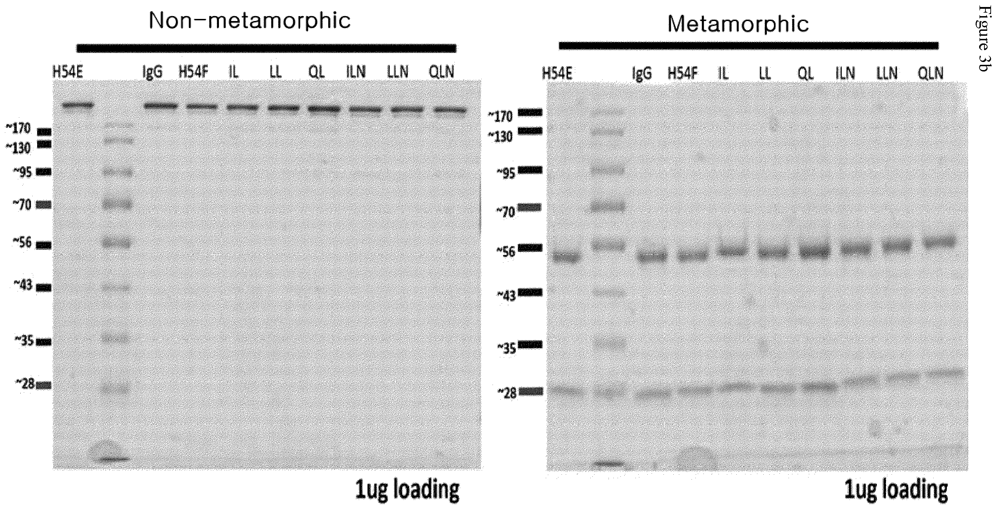

Specifically, the recovered supernatant was loaded on a Hitrap MabselectSure column (GE Healthcare Life Sciences) and washed with PBS to remove non-specific bonds. Proteins that specifically bind to the column were separated using 100 mM Citrate buffer (pH 3.0) + 300 mM NaCl. The eluate obtained by FPLC (fast protein liquid chromatography) was loaded onto size exclusion chromatography (GE Healthcare Life Sciences) to obtain high purity antibodies. The purified anti-WARS1 antibody was named "GKB101" (Table 1, Figures 3a and 3b).

[Table 1]

| GKB101 | | Amino acid sequence | Sequence number |
|---|---|---|---|
| Heavy chain region | Signal sequence | MGWSYIILFLVATAADVHS | 1 |
| | Heavy chain variable region | EVQLLESGGGLVQPGGSLRLSCAASGFTFS**SYD** **MS**WVRQAPGKGLEWVS**AISSGGSSIYYADSVK** **G**RFTISRDNSKNTLYLQMNSLRAEDTAVYYCAR **DVAWDMLGDFDY**WGQGTLVTVSS | 9 |

(continued)

| GKB101 | | Amino acid sequence | Sequence number |
|---|---|---|---|
| | Heavy chain constant region (CH1) | ASTKGPSVFPLAPSSKSTSGGTAALGCLVKDYFP EPVTVSWNSGALTSGVHTFPAVLQSSGLYSLSSV VTVPSSSLGTQTYICNVNHKPSNTKVDKKV | 10 |
| | Linker | EPPKSCDKTHTCPPCPAPELLGGP | 50 |
| | Fc region (CH2+CH3) | SVFLFPPKPKDTLMISRTPEVTCVVVDVSHEDPE VKFNWYVDGVEVHNAKTKPREEQYNSTYRVVS VLTVLHQDWLNGKEYKCKVSNKALPAPIEKTIS KAKGQPREPQVYTLPPSREEMTKNQVSLTCLVK GFYPSDIAVEWESNGQPENNYKTTPPVLDSDGSF FLYSKLTVDKSRWQQGNVFSCSVMHEALHNHY TQKSLSLPGK | 51 |
| Light chain region (kappa) | Signal sequence | MGWSYIILFLVATAADVHS | 1 |
| | Light chain variable region | QSVLTQPPSASGTPGQRVTISC**TGSSSNIGSNYVY** WYQQLPGTAPKLLIY**ANSHRPS**GVPDRFSGSKS GTSASLAISGLRSEDEADYYC**GAWDDSLSAYV**F GGGTKLTVL | 18 |
| | Light chain constant region | TVAAPSVFIFPPSDEQLKSGTASVVCLLNNFYPRE AKVQWKVDNALQSGNSQESVTEQDSKDSTYSL SSTLTLSKADYEKHKVYACEVTHQGLSSPVTKSF NRGEC | 19 |

**Example 2. Preparation of the variants of GKB101**

To prepare an antibody that specifically binds to WARS 1, several variants were created by changing the sequences of CDR2 and/or CDR3 of the light chain variable region based on the GKB101 in Example 1 above. At this time, the information of the antibody used is shown in Tables 2 and 3 below.

The light chain variant of the GKB101 was amplified through PCR and the primer nucleotide sequences used to amplify the genes included in the mutation sites of CDR2 and CDR3 of the light chain variable region are shown in Table 4.

PCR was performed on L-CDR2 and L-CDR3 variants including each mutation under the conditions of 98 °C/1 min; 98 °C/10 sec, 55 °C/15 sec, 72 °C/5 min, 28 cycles; and 72 °C/5 min using a vector template (pcDNA3.3-GKB101) and the primer (10 pmol each) to amplify the genes containing the mutation site. Afterwards, 0.5 ml of a DpnI restriction enzyme was added to react the PCR product with the enzyme at 37 °C for 30 minutes. After reacting with the enzyme, the purified PCR product was mixed with a NEBuilder Master mix (NEB, Cat# E2621) and reacted at 50 °C for 60 minutes, then transformed in DH5α competent cells to select the positive clones.

[Table 2]

| | H-CDR1 | H-CDR2 | H-CDR3 | L-CDR1 | L-CDR2 | L-CDR3 |
|---|---|---|---|---|---|---|
| GKB 101 | SYDMS (SEQ ID NO: 3) | AISSGGSSI YYADSVKG (SEQ ID NO: 5) | DVAWDM LGDFDY (SEQ ID NO: 7) | TGSSSNIG SNYVY (SEQ ID NO: 12) | ANSHRP S (SEQ ID NO: 14) | GAWDD SLSAYV (SEQ ID NO: 16) |

(continued)

|  | H-CDR1 | H-CDR2 | H-CDR3 | L-CDR1 | L-CDR2 | L-CDR3 |
|---|---|---|---|---|---|---|
| S53I | SYDMS (SEQ ID NO: 3) | AISSGGSSI YYADSVKG (SEQ ID NO: 5) | DVAWDM LGDFDY (SEQ ID NO: 7) | TGSSSNIG SNYVY (SEQ ID NO: 12) | ANIHRPS (SEQ ID NO: 20) | GAWDD SLSAYV (SEQ ID NO: 16) |
| S53L | SYDMS (SEQ ID NO: 3) | AISSGGSSI YYADSVKG (SEQ ID NO: 5) | DVAWDM LGDFDY (SEQ ID NO: 7) | TGSSSNIG SNYVY (SEQ ID NO: 12) | ANLHRP S (SEQ ID NO: 22) | GAWDD SLSAYV (SEQ ID NO: 16) |
| S53T | SYDMS (SEQ ID NO: 3) | AISSGGSSI YYADSVKG (SEQ ID NO: 5) | DVAWDM LGDFDY (SEQ ID NO: 7) | TGSSSNIG SNYVY (SEQ ID NO: 12) | ANTHRP S (SEQ ID NO: 24) | GAWDD SLSAYV (SEQ ID NO: 16) |
| S53V | SYDMS (SEQ ID NO: 3) | AISSGGSSI YYADSVKG (SEQ ID NO: 5) | DVAWDM LGDFDY (SEQ ID NO: 7) | TGSSSNIG SNYVY (SEQ ID NO: 12) | ANVHRP S (SEQ ID NO: 26) | GAWDD SLSAYV (SEQ ID NO: 16) |
| S53Q | SYDMS (SEQ ID NO: 3) | AISSGGSSI YYADSVKG (SEQ ID NO: 5) | DVAWDM LGDFDY (SEQ ID NO: 7) | TGSSSNIG SNYVY (SEQ ID NO: 12) | ANQHRP S (SEQ ID NO: 28) | GAWDD SLSAYV (SEQ ID NO: 16) |
| H54I | SYDMS (SEQ ID NO: 3) | AISSGGSSI YYADSVKG (SEQ ID NO: 5) | DVAWDM LGDFDY (SEQ ID NO: 7) | TGSSSNIG SNYVY (SEQ ID NO: 12) | ANSIRPS (SEQ ID NO: 30) | GAWDD SLSAYV (SEQ ID NO: 16) |
| H54L | SYDMS (SEQ ID NO: 3) | AISSGGSSI YYADSVKG (SEQ ID NO: 5) | DVAWDM LGDFDY (SEQ ID NO: 7) | TGSSSNIG SNYVY (SEQ ID NO: 12) | ANSLRPS (SEQ ID NO: 32) | GAWDD SLSAYV (SEQ ID NO: 16) |
| H54 K | SYDMS (SEQ ID NO: 3) | AISSGGSSI YYADSVKG (SEQ ID NO: 5) | DVAWDM LGDFDY (SEQ ID NO: 7) | TGSSSNIG SNYVY (SEQ ID NO: 12) | ANSKRP S (SEQ ID NO: 34) | GAWDD SLSAYV (SEQ ID NO: 16) |
| H54 Q | SYDMS (SEQ ID NO: 3) | AISSGGSSI YYADSVKG (SEQ ID NO: 5) | DVAWDM LGDFDY (SEQ ID NO: 7) | TGSSSNIG SNYVY (SEQ ID NO: 12) | ANSQRP S (SEQ ID NO: 36) | GAWDD SLSAYV (SEQ ID NO: 16) |
| H54F | SYDMS (SEQ ID NO: 3) | AISSGGSSI YYADSVKG (SEQ ID NO: 5) | DVAWDM LGDFDY (SEQ ID NO: 7) | TGSSSNIG SNYVY (SEQ ID NO: 12) | ANSFRPS (SEQ ID NO: 38) | GAWDD SLSAYV (SEQ ID NO: 16) |

(continued)

| | H-CDR1 | H-CDR2 | H-CDR3 | L-CDR1 | L-CDR2 | L-CDR3 |
|---|---|---|---|---|---|---|
| SH/I L | SYDMS (SEQ ID NO: 3) | AISSGGSSI YYADSVKG (SEQ ID NO: 5) | DVAWDM LGDFDY (SEQ ID NO: 7) | TGSSSNIG SNYVY (SEQ ID NO: 12) | ANILRPS (SEQ ID NO: 40) | GAWDD SLSAYV (SEQ ID NO: 16) |
| SH/L L | SYDMS (SEQ ID NO: 3) | AISSGGSSI YYADSVKG (SEQ ID NO: 5) | DVAWDM LGDFDY (SEQ ID NO: 7) | TGSSSNIG SNYVY (SEQ ID NO: 12) | ANLLRP S (SEQ ID NO: 42) | GAWDD SLSAYV (SEQ ID NO: 16) |
| SH/Q L | SYDMS (SEQ ID NO: 3) | AISSGGSSI YYADSVKG (SEQ ID NO: 5) | DVAWDM LGDFDY (SEQ ID NO: 7) | TGSSSNIG SNYVY (SEQ ID NO: 12) | ANQLRP S (SEQ ID NO: 44) | GAWDD SLSAYV (SEQ ID NO: 16) |
| SHL/ ILN | SYDMS (SEQ ID NO: 3) | AISSGGSSI YYADSVKG (SEQ ID NO: 5) | DVAWDM LGDFDY (SEQ ID NO: 7) | TGSSSNIG SNYVY (SEQ ID NO: 12) | ANILRPS (SEQ ID NO: 40) | GAWDD SNSAYV (SEQ ID NO: 46) |
| SHL/ LLN | SYDMS (SEQ ID NO: 3) | AISSGGSSI YYADSVKG (SEQ ID NO: 5) | DVAWDM LGDFDY (SEQ ID NO: 7) | TGSSSNIG SNYVY (SEQ ID NO: 12) | ANLLRP S (SEQ ID NO: 42) | GAWDD SNSAYV (SEQ ID NO: 46) |
| SHL/ QLN | SYDMS (SEQ ID NO: 3) | AISSGGSSI YYADSVKG (SEQ ID NO: 5) | DVAWDM LGDFDY (SEQ ID NO: 7) | TGSSSNIG SNYVY (SEQ ID NO: 12) | ANQLRP S (SEQ ID NO: 44) | GAWDD SNSAYV (SEQ ID NO: 46) |

[Table 3]

| Region | FR1 | FR2 | FR3 | FR4 |
|---|---|---|---|---|
| VH-FR | EVQLLESGGGLV QPGGSLRLSCAA SGFTFS (SEQ ID NO: 2) | WVRQAPGKG LEWVS (SEQ ID NO: 4) | RFTISRDNSKNTLYL QMNSLRAEDTAVYY CAR (SEQ ID NO: 6) | WGQGTLV TVSS (SEQ ID NO: 8) |
| LH-FR | QSVLTQPPSASG TPGQRVTISC (SEQ ID NO: 11) | WYQQLPGTA PKLLIY (SEQ ID NO: 13) | GVPDRFSGSKSGTSA SLAISGLRSEDEADY YC (SEQ ID NO: 15) | FGGGTKLT VL (SEQ ID NO: 17) |

[Table 4]

| Antibody | Region | Forward primer | Reverse primer |
|---|---|---|---|
| S53I | L-CDR2 | CTAATATTCATCGGCCAAG CGGGGTC (SEQ ID NO: 72) | GCCGATGAATATTAGCATAGAT GAGGAGTTTGGG (SEQ ID NO: 73) |
| S53L | L-CDR2 | CTAATCTGCATCGGCCAAG CGGGGTC (SEQ ID NO: 74) | GCCGATGCAGATTAGCATAGA TGAGGAGTTTGGG (SEQ ID NO: 75) |
| S53T | L-CDR2 | CTAATACTCATCGGCCAAG CGGGGTC (SEQ ID NO: 76) | GCCGATGAGTATTAGCATAGAT GAGGAGTTTGGG (SEQ ID NO: 77) |
| S53V | L-CDR2 | CTAATGTGCATCGGCCAAG CGGGGTC (SEQ ID NO: 78) | GCCGATGCACATTAGCATAGAT GAGGAGTTTGGG (SEQ ID NO: 79) |
| S53Q | L-CDR2 | CTAATCAGCATCGGCCAAG CGGGGTC (SEQ ID NO: 80) | GCCGATGCTGATTAGCATAGAT GAGGAGTTTGGG (SEQ ID NO: 81) |
| H54I | L-CDR2 | GCTAATAGTATTCGGCCAA GCGGGGTCC (SEQ ID NO: 82) | CCGAATACTATTAGCATAGATG AGGAGTTTGGG (SEQ ID NO: 83) |
| H54L | L-CDR2 | GCTAATAGTCTTCGGCCAA GCGGGGTCC (SEQ ID NO: 84) | CCGAAGACTATTAGCATAGAT GAGGAGTTTGGG (SEQ ID NO: 85) |
| H54K | L-CDR2 | GCTAATAGTAAGCGGCCAA GCGGGGTCC (SEQ ID NO: 86) | CCGCTTACTATTAGCATAGATG AGGAGTTTGGG (SEQ ID NO: 87) |
| H54Q | L-CDR2 | GCTAATAGTCAACGGCCAA GCGGGGTCC (SEQ ID NO: 88) | CCGTTGACTATTAGCATAGATG AGGAGTTTGGG (SEQ ID NO: 89) |
| H54F | L-CDR2 | GCTAATAGTTTTCGGCCAA GCGGGGTCC (SEQ ID NO: 90) | CCGAAAACTATTAGCATAGAT GAGGAGTTTGGG (SEQ ID NO: 91) |
| L96N | L-CDR3 | GATAGCAATAGTGCTTATGT CTTCGGGGGA (SEQ ID NO: 92) | AAGCACTATTGCTATCATCCCA AGCACCA (SEQ ID NO: 93) |

At this time, the nucleic acid sequence encoding ANSHRPS is GCTAATAGTCATCGGCCAAGC (SEQ ID NO: 70), and the nucleic acid sequence encoding GAWDDSLSAYV is GGTGCTTGGGATGATAGCCTGAGTGCTTATGTC (SEQ ID NO: 71).

**Example 2.1. Preparation of S53I**

To prepare an antibody that specifically binds to WARS1 (SEQ ID NO: 94), a polynucleotide (SEQ ID NO: 52) encoding a polypeptide comprising a signal peptide (SEQ ID NO: 1), a variable region (SEQ ID NO: 9) and a constant region (SEQ ID NO: 10) of an anti-WARS1 antibody heavy chain, a linker (SEQ ID NO: 50), and a Fc region (SEQ ID NO: 51); and a polynucleotide (SEQ ID NO: 54) of a polypeptide comprising a signal peptide (SEQ ID NO: 1), a variable region (SEQ ID NO: 21) and a constant region (SEQ ID NO: 19) of an anti-WARS1 antibody light chain were each loaded in pOptiVEC (Thermo Fisher, Figure 1) and a pcDNA 3.3 vector (Thermo Fisher, Figure 2).

The antibody was isolated and purified by introducing the vector into Expi293 cells in the same manner as Example 1, and was named "S53I" (Table 5, Figures 3a and 3b).

[Table 5]

| S53I | | Amino acid sequence | Sequence number |
|---|---|---|---|
| Heavy chain region | Signal sequence | MGWSYIILFLVATAADVHS | 1 |
| | Heavy chain variable region | EVQLLESGGGLVQPGGSLRLSCAASGFTFSSYDM SWVRQAPGKGLEWVSAISSGGSSIYYADSVKGR FTISRDNSKNTLYLQMNSLRAEDTAVYYCARDV AWDMLGDFDYWGQGTLVTVSS | 9 |
| | Heavy chain constant region (CH1) | ASTKGPSVFPLAPSSKSTSGGTAALGCLVKDYFP EPVTVSWNSGALTSGVHTFPAVLQSSGLYSLSSV VTVPSSSLGTQTYICNVNHKPSNTKVDKKV | 10 |
| | Linker | EPPKSCDKTHTCPPCPAPELLGGP | 50 |
| | Fc region (CH2+CH 3) | SVFLFPPKPKDTLMISRTPEVTCVVVDVSHEDPE VKFNWYVDGVEVHNAKTKPREEQYNSTYRVVS VLTVLHQDWLNGKEYKCKVSNKALPAPIEKTIS KAKGQPREPQVYTLPPSREEMTKNQVSLTCLVK GFYPSDIAVEWESNGQPENNYKTTPPVLDSDGSF FLYSKLTVDKSRWQQGNVFSCSVMHEALHNHY TQKSLSLPGK | 51 |
| Light chain region (kappa) | Signal sequence | MGWSYIILFLVATAADVHS | 1 |
| | Light chain variable region | QSVLTQPPSASGTPGQRVTISCTGSSSNIGSNYVY WYQQLPGTAPKLLIYANIHRPSGVPDRFSGSKSG TSASLAISGLRSEDEADYYCGAWDDSLSAYVFG GGTKLTVL | 21 |
| | Light chain constant region | TVAAPSVFIFPPSDEQLKSGTASVVCLLNNFYPRE AKVQWKVDNALQSGNSQESVTEQDSKDSTYSL SSTLTLSKADYEKHKVYACEVTHQGLSSPVTKSF NRGEC | 19 |

**Example 2.2. Preparation of S53L**

To prepare an antibody that specifically binds to WARS1 (SEQ ID NO: 94), a polynucleotide (SEQ ID NO: 52) encoding a polypeptide comprising a signal peptide (SEQ ID NO: 1), a variable region (SEQ ID NO: 9) and a constant region (SEQ ID NO: 10) of an anti-WARS1 antibody heavy chain, a linker (SEQ ID NO: 50), and a Fc region (SEQ ID NO: 51); and a polynucleotide (SEQ ID NO: 55) of a polypeptide comprising a signal peptide (SEQ ID NO: 1), a variable region (SEQ ID NO: 23) and a constant region (SEQ ID NO: 19) of an anti-WARS1 antibody light chain were each loaded in pOptiVEC (Thermo Fisher, Figure 1) and a pcDNA 3.3 vector (Thermo Fisher, Figure 2).

The antibody was isolated and purified by introducing the vector into Expi293 cells in the same manner as Example 1, and was named "S53L" (Table 6, Figures 3a and 3b).

[Table 6]

| S53L | | Amino acid sequence | Sequence number |
|---|---|---|---|
| Heavy chain region | Signal sequence | MGWSYIILFLVATAADVHS | 1 |
| | Heavy chain variable region | EVQLLESGGGLVQPGGSLRLSCAASGFTFSSYDM SWVRQAPGKGLEWVSAISSGGSSIYYADSVKGR FTISRDNSKNTLYLQMNSLRAEDTAVYYCARDV AWDMLGDFDYWGQGTLVTVSS | 9 |
| | Heavy chain constant region (CH1) | ASTKGPSVFPLAPSSKSTSGGTAALGCLVKDYFP EPVTVSWNSGALTSGVHTFPAVLQSSGLYSLSSV VT VPSSSLGTQTYICNVNHKPSNTKVDKKV | 10 |
| | Linker | EPPKSCDKTHTCPPCPAPELLGGP | 50 |
| | Fc region (CH2+CH 3) | SVFLFPPKPKDTLMISRTPEVTCVVVDVSHEDPE VKFNWYVDGVEVHNAKTKPREEQYNSTYRVVS VLTVLHQDWLNGKEYKCKVSNKALPAPIEKTIS KAKGQPREPQVYTLPPSREEMTKNQVSLTCLVK GFYPSDIAVEWESNGQPENNYKTTPPVLDSDGSF FLYSKLTVDKSRWQQGNVFSCSVMHEALHNHY TQKSLSLPGK | 51 |
| Light chain region (kappa) | Signal sequence | MGWSYIILFLVATAADVHS | 1 |
| | Light chain variable region | QSVLTQPPSASGTPGQRVTISCTGSSSNIGSNYVY WYQQLPGTAPKLLIYANLHRPSGVPDRFSGSKSG TSASLAISGLRSEDEADYYCGAWDDSLSAYVFG GGTKLTVL | 23 |
| | Light chain constant region | TVAAPSVFIFPPSDEQLKSGTASVVCLLNNFYPRE AKVQWKVDNALQSGNSQESVTEQDSKDSTYSL SSTLTLSKADYEKHKVYACEVTHQGLSSPVTKSF NRGEC | 19 |

**Example 2.3. Preparation of S53T**

To prepare an antibody that specifically binds to WARS1 (SEQ ID NO: 94), a polynucleotide (SEQ ID NO: 52) encoding a polypeptide comprising a signal peptide (SEQ ID NO: 1), a variable region (SEQ ID NO: 9) and a constant region (SEQ ID NO: 10) of an anti-WARS1 antibody heavy chain, a linker (SEQ ID NO: 50), and a Fc region (SEQ ID NO: 51); and a polynucleotide (SEQ ID NO: 56) of a polypeptide comprising a signal peptide (SEQ ID NO: 1), a variable region (SEQ ID NO: 25) and a constant region (SEQ ID NO: 19) of an anti-WARS1 antibody light chain were each loaded in pOptiVEC (Thermo Fisher, Figure 1) and a pcDNA 3.3 vector (Thermo Fisher, Figure 2).

The antibody was isolated and purified by introducing the vector into Expi293 cells in the same manner as Example 1, and was named "S53T" (Table 7, Figures 3a and 3b).

[Table 7]

| S53T | | Amino acid sequence | Sequence number |
|---|---|---|---|
| Heavy chain region | Signal sequence | MGWSYIILFLVATAADVHS | 1 |
| | Heavy chain variable region | EVQLLESGGGLVQPGGSLRLSCAASGFTFSSYDM SWVRQAPGKGLEWVSAISSGGSSIYYADSVKGR FTISRDNSKNTLYLQMNSLRAEDTAVYYCARDV AWDMLGDFDYWGQGTLVTVSS | 9 |
| | Heavy chain constant region (CH1) | ASTKGPSVFPLAPSSKSTSGGTAALGCLVKDYFP EPVTVSWNSGALTSGVHTFPAVLQSSGLYSLSSV VT VPSSSLGTQTYICNVNHKPSNTKVDKKV | 10 |
| | Linker | EPPKSCDKTHTCPPCPAPELLGGP | 50 |
| | Fc region (CH2+CH 3) | SVFLFPPKPKDTLMISRTPEVTCVVVDVSHEDPE VKFNWYVDGVEVHNAKTKPREEQYNSTYRVVS VLTVLHQDWLNGKEYKCKVSNKALPAPIEKTIS KAKGQPREPQVYTLPPSREEMTKNQVSLTCLVK GFYPSDIAVEWESNGQPENNYKTTPPVLDSDGSF FLYSKLTVDKSRWQQGNVFSCSVMHEALHNHY TQKSLSLPGK | 51 |
| Light chain region (kappa) | Signal sequence | MGWSYIILFLVATAADVHS | 1 |
| | Light chain variable region | QSVLTQPPSASGTPGQRVTISCTGSSSNIGSNYVY WYQQLPGTAPKLLIYANTHRPSGVPDRFSGSKSG TSASLAISGLRSEDEADYYCGAWDDSLSAYVFG GGTKLTVL | 25 |
| | Light chain constant region | TVAAPSVFIFPPSDEQLKSGTASVVCLLNNFYPRE AKVQWKVDNALQSGNSQESVTEQDSKDSTYSL SSTLTLSKADYEKHKVYACEVTHQGLSSPVTKSF NRGEC | 19 |

## Example 2.4. Preparation of S53V

To prepare an antibody that specifically binds to WARS1 (SEQ ID NO: 94), a polynucleotide (SEQ ID NO: 52) encoding a polypeptide comprising a signal peptide (SEQ ID NO: 1), a variable region (SEQ ID NO: 9) and a constant region (SEQ ID NO: 10) of an anti-WARS1 antibody heavy chain, a linker (SEQ ID NO: 50), and a Fc region (SEQ ID NO: 51); and a polynucleotide (SEQ ID NO: 57) of a polypeptide comprising a signal peptide (SEQ ID NO: 1), a variable region (SEQ ID NO: 27) and a constant region (SEQ ID NO: 19) of an anti-WARS1 antibody light chain were each loaded in pOptiVEC (Thermo Fisher, Figure 1) and a pcDNA 3.3 vector (Thermo Fisher, Figure 2).

The antibody was isolated and purified by introducing the vector into Expi293 cells in the same manner as Example 1, and was named "S53V" (Table 8, Figures 3a and 3b).

[Table 8]

| S53V | | Amino acid sequence | Sequence number |
|---|---|---|---|
| Heavy chain region | Signal sequence | MGWSYIILFLVATAADVHS | 1 |
| | Heavy chain variable region | EVQLLESGGGLVQPGGSLRLSCAASGFTFSSYDM SWVRQAPGKGLEWVSAISSGGSSIYYADSVKGR FTISRDNSKNTLYLQMNSLRAEDTAVYYCARDV AWDMLGDFDYWGQGTLVTVSS | 9 |
| | Heavy chain constant region (CH1) | ASTKGPSVFPLAPSSKSTSGGTAALGCLVKDYFP EPVTVSWNSGALTSGVHTFPAVLQSSGLYSLSSV VT VPSSSLGTQTYICNVNHKPSNTKVDKKV | 10 |
| | Linker | EPPKSCDKTHTCPPCPAPELLGGP | 50 |
| | Fc region (CH2+CH 3) | SVFLFPPKPKDTLMISRTPEVTCVVVDVSHEDPE VKFNWYVDGVEVHNAKTKPREEQYNSTYRVVS VLTVLHQDWLNGKEYKCKVSNKALPAPIEKTIS KAKGQPREPQVYTLPPSREEMTKNQVSLTCLVK GFYPSDIAVEWESNGQPENNYKTTPPVLDSDGSF FLYSKLTVDKSRWQQGNVFSCSVMHEALHNHY TQKSLSLPGK | 51 |
| Light chain region (kappa) | Signal sequence | MGWSYIILFLVATAADVHS | 1 |
| | Light chain variable region | QSVLTQPPSASGTPGQRVTISCTGSSSNIGSNYVY WYQQLPGTAPKLLIYANVHRPSGVPDRFSGSKS GTSASLAISGLRSEDEADYYCGAWDDSLSAYVF GGGTKLTVL | 27 |
| | Light chain constant region | TVAAPSVFIFPPSDEQLKSGTASVVCLLNNFYPRE AKVQWKVDNALQSGNSQESVTEQDSKDSTYSL SSTLTLSKADYEKHKVYACEVTHQGLSSPVTKSF NRGEC | 19 |

## Example 2.5. Preparation of S53Q

To prepare an antibody that specifically binds to WARS1 (SEQ ID NO: 94), a polynucleotide (SEQ ID NO: 52) encoding a polypeptide comprising a signal peptide (SEQ ID NO: 1), a variable region (SEQ ID NO: 9) and a constant region (SEQ ID NO: 10) of an anti-WARS1 antibody heavy chain, a linker (SEQ ID NO: 50), and a Fc region (SEQ ID NO: 51); and a polynucleotide (SEQ ID NO: 58) of a polypeptide comprising a signal peptide (SEQ ID NO: 1), a variable region (SEQ ID NO: 29) and a constant region (SEQ ID NO: 19) of an anti-WARS1 antibody light chain were each loaded in pOptiVEC (Thermo Fisher, Figure 1) and a pcDNA 3.3 vector (Thermo Fisher, Figure 2).

The antibody was isolated and purified by introducing the vector into Expi293 cells in the same manner as Example 1, and was named "S53Q" (Table 9, Figures 3a and 3b).

[Table 9]

| S53Q | | Amino acid sequence | Sequence number |
|---|---|---|---|
| Heavy chain region | Signal sequence | MGWSYIILFLVATAADVHS | 1 |
| | Heavy chain variable region | EVQLLESGGGLVQPGGSLRLSCAASGFTFSSYDM SWVRQAPGKGLEWVSAISSGGSSIYYADSVKGR FTISRDNSKNTLYLQMNSLRAEDTAVYYCARDV AWDMLGDFDYWGQGTLVTVSS | 9 |
| | Heavy chain constant region (CH1) | ASTKGPSVFPLAPSSKSTSGGTAALGCLVKDYFP EPVTVSWNSGALTSGVHTFPAVLQSSGLYSLSSV VT VPSSSLGTQTYICNVNHKPSNTKVDKKV | 10 |
| | Linker | EPPKSCDKTHTCPPCPAPELLGGP | 50 |
| | Fc region (CH2+CH 3) | SVFLFPPKPKDTLMISRTPEVTCVVVDVSHEDPE VKFNWYVDGVEVHNAKTKPREEQYNSTYRVVS VLTVLHQDWLNGKEYKCKVSNKALPAPIEKTIS KAKGQPREPQVYTLPPSREEMTKNQVSLTCLVK GFYPSDIAVEWESNGQPENNYKTTPPVLDSDGSF FLYSKLTVDKSRWQQGNVFSCSVMHEALHNHY TQKSLSLPGK | 51 |
| Light chain region (kappa) | Signal sequence | MGWSYIILFLVATAADVHS | 1 |
| | Light chain variable region | QSVLTQPPSASGTPGQRVTISCTGSSSNIGSNYVY WYQQLPGTAPKLLIYANQHRPSGVPDRFSGSKS GTSASLAISGLRSEDEADYYCGAWDDSLSAYVF GGGTKLTVL | 29 |
| | Light chain constant region | TVAAPSVFIFPPSDEQLKSGTASVVCLLNNFYPRE AKVQWKVDNALQSGNSQESVTEQDSKDSTYSL SSTLTLSKADYEKHKVYACEVTHQGLSSPVTKSF NRGEC | 19 |

### Example 2.6. Preparation of H54I

To prepare an antibody that specifically binds to WARS1 (SEQ ID NO: 94), a polynucleotide (SEQ ID NO: 52) encoding a polypeptide comprising a signal peptide (SEQ ID NO: 1), a variable region (SEQ ID NO: 9) and a constant region (SEQ ID NO: 10) of an anti-WARS1 antibody heavy chain, a linker (SEQ ID NO: 50), and a Fc region (SEQ ID NO: 51); and a polynucleotide (SEQ ID NO: 59) of a polypeptide comprising a signal peptide (SEQ ID NO: 1), a variable region (SEQ ID NO: 31) and a constant region (SEQ ID NO: 19) of an anti-WARS1 antibody light chain were each loaded in pOptiVEC (Thermo Fisher, Figure 1) and a pcDNA 3.3 vector (Thermo Fisher, Figure 2).

The antibody was isolated and purified by introducing the vector into Expi293 cells in the same manner as Example 1, and was named "S53I" (Table 10, Figures 3a and 3b).

[Table 10]

| H54I | | Amino acid sequence | Sequence number |
|---|---|---|---|
| Heavy chain region | Signal sequence | MGWSYIILFLVATAADVHS | 1 |
| | Heavy chain variable region | EVQLLESGGGLVQPGGSLRLSCAASGFTFSSYDM SWVRQAPGKGLEWVSAISSGGSSIYYADSVKGR FTISRDNSKNTLYLQMNSLRAEDTAVYYCARDV AWDMLGDFDYWGQGTLVTVSS | 9 |
| | Heavy chain constant region (CH1) | ASTKGPSVFPLAPSSKSTSGGTAALGCLVKDYFP EPVTVSWNSGALTSGVHTFPAVLQSSGLYSLSSV VT VPSSSLGTQTYICNVNHKPSNTKVDKKV | 10 |
| | Linker | EPPKSCDKTHTCPPCPAPELLGGP | 50 |
| | Fc region (CH2+CH 3) | SVFLFPPKPKDTLMISRTPEVTCVVVDVSHEDPE VKFNWYVDGVEVHNAKTKPREEQYNSTYRVVS VLTVLHQDWLNGKEYKCKVSNKALPAPIEKTIS KAKGQPREPQVYTLPPSREEMTKNQVSLTCLVK GFYPSDIAVEWESNGQPENNYKTTPPVLDSDGSF FLYSKLTVDKSRWQQGNVFSCSVMHEALHNHY TQKSLSLPGK | 51 |
| Light chain region (kappa) | Signal sequence | MGWSYIILFLVATAADVHS | 1 |
| | Light chain variable region | QSVLTQPPSASGTPGQRVTISCTGSSSNIGSNYVY WYQQLPGTAPKLLIYANSIRPSGVPDRFSGSKSG TSASLAISGLRSEDEADYYCGAWDDSLSAYVFG GGTKLTVL | 31 |
| | Light chain constant region | TVAAPSVFIFPPSDEQLKSGTASVVCLLNNFYPRE AKVQWKVDNALQSGNSQESVTEQDSKDSTYSL SSTLTLSKADYEKHKVYACEVTHQGLSSPVTKSF NRGEC | 19 |

## Example 2.7. Preparation of H54L

To prepare an antibody that specifically binds to WARS1 (SEQ ID NO: 94), a polynucleotide (SEQ ID NO: 52) encoding a polypeptide comprising a signal peptide (SEQ ID NO: 1), a variable region (SEQ ID NO: 9) and a constant region (SEQ ID NO: 10) of an anti-WARS1 antibody heavy chain, a linker (SEQ ID NO: 50), and a Fc region (SEQ ID NO: 51); and a polynucleotide (SEQ ID NO: 60) of a polypeptide comprising a signal peptide (SEQ ID NO: 1), a variable region (SEQ ID NO: 33) and a constant region (SEQ ID NO: 19) of an anti-WARS1 antibody light chain were each loaded in pOptiVEC (Thermo Fisher, Figure 1) and a pcDNA 3.3 vector (Thermo Fisher, Figure 2).

The antibody was isolated and purified by introducing the vector into Expi293 cells in the same manner as Example 1, and was named "H54L" (Table 11, Figures 3a and 3b).

[Table 11]

| H54L | | Amino acid sequence | Sequence number |
|---|---|---|---|
| Heavy chain region | Signal sequence | MGWSYIILFLVATAADVHS | 1 |
| | Heavy chain variable region | EVQLLESGGGLVQPGGSLRLSCAASGFTFSSYDM SWVRQAPGKGLEWVSAISSGGSSIYYADSVKGR FTISRDNSKNTLYLQMNSLRAEDTAVYYCARDV AWDMLGDFDYWGQGTLVTVSS | 9 |
| | Heavy chain constant region (CH1) | ASTKGPSVFPLAPSSKSTSGGTAALGCLVKDYFP EPVTVSWNSGALTSGVHTFPAVLQSSGLYSLSSV VT VPSSSLGTQTYICNVNHKPSNTKVDKKV | 10 |
| | Linker | EPPKSCDKTHTCPPCPAPELLGGP | 50 |
| | Fc region (CH2+CH 3) | SVFLFPPKPKDTLMISRTPEVTCVVVDVSHEDPE VKFNWYVDGVEVHNAKTKPREEQYNSTYRVVS VLTVLHQDWLNGKEYKCKVSNKALPAPIEKTIS KAKGQPREPQVYTLPPSREEMTKNQVSLTCLVK GFYPSDIAVEWESNGQPENNYKTTPPVLDSDGSF FLYSKLTVDKSRWQQGNVFSCSVMHEALHNHY TQKSLSLPGK | 51 |
| Light chain region (kappa) | Signal sequence | MGWSYIILFLVATAADVHS | 1 |
| | Light chain variable region | QSVLTQPPSASGTPGQRVTISCTGSSSNIGSNYVY WYQQLPGTAPKLLIYANSLRPSGVPDRFSGSKSG TSASLAISGLRSEDEADYYCGAWDDSLSAYVFG GGTKLTVL | 33 |
| | Light chain constant region | TVAAPSVFIFPPSDEQLKSGTASVVCLLNNFYPRE AKVQWKVDNALQSGNSQESVTEQDSKDSTYSL SSTLTLSKADYEKHKVYACEVTHQGLSSPVTKSF NRGEC | 19 |

## Example 2.8. Preparation of H54K

To prepare an antibody that specifically binds to WARS1 (SEQ ID NO: 94), a polynucleotide (SEQ ID NO: 52) encoding a polypeptide comprising a signal peptide (SEQ ID NO: 1), a variable region (SEQ ID NO: 9) and a constant region (SEQ ID NO: 10) of an anti-WARS1 antibody heavy chain, a linker (SEQ ID NO: 50), and a Fc region (SEQ ID NO: 51); and a polynucleotide (SEQ ID NO: 61) of a polypeptide comprising a signal peptide (SEQ ID NO: 1), a variable region (SEQ ID NO: 35) and a constant region (SEQ ID NO: 19) of an anti-WARS1 antibody light chain were each loaded in pOptiVEC (Thermo Fisher, Figure 1) and a pcDNA 3.3 vector (Thermo Fisher, Figure 2).

The antibody was isolated and purified by introducing the vector into Expi293 cells in the same manner as Example 1, and was named "H54K" (Table 12, Figures 3a and 3b).

[Table 12]

| H54K | | Amino acid sequence | Sequence number |
|---|---|---|---|
| Heavy chain region | Signal sequence | MGWSYIILFLVATAADVHS | 1 |
| | Heavy chain variable region | EVQLLESGGGLVQPGGSLRLSCAASGFTFSSYDM SWVRQAPGKGLEWVSAISSGGSSIYYADSVKGR FTISRDNSKNTLYLQMNSLRAEDTAVYYCARDV AWDMLGDFDYWGQGTLVTVSS | 9 |
| | Heavy chain constant region (CH1) | ASTKGPSVFPLAPSSKSTSGGTAALGCLVKDYFP EPVTVSWNSGALTSGVHTFPAVLQSSGLYSLSSV VT VPSSSLGTQTYICNVNHKPSNTKVDKKV | 10 |
| | Linker | EPPKSCDKTHTCPPCPAPELLGGP | 50 |
| | Fc region (CH2+CH 3) | SVFLFPPKPKDTLMISRTPEVTCVVVDVSHEDPE VKFNWYVDGVEVHNAKTKPREEQYNSTYRVVS VLTVLHQDWLNGKEYKCKVSNKALPAPIEKTIS KAKGQPREPQVYTLPPSREEMTKNQVSLTCLVK GFYPSDIAVEWESNGQPENNYKTTPPVLDSDGSF FLYSKLTVDKSRWQQGNVFSCSVMHEALHNHY TQKSLSLPGK | 51 |
| Light chain region (kappa) | Signal sequence | MGWSYIILFLVATAADVHS | 1 |
| | Light chain variable region | QSVLTQPPSASGTPGQRVTISCTGSSSNIGSNYVY WYQQLPGTAPKLLIYANSKRPSGVPDRFSGSKSG TSASLAISGLRSEDEADYYCGAWDDSLSAYVFG GGTKLTVL | 35 |
| | Light chain constant region | TVAAPSVFIFPPSDEQLKSGTASVVCLLNNFYPRE AKVQWKVDNALQSGNSQESVTEQDSKDSTYSL SSTLTLSKADYEKHKVYACEVTHQGLSSPVTKSF NRGEC | 19 |

## Example 2.9. Preparation of H54Q

To prepare an antibody that specifically binds to WARS1 (SEQ ID NO: 94), a polynucleotide (SEQ ID NO: 52) encoding a polypeptide comprising a signal peptide (SEQ ID NO: 1), a variable region (SEQ ID NO: 9) and a constant region (SEQ ID NO: 10) of an anti-WARS1 antibody heavy chain, a linker (SEQ ID NO: 50), and a Fc region (SEQ ID NO: 51); and a polynucleotide (SEQ ID NO: 62) of a polypeptide comprising a signal peptide (SEQ ID NO: 1), a variable region (SEQ ID NO: 37) and a constant region (SEQ ID NO: 19) of an anti-WARS1 antibody light chain were each loaded in pOptiVEC (Thermo Fisher, Figure 1) and a pcDNA 3.3 vector (Thermo Fisher, Figure 2).

The antibody was isolated and purified by introducing the vector into Expi293 cells in the same manner as Example 1, and was named "H54Q" (Table 13, Figures 3a and 3b).

# EP 4 516 812 A1

[Table 13]

| H54Q | | Amino acid sequence | Sequence number |
|---|---|---|---|
| Heavy chain region | Signal sequence | MGWSYIILFLVATAADVHS | 1 |
| | Heavy chain variable region | EVQLLESGGGLVQPGGSLRLSCAASGFTFSSYDM SWVRQAPGKGLEWVSAISSGGSSIYYADSVKGR FTISRDNSKNTLYLQMNSLRAEDTAVYYCARDV AWDMLGDFDYWGQGTLVTVSS | 9 |
| | Heavy chain constant region (CH1) | ASTKGPSVFPLAPSSKSTSGGTAALGCLVKDYFP EPVTVSWNSGALTSGVHTFPAVLQSSGLYSLSSV VT VPSSSLGTQTYICNVNHKPSNTKVDKKV | 10 |
| | Linker | EPPKSCDKTHTCPPCPAPELLGGP | 50 |
| | Fc region (CH2+CH 3) | SVFLFPPKPKDTLMISRTPEVTCVVVDVSHEDPE VKFNWYVDGVEVHNAKTKPREEQYNSTYRVVS VLTVLHQDWLNGKEYKCKVSNKALPAPIEKTIS KAKGQPREPQVYTLPPSREEMTKNQVSLTCLVK GFYPSDIAVEWESNGQPENNYKTTPPVLDSDGSF FLYSKLTVDKSRWQQGNVFSCSVMHEALHNHY TQKSLSLPGK | 51 |
| Light chain region (kappa) | Signal sequence | MGWSYIILFLVATAADVHS | 1 |
| | Light chain variable region | QSVLTQPPSASGTPGQRVTISCTGSSSNIGSNYVY WYQQLPGTAPKLLIYANSQRPSGVPDRFSGSKSG TSASLAISGLRSEDEADYYCGAWDDSLSAYVFG GGTKLTVL | 37 |
| | Light chain constant region | TVAAPSVFIFPPSDEQLKSGTASVVCLLNNFYPRE AKVQWKVDNALQSGNSQESVTEQDSKDSTYSL SSTLTLSKADYEKHKVYACEVTHQGLSSPVTKSF NRGEC | 19 |

## Example 2.10. Preparation of H54F

To prepare an antibody that specifically binds to WARS1 (SEQ ID NO: 94), a polynucleotide (SEQ ID NO: 52) encoding a polypeptide comprising a signal peptide (SEQ ID NO: 1), a variable region (SEQ ID NO: 9) and a constant region (SEQ ID NO: 10) of an anti-WARS1 antibody heavy chain, a linker (SEQ ID NO: 50), and a Fc region (SEQ ID NO: 51); and a polynucleotide (SEQ ID NO: 63) of a polypeptide comprising a signal peptide (SEQ ID NO: 1), a variable region (SEQ ID NO: 39) and a constant region (SEQ ID NO: 19) of an anti-WARS1 antibody light chain were each loaded in pOptiVEC (Thermo Fisher, Figure 1) and a pcDNA 3.3 vector (Thermo Fisher, Figure 2).

The antibody was isolated and purified by introducing the vector into Expi293 cells in the same manner as Example 1, and was named "H54F" (Table 14, Figures 3a and 3b).

[Table 14]

| H54F | | Amino acid sequence | Sequence number |
|---|---|---|---|
| Heavy chain region | Signal sequence | MGWSYIILFLVATAADVHS | 1 |
| | Heavy chain variable region | EVQLLESGGGLVQPGGSLRLSCAASGFTFSSYDM SWVRQAPGKGLEWVSAISSGGSSIYYADSVKGR FTISRDNSKNTLYLQMNSLRAEDTAVYYCARDV AWDMLGDFDYWGQGTLVTVSS | 9 |
| | Heavy chain constant region (CH1) | ASTKGPSVFPLAPSSKSTSGGTAALGCLVKDYFP EPVTVSWNSGALTSGVHTFPAVLQSSGLYSLSSV VT VPSSSLGTQTYICNVNHKPSNTKVDKKV | 10 |
| | Linker | EPPKSCDKTHTCPPCPAPELLGGP | 50 |
| | Fc region (CH2+CH 3) | SVFLFPPKPKDTLMISRTPEVTCVVVDVSHEDPE VKFNWYVDGVEVHNAKTKPREEQYNSTYRVVS VLTVLHQDWLNGKEYKCKVSNKALPAPIEKTIS KAKGQPREPQVYTLPPSREEMTKNQVSLTCLVK GFYPSDIAVEWESNGQPENNYKTTPPVLDSDGSF FLYSKLTVDKSRWQQGNVFSCSVMHEALHNHY TQKSLSLPGK | 51 |
| Light chain region (kappa) | Signal sequence | MGWSYIILFLVATAADVHS | 1 |
| | Light chain variable region | QSVLTQPPSASGTPGQRVTISCTGSSSNIGSNYVY WYQQLPGTAPKLLIYANSFRPSGVPDRFSGSKSG TSASLAISGLRSEDEADYYCGAWDDSLSAYVFG GGTKLTVL | 39 |
| | Light chain constant region | TVAAPSVFIFPPSDEQLKSGTASVVCLLNNFYPRE AKVQWKVDNALQSGNSQESVTEQDSKDSTYSL SSTLTLSKADYEKHKVYACEVTHQGLSSPVTKSF NRGEC | 19 |

**Example 2.11. Preparation of SH/IL**

To prepare an antibody that specifically binds to WARS1 (SEQ ID NO: 94), a polynucleotide (SEQ ID NO: 52) encoding a polypeptide comprising a signal peptide (SEQ ID NO: 1), a variable region (SEQ ID NO: 9) and a constant region (SEQ ID NO: 10) of an anti-WARS1 antibody heavy chain, a linker (SEQ ID NO: 50), and a Fc region (SEQ ID NO: 51); and a polynucleotide (SEQ ID NO: 64) of a polypeptide comprising a signal peptide (SEQ ID NO: 1), a variable region (SEQ ID NO: 41) and a constant region (SEQ ID NO: 19) of an anti-WARS1 antibody light chain were each loaded in pOptiVEC (Thermo Fisher, Figure 1) and a pcDNA 3.3 vector (Thermo Fisher, Figure 2).

The antibody was isolated and purified by introducing the vector into Expi293 cells in the same manner as Example 1, and was named "SH/IL" (Table 15, Figures 3a and 3b).

[Table 15]

| SH/IL | | Amino acid sequence | Sequence number |
|---|---|---|---|
| Heavy chain region | Signal sequence | MGWSYIILFLVATAADVHS | 1 |
| | Heavy chain variable region | EVQLLESGGGLVQPGGSLRLSCAASGFTFSSYDM SWVRQAPGKGLEWVSAISSGGSSIYYADSVKGR FTISRDNSKNTLYLQMNSLRAEDTAVYYCARDV AWDMLGDFDYWGQGTLVTVSS | 9 |
| | Heavy chain constant region (CH1) | ASTKGPSVFPLAPSSKSTSGGTAALGCLVKDYFP EPVTVSWNSGALTSGVHTFPAVLQSSGLYSLSSV VT VPSSSLGTQTYICNVNHKPSNTKVDKKV | 10 |
| | Linker | EPPKSCDKTHTCPPCPAPELLGGP | 50 |
| | Fc region (CH2+CH 3) | SVFLFPPKPKDTLMISRTPEVTCVVVDVSHEDPE VKFNWYVDGVEVHNAKTKPREEQYNSTYRVVS VLTVLHQDWLNGKEYKCKVSNKALPAPIEKTIS KAKGQPREPQVYTLPPSREEMTKNQVSLTCLVK GFYPSDIAVEWESNGQPENNYKTTPPVLDSDGSF FLYSKLTVDKSRWQQGNVFSCSVMHEALHNHY TQKSLSLPGK | 51 |
| Light chain region (kappa) | Signal sequence | MGWSYIILFLVATAADVHS | 1 |
| | Light chain variable region | QSVLTQPPSASGTPGQRVTISCTGSSSNIGSNYVY WYQQLPGTAPKLLIYANILRPSGVPDRFSGSKSG TSASLAISGLRSEDEADYYCGAWDDSLSAYVFG GGTKLTVL | 41 |
| | Light chain constant region | TVAAPSVFIFPPSDEQLKSGTASVVCLLNNFYPRE AKVQWKVDNALQSGNSQESVTEQDSKDSTYSL SSTLTLSKADYEKHKVYACEVTHQGLSSPVTKSF NRGEC | 19 |

## Example 2.12. Preparation of SH/LL

To prepare an antibody that specifically binds to WARS1 (SEQ ID NO: 94), a polynucleotide (SEQ ID NO: 52) encoding a polypeptide comprising a signal peptide (SEQ ID NO: 1), a variable region (SEQ ID NO: 9) and a constant region (SEQ ID NO: 10) of an anti-WARS1 antibody heavy chain, a linker (SEQ ID NO: 50), and a Fc region (SEQ ID NO: 51); and a polynucleotide (SEQ ID NO: 65) of a polypeptide comprising a signal peptide (SEQ ID NO: 1), a variable region (SEQ ID NO: 43) and a constant region (SEQ ID NO: 19) of an anti-WARS1 antibody light chain were each loaded in pOptiVEC (Thermo Fisher, Figure 1) and a pcDNA 3.3 vector (Thermo Fisher, Figure 2).

The antibody was isolated and purified by introducing the vector into Expi293 cells in the same manner as Example 1, and was named "SH/LL" (Table 16, Figures 3a and 3b).

[Table 16]

| SH/LL | | Amino acid sequence | Sequence number |
|---|---|---|---|
| Heavy chain region | Signal sequence | MGWSYIILFLVATAADVHS | 1 |
| | Heavy chain variable region | EVQLLESGGGLVQPGGSLRLSCAASGFTFSSYDM SWVRQAPGKGLEWVSAISSGGSSIYYADSVKGR FTISRDNSKNTLYLQMNSLRAEDTAVYYCARDV AWDMLGDFDYWGQGTLVTVSS | 9 |
| | Heavy chain constant region (CH1) | ASTKGPSVFPLAPSSKSTSGGTAALGCLVKDYFP EPVTVSWNSGALTSGVHTFPAVLQSSGLYSLSSV VT VPSSSLGTQTYICNVNHKPSNTKVDKKV | 10 |
| | Linker | EPPKSCDKTHTCPPCPAPELLGGP | 50 |
| | Fc region (CH2+CH 3) | SVFLFPPKPKDTLMISRTPEVTCVVVDVSHEDPE VKFNWYVDGVEVHNAKTKPREEQYNSTYRVVS VLTVLHQDWLNGKEYKCKVSNKALPAPIEKTIS KAKGQPREPQVYTLPPSREEMTKNQVSLTCLVK GFYPSDIAVEWESNGQPENNYKTTPPVLDSDGSF FLYSKLTVDKSRWQQGNVFSCSVMHEALHNHY TQKSLSLPGK | 51 |
| Light chain region (kappa) | Signal sequence | MGWSYIILFLVATAADVHS | 1 |
| | Light chain variable region | QSVLTQPPSASGTPGQRVTISCTGSSSNIGSNYVY WYQQLPGTAPKLLIYANLLRPSGVPDRFSGSKSG TSASLAISGLRSEDEADYYCGAWDDSLSAYVFG GGTKLTVL | 43 |
| | Light chain constant region | TVAAPSVFIFPPSDEQLKSGTASVVCLLNNFYPRE AKVQWKVDNALQSGNSQESVTEQDSKDSTYSL SSTLTLSKADYEKHKVYACEVTHQGLSSPVTKSF NRGEC | 19 |

**Example 2.13. Preparation of** SH/QL

To prepare an antibody that specifically binds to WARS1 (SEQ ID NO: 94), a polynucleotide (SEQ ID NO: 52) encoding a polypeptide comprising a signal peptide (SEQ ID NO: 1), a variable region (SEQ ID NO: 9) and a constant region (SEQ ID NO: 10) of an anti-WARS1 antibody heavy chain, a linker (SEQ ID NO: 50), and a Fc region (SEQ ID NO: 51); and a polynucleotide (SEQ ID NO: 66) of a polypeptide comprising a signal peptide (SEQ ID NO: 1), a variable region (SEQ ID NO: 45) and a constant region (SEQ ID NO: 19) of an anti-WARS1 antibody light chain were each loaded in pOptiVEC (Thermo Fisher, Figure 1) and a pcDNA 3.3 vector (Thermo Fisher, Figure 2).

The antibody was isolated and purified by introducing the vector into Expi293 cells in the same manner as Example 1, and was named "SH/QL" (Table 17, Figures 3a and 3b).

[Table 17]

| SH/QL | | Amino acid sequence | Sequence number |
|---|---|---|---|
| Heavy chain region | Signal sequence | MGWSYIILFLVATAADVHS | 1 |
| | Heavy chain variable region | EVQLLESGGGLVQPGGSLRLSCAASGFTFSSYDM SWVRQAPGKGLEWVSAISSGGSSIYYADSVKGR FTISRDNSKNTLYLQMNSLRAEDTAVYYCARDV AWDMLGDFDYWGQGTLVTVSS | 9 |
| | Heavy chain constant region (CH1) | ASTKGPSVFPLAPSSKSTSGGTAALGCLVKDYFP EPVTVSWNSGALTSGVHTFPAVLQSSGLYSLSSV VT VPSSSLGTQTYICNVNHKPSNTKVDKKV | 10 |
| | Linker | EPPKSCDKTHTCPPCPAPELLGGP | 50 |
| | Fc region (CH2+CH 3) | SVFLFPPKPKDTLMISRTPEVTCVVVDVSHEDPE VKFNWYVDGVEVHNAKTKPREEQYNSTYRVVS VLTVLHQDWLNGKEYKCKVSNKALPAPIEKTIS KAKGQPREPQVYTLPPSREEMTKNQVSLTCLVK GFYPSDIAVEWESNGQPENNYKTTPPVLDSDGSF FLYSKLTVDKSRWQQGNVFSCSVMHEALHNHY TQKSLSLPGK | 51 |
| Light chain region (kappa) | Signal sequence | MGWSYIILFLVATAADVHS | 1 |
| | Light chain variable region | QSVLTQPPSASGTPGQRVTISCTGSSSNIGSNYVY WYQQLPGTAPKLLIYANQLRPSGVPDRFSGSKSG TSASLAISGLRSEDEADYYCGAWDDSLSAYVFG GGTKLTVL | 45 |
| | Light chain constant region | TVAAPSVFIFPPSDEQLKSGTASVVCLLNNFYPRE AKVQWKVDNALQSGNSQESVTEQDSKDSTYSL SSTLTLSKADYEKHKVYACEVTHQGLSSPVTKSF NRGEC | 19 |

## Example 2.14. Preparation of SHL/ILN

To prepare an antibody that specifically binds to WARS1 (SEQ ID NO: 94), a polynucleotide (SEQ ID NO: 52) encoding a polypeptide comprising a signal peptide (SEQ ID NO: 1), a variable region (SEQ ID NO: 9) and a constant region (SEQ ID NO: 10) of an anti-WARS1 antibody heavy chain, a linker (SEQ ID NO: 50), and a Fc region (SEQ ID NO: 51); and a polynucleotide (SEQ ID NO: 67) of a polypeptide comprising a signal peptide (SEQ ID NO: 1), a variable region (SEQ ID NO: 47) and a constant region (SEQ ID NO: 19) of an anti-WARS1 antibody light chain were each loaded in pOptiVEC (Thermo Fisher, Figure 1) and a pcDNA 3.3 vector (Thermo Fisher, Figure 2).

The antibody was isolated and purified by introducing the vector into Expi293 cells in the same manner as Example 1, and was named "SHL/ILN" (Table 18, Figures 3a and 3b).

[Table 18]

| SHL/ILN | | Amino acid sequence | Sequence number |
|---|---|---|---|
| Heavy chain region | Signal sequence | MGWSYIILFLVATAADVHS | 1 |
| | Heavy chain variable region | EVQLLESGGGLVQPGGSLRLSCAASGFTFSSYDM SWVRQAPGKGLEWVSAISSGGSSIYYADSVKGR FTISRDNSKNTLYLQMNSLRAEDTAVYYCARDV AWDMLGDFDYWGQGTLVTVSS | 9 |
| | Heavy chain constant region (CH1) | ASTKGPSVFPLAPSSKSTSGGTAALGCLVKDYFP EPVTVSWNSGALTSGVHTFPAVLQSSGLYSLSSV VT VPSSSLGTQTYICNVNHKPSNTKVDKKV | 10 |
| | Linker | EPPKSCDKTHTCPPCPAPELLGGP | 50 |
| | Fc region (CH2+CH 3) | SVFLFPPKPKDTLMISRTPEVTCVVVDVSHEDPE VKFNWYVDGVEVHNAKTKPREEQYNSTYRVVS VLTVLHQDWLNGKEYKCKVSNKALPAPIEKTIS KAKGQPREPQVYTLPPSREEMTKNQVSLTCLVK GFYPSDIAVEWESNGQPENNYKTTPPVLDSDGSF FLYSKLTVDKSRWQQGNVFSCSVMHEALHNHY TQKSLSLPGK | 51 |
| Light chain region (kappa) | Signal sequence | MGWSYIILFLVATAADVHS | 1 |
| | Light chain variable region | QSVLTQPPSASGTPGQRVTISCTGSSSNIGSNYVY WYQQLPGTAPKLLIYANILRPSGVPDRFSGSKSG TSASLAISGLRSEDEADYYCGAWDDSLSAYVFG GGTKLTVL | 47 |
| | Light chain constant region | TVAAPSVFIFPPSDEQLKSGTASVVCLLNNFYPRE AKVQWKVDNALQSGNSQESVTEQDSKDSTYSL SSTLTLSKADYEKHKVYACEVTHQGLSSPVTKSF NRGEC | 19 |

## Example 2.15. Preparation of SHL/LLN

To prepare an antibody that specifically binds to WARS1 (SEQ ID NO: 94), a polynucleotide (SEQ ID NO: 52) encoding a polypeptide comprising a signal peptide (SEQ ID NO: 1), a variable region (SEQ ID NO: 9) and a constant region (SEQ ID NO: 10) of an anti-WARS1 antibody heavy chain, a linker (SEQ ID NO: 50), and a Fc region (SEQ ID NO: 51); and a polynucleotide (SEQ ID NO: 68) of a polypeptide comprising a signal peptide (SEQ ID NO: 1), a variable region (SEQ ID NO: 48) and a constant region (SEQ ID NO: 19) of an anti-WARS1 antibody light chain were each loaded in pOptiVEC (Thermo Fisher, Figure 1) and a pcDNA 3.3 vector (Thermo Fisher, Figure 2).

The antibody was isolated and purified by introducing the vector into Expi293 cells in the same manner as Example 1, and was named "SHL/LLN" (Table 19, Figures 3a and 3b).

[Table 19]

| SHL/LLN | | Amino acid sequence | Sequence number |
|---|---|---|---|
| Heavy chain region | Signal sequence | MGWSYIILFLVATAADVHS | 1 |
| | Heavy chain variable region | EVQLLESGGGLVQPGGSLRLSCAASGFTFSSYDM SWVRQAPGKGLEWVSAISSGGSSIYYADSVKGR FTISRDNSKNTLYLQMNSLRAEDTAVYYCARDV AWDMLGDFDYWGQGTLVTVSS | 9 |
| | Heavy chain constant region (CH1) | ASTKGPSVFPLAPSSKSTSGGTAALGCLVKDYFP EPVTVSWNSGALTSGVHTFPAVLQSSGLYSLSSV VT VPSSSLGTQTYICNVNHKPSNTKVDKKV | 10 |
| | Linker | EPPKSCDKTHTCPPCPAPELLGGP | 50 |
| | Fc region (CH2+CH 3) | SVFLFPPKPKDTLMISRTPEVTCVVVDVSHEDPE VKFNWYVDGVEVHNAKTKPREEQYNSTYRVVS VLTVLHQDWLNGKEYKCKVSNKALPAPIEKTIS KAKGQPREPQVYTLPPSREEMTKNQVSLTCLVK GFYPSDIAVEWESNGQPENNYKTTPPVLDSDGSF FLYSKLTVDKSRWQQGNVFSCSVMHEALHNHY TQKSLSLPGK | 51 |
| Light chain region (kappa) | Signal sequence | MGWSYIILFLVATAADVHS | 1 |
| | Light chain variable region | QSVLTQPPSASGTPGQRVTISCTGSSSNIGSNYVY WYQQLPGTAPKLLIYANLLRPSGVPDRFSGSKSG TSASLAISGLRSEDEADYYCGAWDDSLSAYVFG GGTKLTVL | 48 |
| | Light chain constant region | TVAAPSVFIFPPSDEQLKSGTASVVCLLNNFYPRE AKVQWKVDNALQSGNSQESVTEQDSKDSTYSL SSTLTLSKADYEKHKVYACEVTHQGLSSPVTKSF NRGEC | 19 |

## Example 2.16. Preparation of SHL/QLN

To prepare an antibody that specifically binds to WARS1 (SEQ ID NO: 94), a polynucleotide (SEQ ID NO: 52) encoding a polypeptide comprising a signal peptide (SEQ ID NO: 1), a variable region (SEQ ID NO: 9) and a constant region (SEQ ID NO: 10) of an anti-WARS1 antibody heavy chain, a linker (SEQ ID NO: 50), and a Fc region (SEQ ID NO: 51); and a polynucleotide (SEQ ID NO: 69) of a polypeptide comprising a signal peptide (SEQ ID NO: 1), a variable region (SEQ ID NO: 49) and a constant region (SEQ ID NO: 19) of an anti-WARS1 antibody light chain were each loaded in pOptiVEC (Thermo Fisher, Figure 1) and a pcDNA 3.3 vector (Thermo Fisher, Figure 2).

The antibody was isolated and purified by introducing the vector into Expi293 cells in the same manner as Example 1, and was named "SHL/QLN" (Table 20, Figures 3a and 3b).

[Table 20]

| SHL/QLN | | Amino acid sequence | Sequence number |
|---|---|---|---|
| Heavy chain region | Signal sequence | MGWSYIILFLVATAADVHS | 1 |
| | Heavy chain variable region | EVQLLESGGGLVQPGGSLRLSCAASGFTFSSYDM SWVRQAPGKGLEWVSAISSGGSSIYYADSVKGR FTISRDNSKNTLYLQMNSLRAEDTAVYYCARDV AWDMLGDFDYWGQGTLVTVSS | 9 |
| | Heavy chain constant region (CH1) | ASTKGPSVFPLAPSSKSTSGGTAALGCLVKDYFP EPVTVSWNSGALTSGVHTFPAVLQSSGLYSLSSV VT VPSSSLGTQTYICNVNHKPSNTKVDKKV | 10 |
| | Linker | EPPKSCDKTHTCPPCPAPELLGGP | 50 |
| | Fc region (CH2+CH 3) | SVFLFPPKPKDTLMISRTPEVTCVVVDVSHEDPE VKFNWYVDGVEVHNAKTKPREEQYNSTYRVVS VLTVLHQDWLNGKEYKCKVSNKALPAPIEKTIS KAKGQPREPQVYTLPPSREEMTKNQVSLTCLVK GFYPSDIAVEWESNGQPENNYKTTPPVLDSDGSF FLYSKLTVDKSRWQQGNVFSCSVMHEALHNHY TQKSLSLPGK | 51 |
| Light chain region (kappa) | Signal sequence | MGWSYIILFLVATAADVHS | 1 |
| | Light chain variable region | QSVLTQPPSASGTPGQRVTISCTGSSSNIGSNYVY WYQQLPGTAPKLLIYANQLRPSGVPDRFSGSKSG TSASLAISGLRSEDEADYYCGAWDDSLSAYVFG GGTKLTVL | 49 |
| | Light chain constant region | TVAAPSVFIFPPSDEQLKSGTASVVCLLNNFYPRE AKVQWKVDNALQSGNSQESVTEQDSKDSTYSL SSTLTLSKADYEKHKVYACEVTHQGLSSPVTKSF NRGEC | 19 |

**Example 3. Identification of the binding ability of the anti-WARS1 antibody to WARS1**

**Example 3.1. Identification of the binding ability of the anti-WARS1 antibody to WARS1 *in vitro***

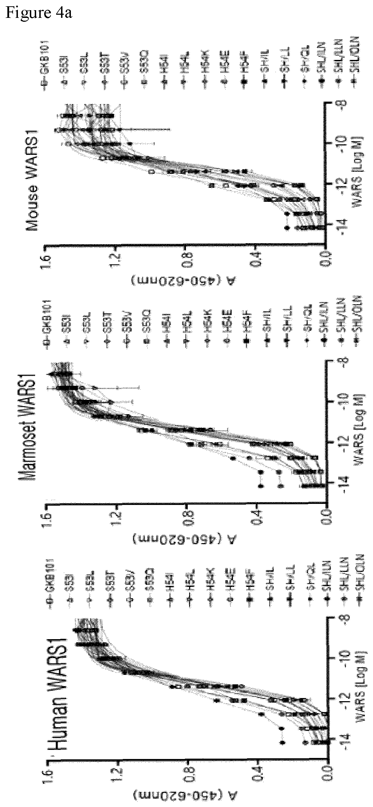

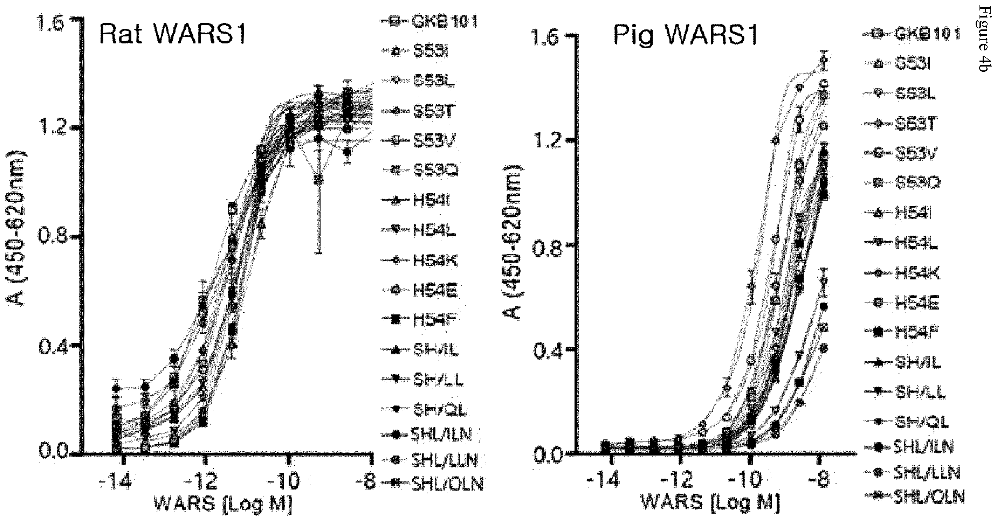

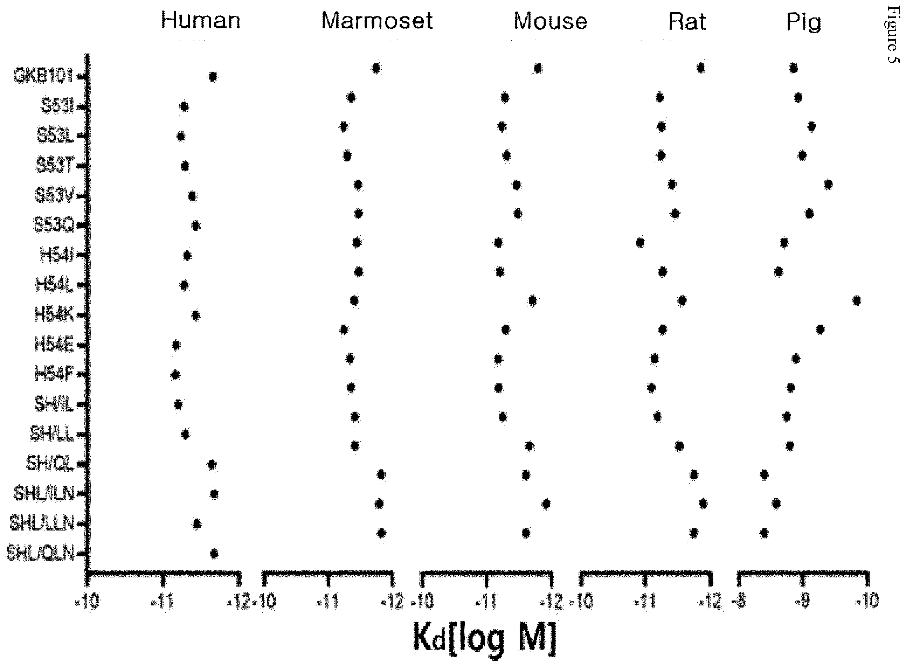

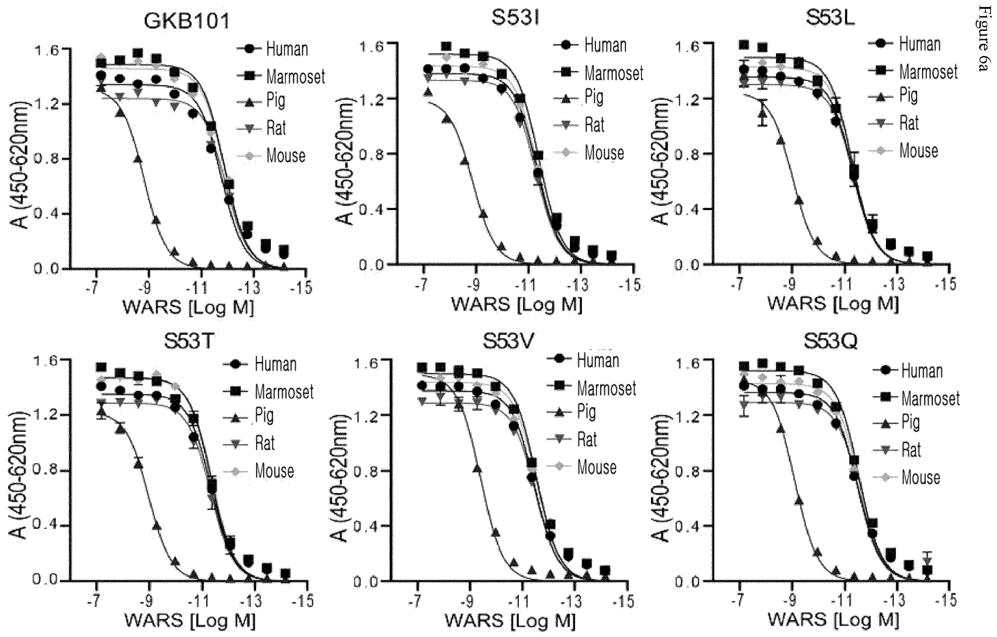

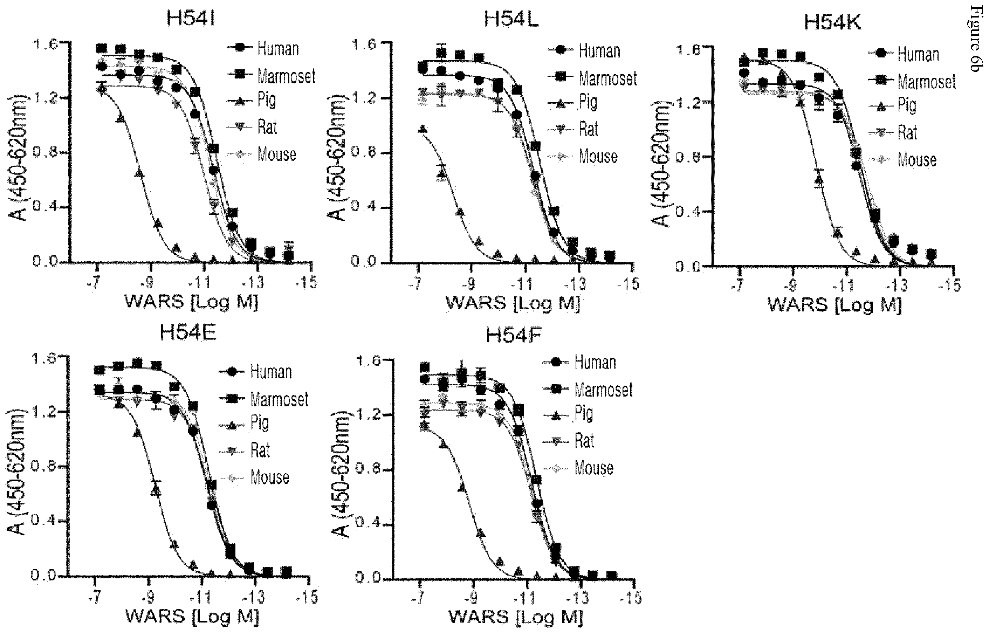

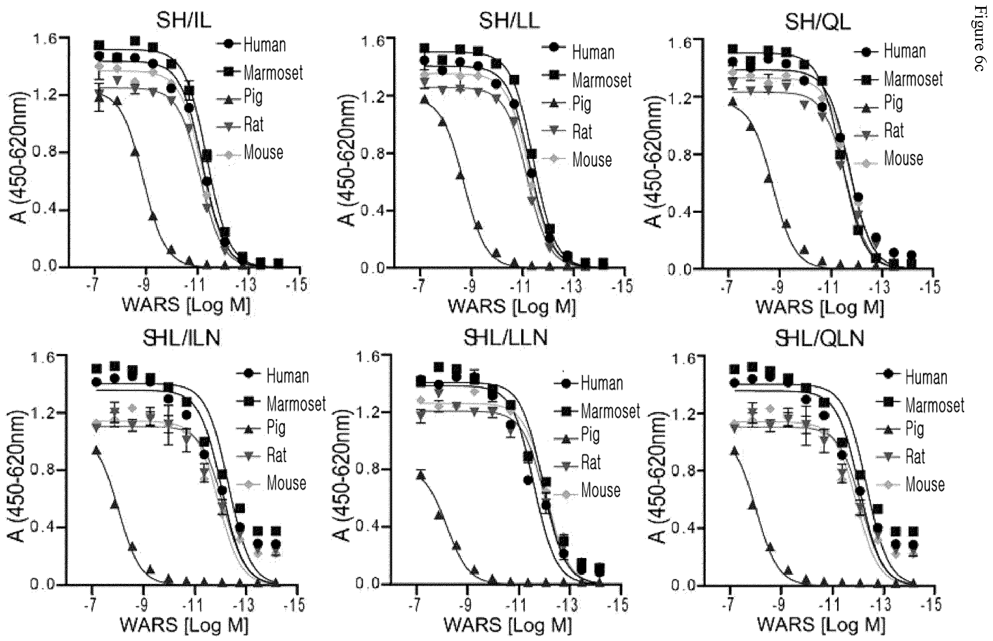

The binding ability of the anti-WARS1 antibody of the present invention prepared in the same manner as Examples 1 and 2 to a WARS 1 protein was identified using direct ELISA and sandwich ELISA methods.
Specifically, for direct ELISA, a 96-well plate (Maxisorp, Thermo Fisher) was coated with WARS1 having a 1 μg/ml concentration of a recombinant protein derived from human, marmoset (NHP), mouse, rat, and pig, and then blocked with PBS containing 1% BSA at room temperature for 1 hour. The GKB 101 antibody or a variant antibody thereof was diluted in a concentration-dependent manner, added to each coated well, reacted at room temperature for 1 hour, and then washed four times with PBST (PBS+ Tween20). Each well was treated and reacted for 1 hour with an antibody (anti-Human IgG, cell signaling) to which HRP is attached, and then washed with PBST. Lastly, after developing with a TMB substrate (BD), its absorbance was measured at 490 nm (NIVO) (Figures 4a to 5).
Sandwich ELISA was performed by coating a 96-well plate (Maxisorp, Thermo Fisher) with the GKB 101 antibody or a variant antibody thereof at a concentration of 1 μg/ml, and then blocking with PBS containing 1% BSA at room temperature for 1 hour. WARS1 recombinant protein derived from human, marmoset (NHP), mouse, rat, or pig was diluted at a concentration-dependent manner and added to each well coated with the antibody to be reacted at room temperature for 1 hour, and then washed with PBST (PBS+ Tween20) four times. Each well was reacted with an anti-His tag antibody (Santa cruz) for 1 hour. After washing with PBST (PBS+ Tween20) four times, it was reacted with an antibody (anti-mouse IgG, Santa cruz) to which HRP is attached for 1 hour, and then after developing with a TMB substrate (BD), its absorbance was measured at 490 nm (NIVO) (Figures 6a to 6c).

As a result, the binding ability of the anti-WARS 1 antibody to the recombinant WARS 1 protein of the GKB101 antibody and a variant antibody thereof was measured and compared, through which a strong bonding force equivalent to or greater than the GKB101 antibody to human, marmoset (NHP), mouse, rat, or pig-derived WARS1 recombinant protein was identified.

In addition, for human-derived recombinant proteins, the S53Q, H54K, SH/QL, SHL/ILN, SHL/LLN, and SHL/QLN antibodies have a $K_d$ value of about $10^{-12}$ M, and for marmoset (NHP)-derived recombinant proteins, the SHL/ILN, SHL/LLN, and SHL/QLN antibodies have a $K_d$ value of about $10^{-12}$ M.

In addition, for mouse-derived recombinant proteins, the S53V, S53Q, H54K, H54Q, SH/QL, SHL/ILN, SHL/LLN, and SHL/QLN antibodies have a $K_d$ value of about $10^{-12}$ M. In addition, for rat-derived recombinant proteins, the S53V, S53Q, H54L, H54K, H54Q, SH/QL, SHL/ILN, SHL/LLN, and SHL/QLN antibodies have a $K_d$ value of about $10^{-12}$ M. In addition, for pig-derived recombinant proteins, the S53L, S53V, S53Q, H54K, and H54Q antibodies have a $K_d$ value of about $10^{-9}$ M. As a result, for WARS1 recombinant proteins, the S53V, S53Q, H54L, H54K, H54Q, SH/QL, SHL/ILN, SHL/LLN, and SHL/QLN antibodies have a high binding ability at a $K_d$ value of about $10^{-12}$ M.

## Example 3.2. Identification of the binding affinity of the anti-WARS1 antibody to WARS1 using western blot

The binding affinity of the anti-WARS 1 antibody of the present invention prepared in the same manner as Examples 1 and 2 to WARS1 protein was identified through western blot.

At this time, a WARS 1 recombinant protein (human, mouse, pig, marmoset (NHP), or rat-derived), cell culture medium, and human or mouse serum were used as WARS1 protein samples. In particular, the cell culture medium was used by treating the human monocyte cell line, THP-1, or the mouse macrophage cell line, J774.1A, with LPS (lipopolysaccharide, 100 ng/ml) for 24 hours to induce an inflammatory response, and then the cell culture supernatant was obtained for use.

30 $\mu$g of each of the WARS1 recombinant protein (human, mouse, pig, marmoset (NHP), or rat-derived), the cell supernatant obtained using the method above, and mouse or human serum was loaded onto 8% SDS-PAGE, then transferred to a PVDF membrane (Millipore). Afterwards, the membrane was reacted with 1 $\mu$g/ml of GKB101 or a variant antibody thereof for 1 hour each, then washed with TBST (TBS+Tween20) three times. Afterwards, it was reacted with a secondary antibody (anti-human IgG HRP, Millipore) for 1 hour, then washed with TBST three times to be treated with ECL (West® bright ECL, Advanta) to identify its protein expression (LAS-4000, Fujifilm).

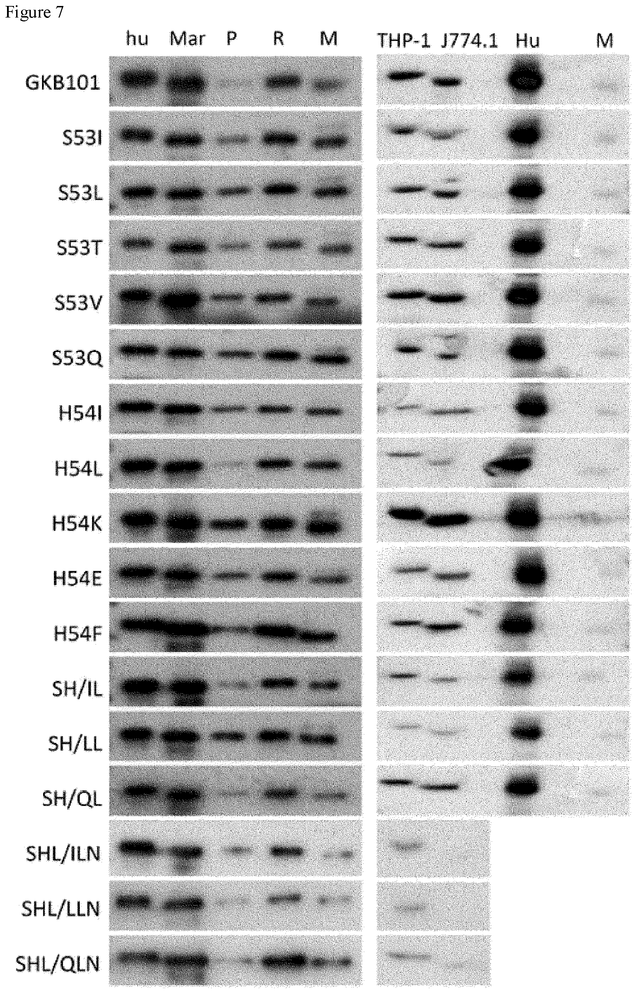

As a result, as shown in Figure 7, the GKB101 antibody and a variant antibody thereof of the present invention were able to specifically detect WARS1 in recombinant proteins, the culture supernatant of THP-1 cells or J774A.1 cells, and human or mouse serum. In particular, the S53I, S53L, S53V, S53Q, H54I, H54K, H54Q, H54F, and SH/LL antibodies were able to detect an equivalent or a higher level of pig-derived recombinant proteins compared to the GKB101 antibody.

## Example 4. Epitope mapping

A peptide corresponding to the human WARS 1 protein WHEP domain sequence of the anti-WARS1 antibody prepared in the present invention and an alanine mutant human WARS 1 protein were prepared to identify the binding ability of the anti-WARS1 antibody thereto through ELISA.

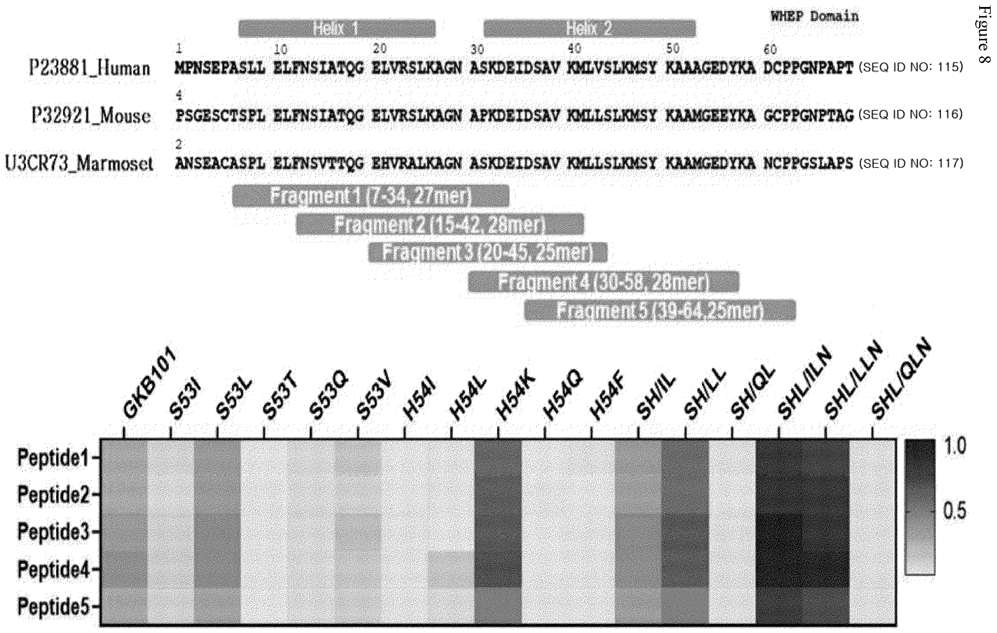

Specifically, for epitope mapping using the peptide, a 96-well plate (Maxisorp, Thermo Fisher) was coated with the anti-WARS1 antibody at a concentration of 1 $\mu$g/ml, and then blocked with PBS containing 1% BSA at room temperature for 1 hour. Each of the biotin-labeled peptides was diluted in a concentration-dependent manner and added to each coated well and reacted at room temperature for 1 hour, then washed with PBST (PBS+ Tween20) four times. Each well was treated with streptavidin (R&D systems) to which HRP is attached, reacted for 1 hour, and then washed with PBST. Lastly, after developing with a TMB substrate (BD), the absorbance was measured at 490 nm (NIVO) (Figure 8).

As a result, S53L, H54K, SH/IL, SH/LL, SHL/ILN, and SHL/LNN among the GKB101 antibodies and the variant antibodies thereof displayed a strong binding ability to all five peptides, and among which, H54K, SH/QL, SHL/ILN, and SHL/QLN displayed a binding ability to the WHEP domain sequence peptide of human WARS 1 that is equivalent to or higher than to GKB101.

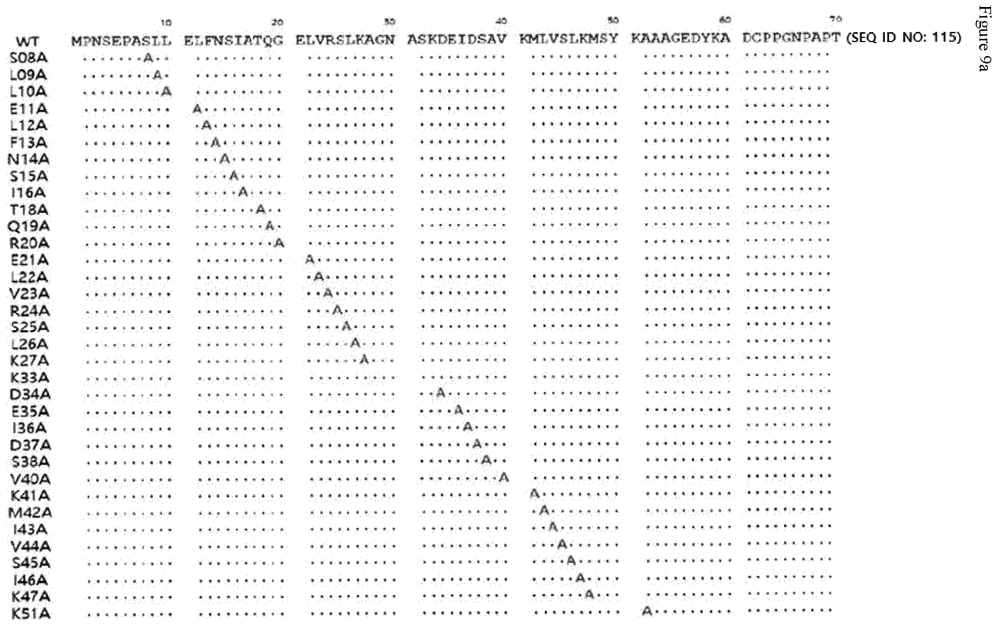

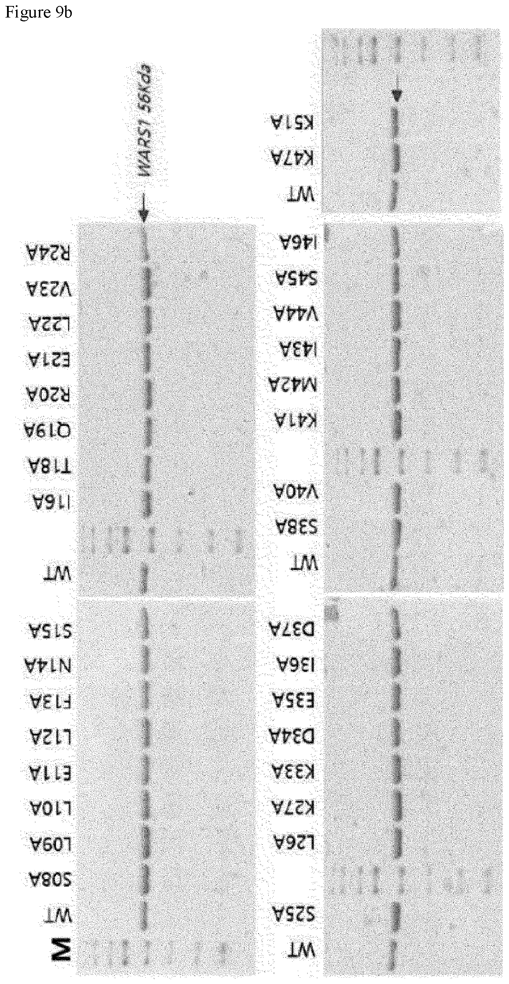

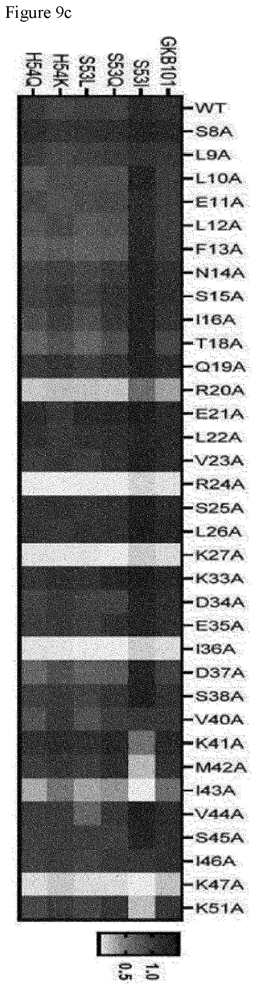

For epitope mapping using a human recombinant WARS1 protein in which a specific amino acid at the WHEP domain site is substituted to alanine, a 96-well plate (Maxisorp, Thermo Fisher) was coated with the anti-WARS 1 antibody at a concentration of 1 $\mu$g/ml, then blocked with PBS containing 1% BSA at room temperature for 1 hour. Each WARS1 protein was diluted to 50 ng/ml to be added to each well, reacted at room temperature for 1 hour, and then washed with PBST (PBS+ Tween20) four times. Each well was reacted with an anti-His tag antibody (Santa cruz) for 1 hour. After washing with PBST (PBS+ Tween20), it was reacted with an antibody (anti-mouse IgG, Santa cruz) to which HRP is attached for 1 hour, then after developing with a TMB substrate (BD), its absorbance was measured at 490 nm (NIVO) (Figures 9a to 9c).

As a result, it could be identified that the sequence R20, R24, K27, I36, I43, and K47 sites in the WHEP domain of the human WARS1 are all epitopes in GKB101, S53I, S53Q, S53I, H54K, and H54Q antibodies.

## Example 5. Identification of the neutralizing ability of the anti-WARS1 antibody to WARS1 *in vitro*

The ability of the anti-WARS 1 antibody of the present invention prepared in the same manner as Examples 1 and 2 of neutralizing WARS1 was identified *in vitro*.

Specifically, mouse macrophage cell line J774A.1 cells were inoculated in a 96-well culture plate (Nunc) at $4 \times 10^4$ cells/well and cultured for 24 hours. At this time, the cells were cultured using high glucose DMEM (Gibco) containing 10% FBS and 1% penicillin-streptomycin. The antigen-antibody mixture was prepared by mixing a human-derived WARS 1 recombinant protein (5 μg) and the anti-WARS 1 antibody of the present invention (5 μg) and reacting it in an incubator for 2 hours at 37 °C.

2 hours before treating the cells with the antigen-antibody mixture, the cell medium was replaced with a new one, and then the antigen-antibody mixture prepared using the method above was treated with the cells. After about 15 hours, the culture medium of each cell was centrifuged to collect the supernatant, and then the concentration of TNF-$\alpha$ in the supernatant was measured (ELISA MAX™ Standard Set Mouse TNF-$\alpha$, BioLegend). At this time, IgG was used as a control for the anti-WARS 1 antibody.

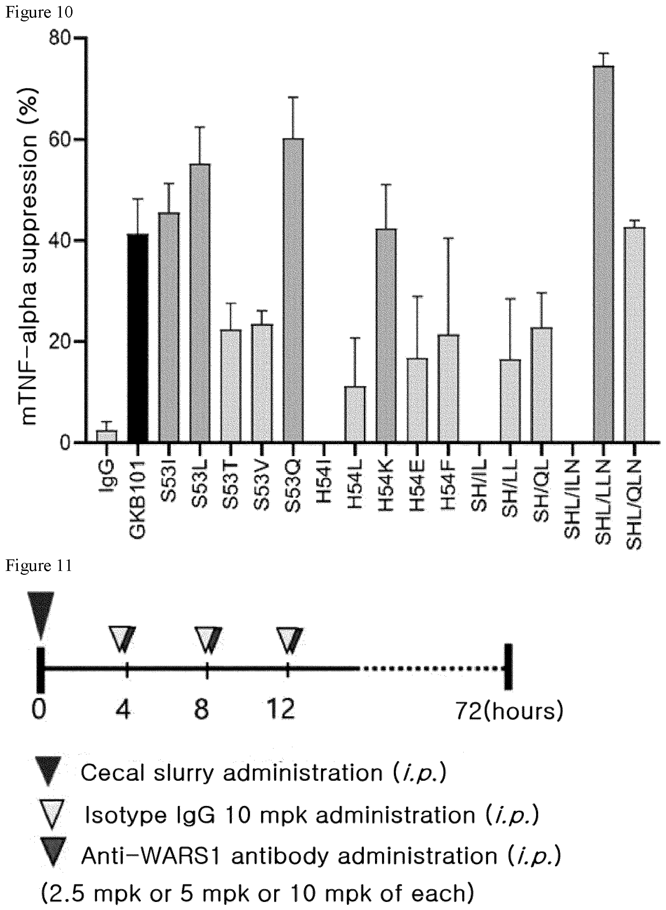

As a result, 7 antibodies (GKB101, S53I, S53L, S53Q, H54K, SHL/LLN, and SHL/QLN antibodies) among the 17 types of anti-WARS 1 antibodies according to the present invention suppressed 40% or more of the secretion of TNF-$\alpha$ increased by WARS1 in mouse macrophages. In addition, the S53I, S53L, S53Q, and SHL/LLN antibodies displayed an ability of neutralizing WARS1 that is equivalent to or higher than that of the GKB101 antibody (Figure 10).

## Example 6. Identification of the activity of the anti-WARS1 antibody *in vivo*

To identify the activity of the anti-WARS 1 antibody of the present invention at an *in vivo* level, mice in which severe sepsis was induced by injecting of CS (cecal slurry) were used.

Specifically, 21 mg of CS (cecal slurry) was injected into mice (C57BL/6, Daehan Biolink, 8-weeks-old) and an Isotype IgG1, a GKB101 antibody, an S53I antibody, an S53Q or an S53K antibody were each intraperitoneally administered three times in total at concentrations of 2.5 mg/kg, 5 mg/kg, or 10 mg/kg after 4, 8, and 12 hours (Figure 11). Afterwards, the survival of the mice was observed for up to 72 hours.

As a result of evaluating the efficacy of S53I, S53Q, and H54K using a mouse model of severe sepsis, S53I did not show a pattern of increased survival in a dose-dependent manner, whereas S53Q and H54K antibodies had a significantly increased survival rate at concentrations of 2.5 mg/kg and 10 mg/kg.

Below are the examples to identify the effect of the anti-WARS 1 antibody in improving inflammatory diseases using H54K, which is a variant of the anti-WARS 1 antibody. Therefore, anti-WARS1 antibodies, H54K, and M105A may be used interchangeably in the examples below.

## II. Evaluation of the efficacy of the anti-WARS1 antibody in suppressing respiratory damage using a mouse model of chronic obstructive pulmonary disease

## Example 7. Identification of the anti-inflammatory activity of the anti-WARS1 antibody in a cell line related to chronic obstructive pulmonary disease

To identify the effect of the anti-WARS1 antibody (H54K) in treating chronic obstructive pulmonary disease, the anti-inflammatory activity of the anti-WARS 1 antibody was identified using the cells involved.

At this time, human lung fibroblast cell lines (HLF, ATCC, PCS-201-013) were used as the cells, which were cultured in a DMEM containing 10% FBS and 1% antibiotic/antifungal agent.

Specifically, the cell lines were inoculated into 96-well plates at $2 \times 10^4$ cells/well to be cultured for 24 hours in a DMEM containing 0.1% FBS. At this time, WARS1 (10 μg/ml) and the anti-WARS 1 antibody (3C6, GKB101, H54K, 5 μg/ml) or PBS were mixed to be reacted for 1 hour at 37 °C. The WARS 1/anti-WARS1 antibody mixture was treated in the prepared cells and cultured for an additional 16 hours. After 16 hours, the culture medium was used to measure the concentration of IL-8 (BioLegend, 430515) and IL-6 (BioLegend, 431304) in the culture medium using ELISA. At this time, WARS1 treated with PBS was used as the control.

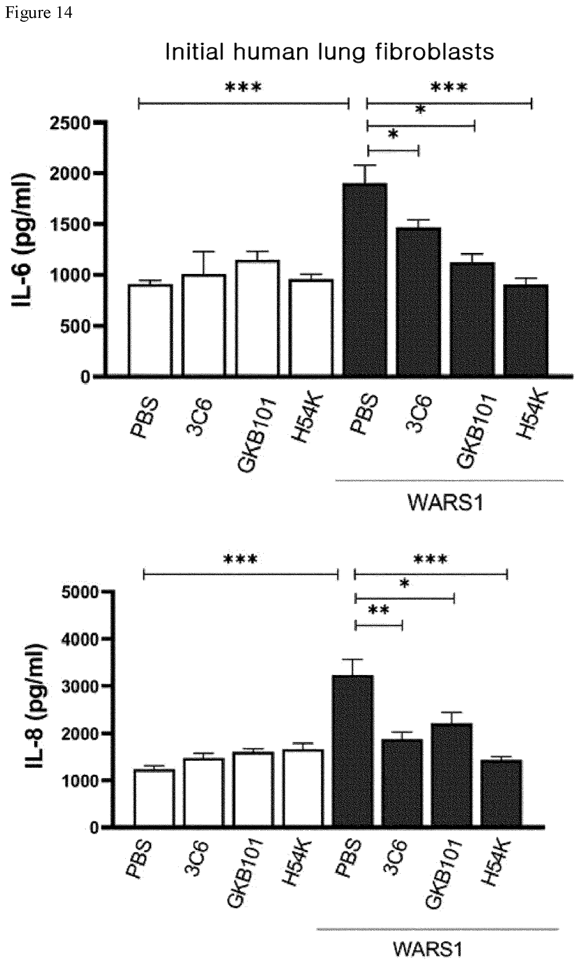

As a result, as shown in Figure 14, the secretion of IL-8 and IL-6 was increased in human lung fibroblasts in the WARS 1-only-treated group (control). On the other hand, in the WARS1 and the anti-WARS1 antibody-treated group, the secretion of IL-8 and IL-6 had decreased in a concentration-dependent manner compared to the control. In particular, the greatest decrease was identified in the H54K antibody-treated group.

## Example 8. Preparation of standard tobacco extract

As experimental materials, 60 standard CM7 (coresta monitoring cigarette 7) cigarettes and isopropanol (Merck), ethanol (Merck), and n-heptadecane (Sigma-Aldrich) were prepared, and an automatic smoking machine (ISO3308 regulated product, Model: RM20, Heinr Borgwaldt) was used as the experimental equipment.

## Example 8.1. Collection of standard cigarette smoke

The collection of standard cigarettes CM7 smoke condensate was conducted in a smoking room (temperature 22 $\pm$2 °C, relative humidity 60$\pm$5%) according to ISO3402 regulations. According to ISO3308 regulations, the RM20 (Heinr Borgwaldt) automatic smoking device was used to burn cigarettes using a smoking volume of 35.0$\pm$0.3 ml, a smoking cycle of 60$\pm$0.5 seconds, a smoking time of 2.00$\pm$0.02 seconds, a tip paper length of +3 mm (overwrap +3 mm), and an ISO regulated smoking method, and the cigarette smoke condensate was collected using a 92 mm Cambridge filter (ISO3308 regulated product).

## Example 8.2. Measurement of cigarette smoke condensate weight

Before smoking, the weight of the cigarette holder containing a Cambridge filter was measured before burning according to ISO4387 regulations, the cigarette smoke condensate was burned using the method of Example 8.1, and then the TPM (total particulate matter) content was calculated by measuring the weight of the cigarette holder in which cigarette smoke had been collected by a Cambridge filter (ISO4387, 2000). When a standard cigarette was burned three times, the TPM weight was 16.0621 mg for 19 cigarettes, 15.9135 mg for 20 cigarettes, and 15.5380 mg/cig for 20 cigarettes. Therefore, the total number of standard cigarette samples was 59 cigarettes and the TPM weight was 47.5136 mg.

## Example 8.3. Extraction of the standard cigarette smoke condensate

The Cambridge filter in which the cigarette smoke condensate was collected using the method of Example 8.1 was separated from the cigarette holder and each placed into a 100 ml Erlenmeyer flask, then as an extraction solvent, 50 ml of isopropanol was added and mixed. The mixture was left at room temperature for more than 8 hours to extract the cigarette smoke condensate. The extract was filtered after extraction and concentrated using a vacuum filtration concentrator, and then the concentrates in three Erlenmeyer flasks were collected in a scintillation vial and completely concentrated using nitrogen gas.

The total particulate matter (TPM) content was calculated from the cigarette smoke condensate concentrated by the method above using Equation 1 below.

$$\textbf{<Equation 1>}$$
$$\text{TPM(mg/cig)} = (W_{FHA} - W_{FBH})/N$$

wherein TPM: Total particulate matter
wherein $W_{FHA}$: Weight of the filter holder after smoking
wherein $W_{FHB}$: Weight of the filter holder before smoking, and
wherein N: Number of cigarettes smoked per trap (cig.).

## Example 9. Verification of the effect of the anti-WARS1 antibody in suppressing respiratory damage using a mouse model of chronic obstructive pulmonary disease

### Example 9.1. Preparation of a mouse model of chronic obstructive pulmonary disease

7-week-old male specific pathogen-free (SPF) BALB/c mice (18 g to 20 g) were supplied by Orient Bio. The animals were provided with sufficient solid feed (no antibiotics, Samyang Feed Co.) and water until the day of the experiment, and were acclimatized for one week in an environment having a temperature of 22$\pm$2 °C, humidity of 55 $\pm$15%, and 12 hours (light-dark cycles) before use in the experiment. Also, approval of the Institutional Animal Care and Use Committee (IACUC) of Daejeon University was obtained for review and efficient management of the ethical and scientific validity of the animal experiments.

The LPS/CS mixture for establishing a mouse model of chronic obstructive pulmonary disease (COPD) was prepared by mixing 100 μg/ml of lipopolysaccharide (LPS) and 4 mg of a standard cigarette smoking extract (CSE) at a ratio of 1:1. After temporarily anesthetizing 7-week-old BALB/c male mice with ketamine/rompun, a total of 100 μl (50 μl each) of the LPS/CS mixture was inhaled into the nose and mouth of the mice once a week for 3 weeks using an INT (intra-nasal-trachea) injection method to produce a COPD mouse model.

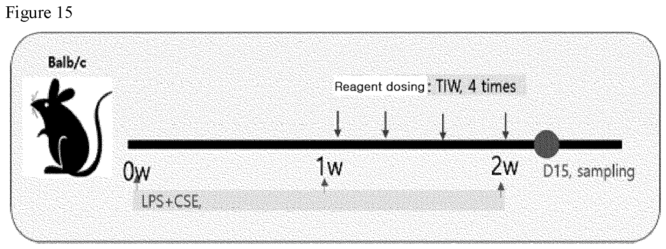

At this time, the experimental groups were divided into (i) an untreated normal group, (ii) PBS (control, CON), (iii) an Isotype IgG1 antibody (3 mg/kg, i.p) administered group, and (iv) an anti-WARS1 antibody (H54K) administered group. At this time, each sample was administered 1 hour before administering LPS and CS (Figure 15). Hereinafter, the H54K antibody, which is the anti-WARS 1 antibody of the present invention, may be interchangeably described with the M105A antibody.

After the experiment, the blood, bronchoalveolar lavage fluid, and lung tissue of the mice in each group were separated to evaluate the effect of the anti-WARS 1 antibody (H54K) in suppressing respiratory damage. At this time, the result of each experimental group was shown as a mean±standard deviation (SEM), and the significance of the numerical data between the experimental groups was verified using a Duncan's multiple comparison test after a one-way analysis of variance (ANOVA).

**Example 9.2. Pathological analysis of lung tissue**

The tissue morphology of the airway and lung tissue of the experimental mice in Example 9.1 was analyzed through observation under an optical microscope after staining each using methods of H&E staining, Masson Trichrome staining (M-T staining), or Periodic acid-Schiff (PAS)-Alcian Blue (AB) staining.

**Example 9.2.1. Observation of inflammatory immune cell infiltration in the airway and lung tissue through H&E staining**

Specifically, after completion of administration, the extracted airway tissue and lung tissue were fixed by reacting in 4% paraformaldehyde (PFA) for one day to produce paraffin tissue section slides. The paraffin tissue section slides were each washed three times with xylene solution and 100% ethanol to remove paraffin.

The slides from which paraffin was removed was observed using the method below. After reacting the slides in hematoxylin solution for 5 minutes, the slides were sequentially washed in running water and 1% hydrochloric acid. Next, the slides were washed in running water then neutralized with 1% ammonia solution (diluted in 70% ethanol). The slides were washed in running water and reacted in an eosin solution for 2 minutes. After the reaction had completed, the slides were sequentially washed with 70% to 100% ethanol solution and reacted in xylene solution for 3 minutes. At this time, the reaction process in the xylene solution was performed three times in total. Finally, the tissue section slides were encapsulated with cover slides and the shape of the tissue was observed under an optical microscope.

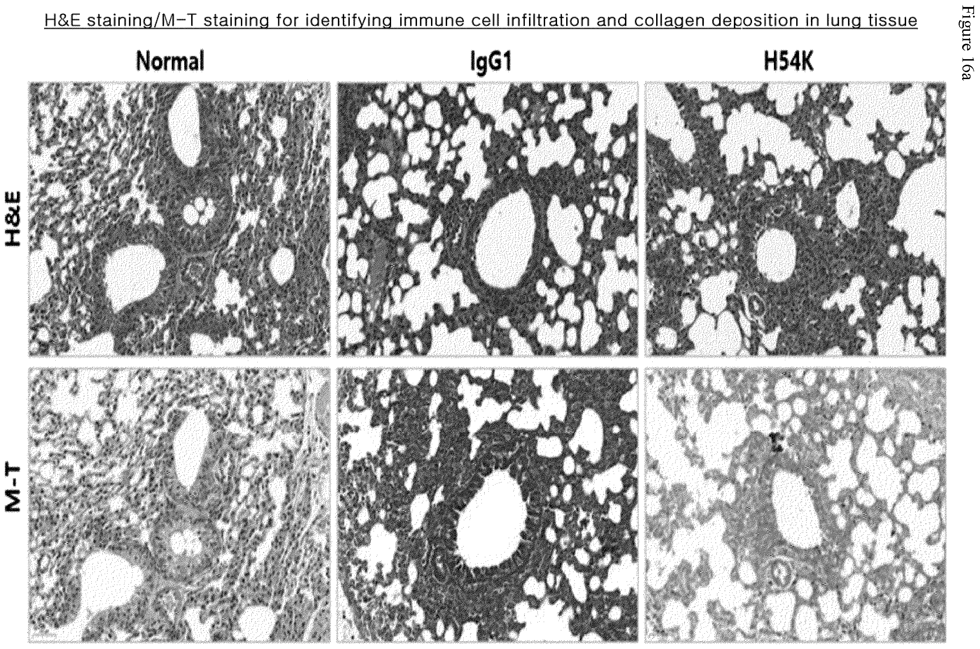

As a result, as shown in Figure 16a, the infiltration of inflammatory immune cells around the respiratory tract in the IgG1-administered group was observed compared to the normal group. In addition, the progress of the destruction of alveolar cells due to worsening inflammation was also observed. On the other hand, it was identified that the infiltration of inflammatory immune cells had decreased, and alveolar cells were maintained in the anti-WARS1 antibody (H54) administered group.

**Example 9.2.2. Observation of collagen deposition in lung tissue through Masson Trichrome staining**

Masson Trichrome (M-T) staining was performed on the lung tissue slides prepared in the same manner as Example 9.2.1.

Specifically, the slides from which paraffin was removed were reacted in Bouin's solution at 60 °C, then taken out and cooled, and sequentially reacted in Weigert's Hematoxylin solution, Briebrich Scarlet solution, Phosphotungstic phosphomolybdic acid, and Aniline blue solution. Finally, after reacting in 1% acetic acid solution, the lung tissue section slides were encapsulated with cover slides and the degree of collagen deposition in the tissue was identified under an optical microscope.

As a result, as shown in Figure 16a, a high level of collagen deposition was observed around the tracheole and alveolar cells in the IgG1-administered group. On the other hand, it was identified that collagen deposition had decreased in the anti-WARS1 antibody (H54) administered group.

**Example 9.2.3. Observation of mucus secretion in the airway tissue through Periodic acid-Schiff-Alcian Blue staining**

Tissue slides prepared in the same manner as Example 9.2.1 using the airway tissue extracted from the experimental mice in Example 9.1 were stained through a Periodic acid-Schiff-Alcian Blue (PAS-AB) staining method using Periodic Acid Schiff (Sigma Aldrich, 395B).

Specifically, slides from which paraffin was removed was sequentially reacted in Alcian blue solution, 0.5% Periocid acid solution, and Schiff's solution. After reacting with each solution, the slides were thoroughly washed in running water. The slides stained through the process above were reacted in a hematoxylin solution, then washed with water and dehydrated by sequentially reacting in an ethanol solution with a concentration of 70% to 100%. Finally, the tissue sections were encapsulated with a cover glass and observed under an optical microscope.

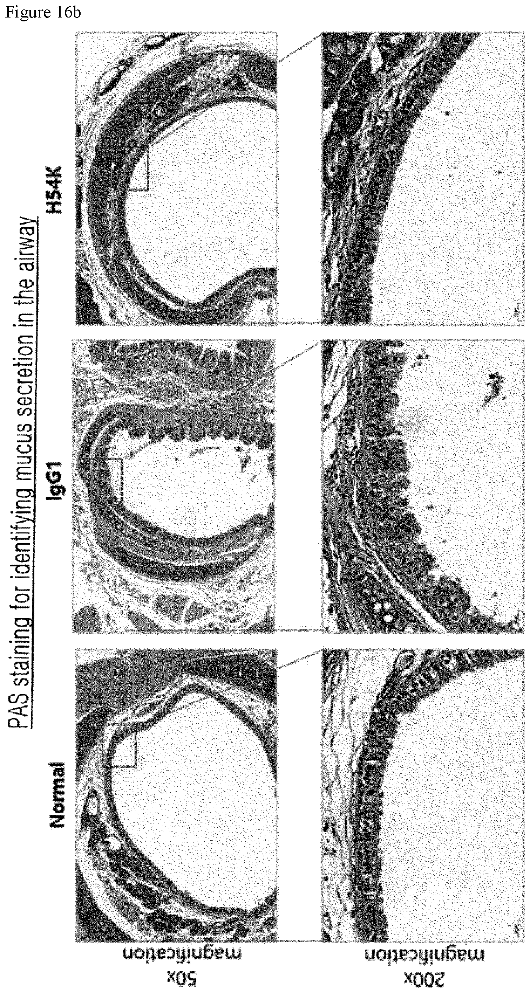

As a result, as shown in Figure 16b, it was observed that the amount of mucin in goblet cells had decreased around the airway in the anti-WARS 1 antibody (H54K) administered group compared to the IgG1-administered group.

**Example 9.3. Analysis of inflammatory cytokine concentrations in the airway, alveolar lavage fluid, lung tissue, and serum**

The concentrations of inflammatory cytokines and chemokines were measured in the lung tissue obtained from the experimental mice in Example 9.1.

At this time, collection of each sample was performed as below.

First, alveolar lavage fluid was collected after washing the bronchial tubes and lung tissue with 1 ml of PBS by intubating the airway of the mice. The lavage fluid was used as bronchoalveolar lavage (BAL) fluid (BALF).

In addition, a portion of the lung tissue was extracted, and the tissue was dissolved by adding a protein lysis buffer for the concentration to become 100 $\mu$g/ml. The lysate was centrifuged at 13,000 rpm, and only the supernatant was collected and used as a lung tissue sample.

Serum samples were obtained by collecting blood through cardiac puncture, and then transferring it to a heparin tube, and centrifuging it at 2,000 rpm for 20 minutes at room temperature.

To measure the concentrations of mCXCL2/MIP-2, mIL-6, and mMIP1$\alpha$ in the obtained samples, mouse CXCL2/MIP-2 ELISA (R&D systems), mouse IL-6 ELISA (BioLegend), and mouse MIP1-$\alpha$ ELISA (R&D systems) kits were used to measure the concentrations of cytokines or chemokines of each.

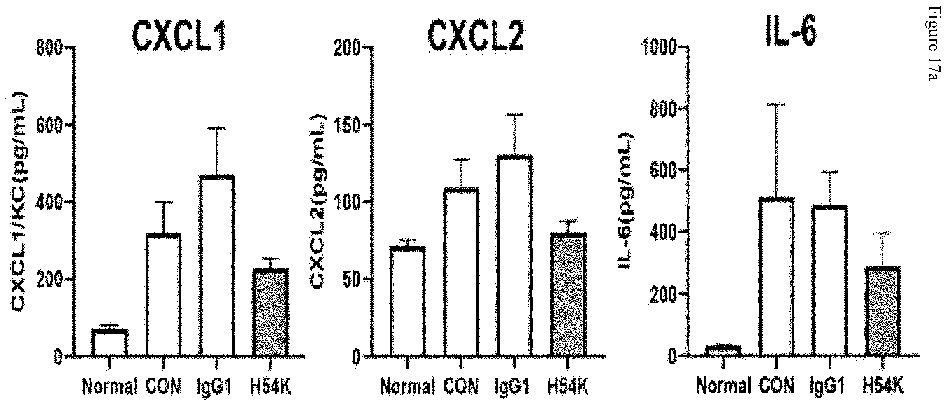

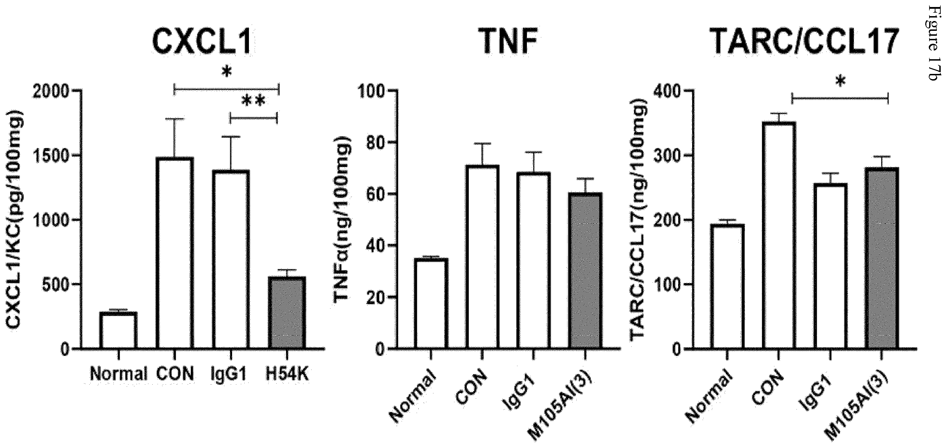

As a result, as shown in Figures 17a (alveolar lavage fluid) and Figure 17b (lung tissue), the CXCL1, CXCL2, IL-6, TNF-$\alpha$, and TARC had increased in the Isotype IgG1-administered group or PBS-administered group compared to the normal group. On the contrast, in the anti-WARS1 antibody (H54K) administered group, a decrease of about 1/4 to about 1/2 was observed compared to the Isotype IgG1-administered group or PBS-administered group. All data was expressed as standard deviation (SEM) and analyzed using the ANOVA statistical program.

In addition, to identify gene expression, portion of the lung tissue was extracted, Trizol (Invitrogen, 15596026) was added, and then mRNA was extracted according to the manufacturer's protocol. The mRNA was used as a template to synthesize the cDNA using a cDNA EcoDry Premix (Random Hexamers) kit (Takara, 639546) and identify the expression of each gene through q-PCR using the SYBR Green real time PCR master mix (Applied biosystems, 4309155). At this time, the primers used are shown in Table 21 below.

[Table 21]

| Gene Name | Forward primer (5' to 3') | Reverse primer (5' to 3') |
|---|---|---|
| MUC5A C | AGAATATCTTTCAGGACCCCTGCT (SEQ ID NO: 146) | ACACCAGTGCTGAGCATACTTT (SEQ ID NO: 147) |
| TARC | TCCAGGGCAAGCTCATCTGT (SEQ ID NO: 148) | AATGCCTCAGCGGGAAGGTC (SEQ ID NO: 149) |
| TRPA1 | TGAGATCGACCGGAGT (SEQ ID NO: 150) | TGCTGAAGGCATCTT (SEQ ID NO: 151) |
| Eotaxin | TGCTGAAGGCATCTTG (SEQ ID NO: 152) | AATATCAGCACCAGTCGCCC (SEQ ID NO: 153) |

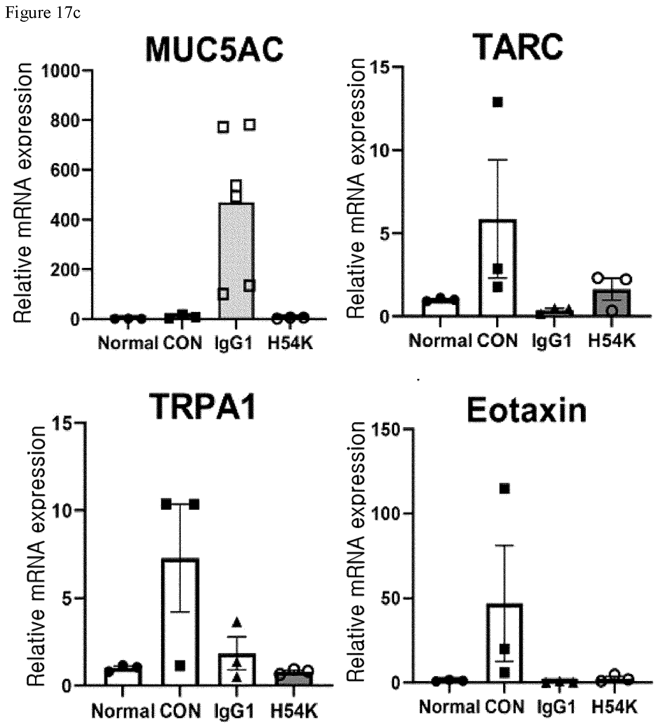

As a result, as shown in Figure 17c, it was identified that the expression of cough-inducing genes (MUC5AC, TRPA1, TRPV1) and eotaxin, which is an eosinophil inflammatory gene, had significantly increased in the PBS-administered group compared to the normal group. On the other hand, in the anti-WARS 1 antibody (H54K) administered group, it was identified that the expression of the gene had decreased compared to the PBS-administered group or the Isotype IgG1-administered group.

Through the results above, it was identified that the anti-WARS 1 antibody (H54K) can suppress the expression of inflammatory cytokines, chemokines, cough-inducing genes, and eosinophil inflammatory genes in COPD model mice.

**Example 9.4. Analysis of immune cells in the airways, alveolar lavage fluid, lung tissue, and serum**

The immune cells in the serum, alveolar lavage fluid, and lung tissue obtained from the experimental mice in Example 9.1 were analyzed through flow cytometry.

At this time, alveolar lavage fluid was obtained in the same manner as Example 9.3.

The lung tissue sample was prepared using the method below. First, a portion of the lung tissue was mixed with a collagenase solution at a concentration of 1 mg/ml and disassembled using the C-tube of GentleMacs, then incubated with shaking at 37 °C for 30 minutes. The shaken culture solution was passed through a 70 $\mu$m cell strainer to collect single cells.

Immunofluorescence staining was performed on the lung tissue sample prepared as above and the cells obtained from the alveolar lavage fluid. The antibodies used at this time were a PE-anti-CD3e antibody (553064, BD Pharmingen), a FITC-anti-CD8 antibody (553031, BD Pharmingen), a PE-anti-CD4 antibody (553047, BD Pharmingen), a PE-anti-Gr-1 antibody (553128, BD Pharmingen), a FITC-anti-CD69 antibody (55732, BD Pharmingen), a FITC-anti-CD11b antibody (553310, BD Pharmingen), and a PE-anti-B220 antibody (561878, BD Pharmingen). Each cell was treated with the antibodies and incubated in the dark at 4 °C for 30 minutes. After the reaction, the cells were washed with a FACS buffer (0.5% FBS, 0.02% NaN$_3$ in PBS) three times or more, the cells were analyzed using a flow cytometer. The analysis values were CD3+, CD4+, CD8+, CD4+CD69+, CD8+CD69+, CD4+CD62L-CD44+high, Gr-1+SiglecF-(neutrophils), CD11b+Gr-1+(granulocytes), Gr-1-SiglecF+(eosinophils), and Gr-1+highSiglecF+(eosinophil_high) as analyzed in the FlowJo program (FlowJo™ v7 Software, BD BioSciences), and each immune cell was classified and the distribution (%) of each cell was analyzed.

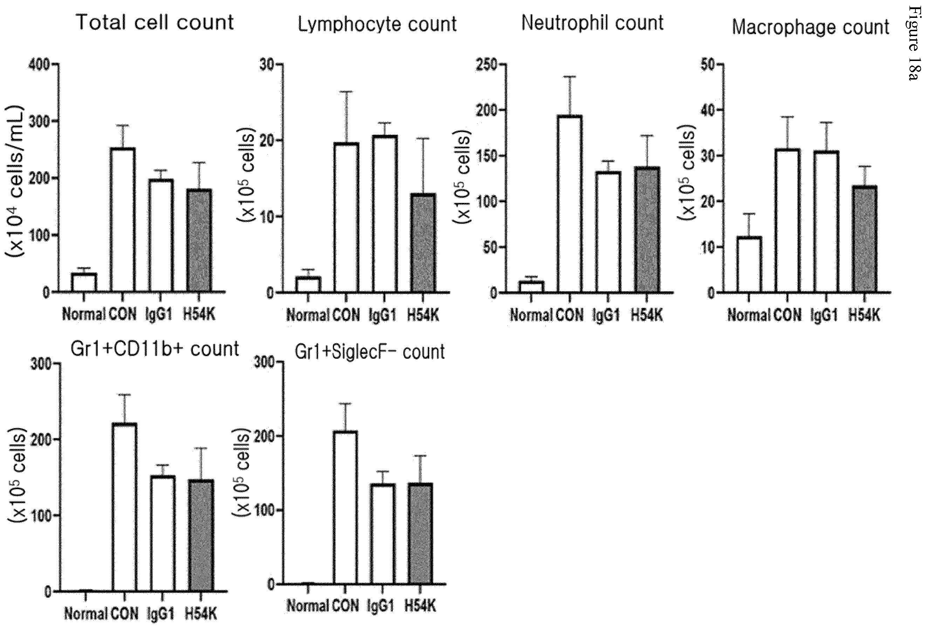

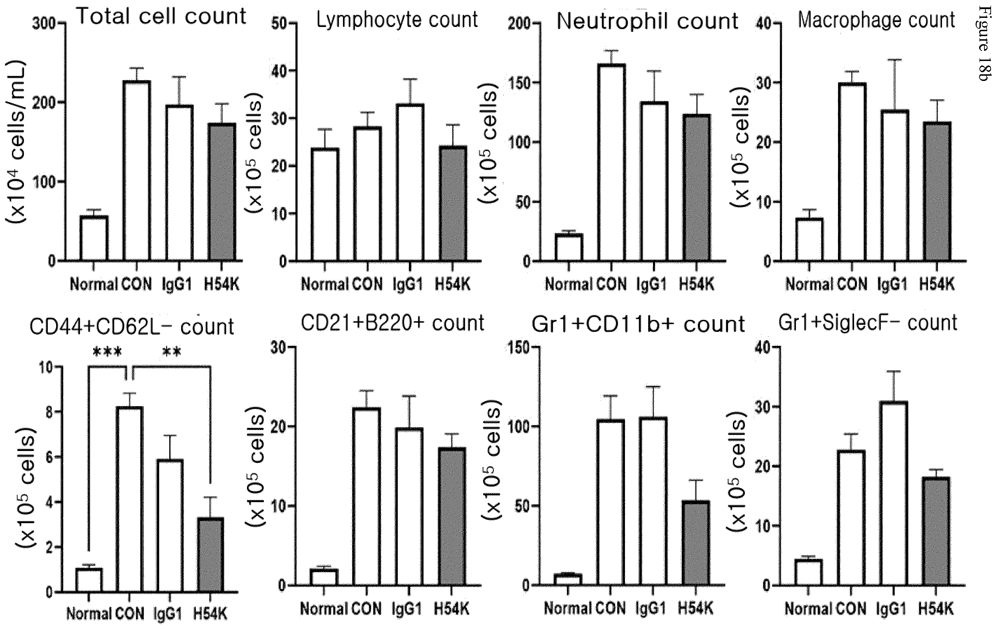

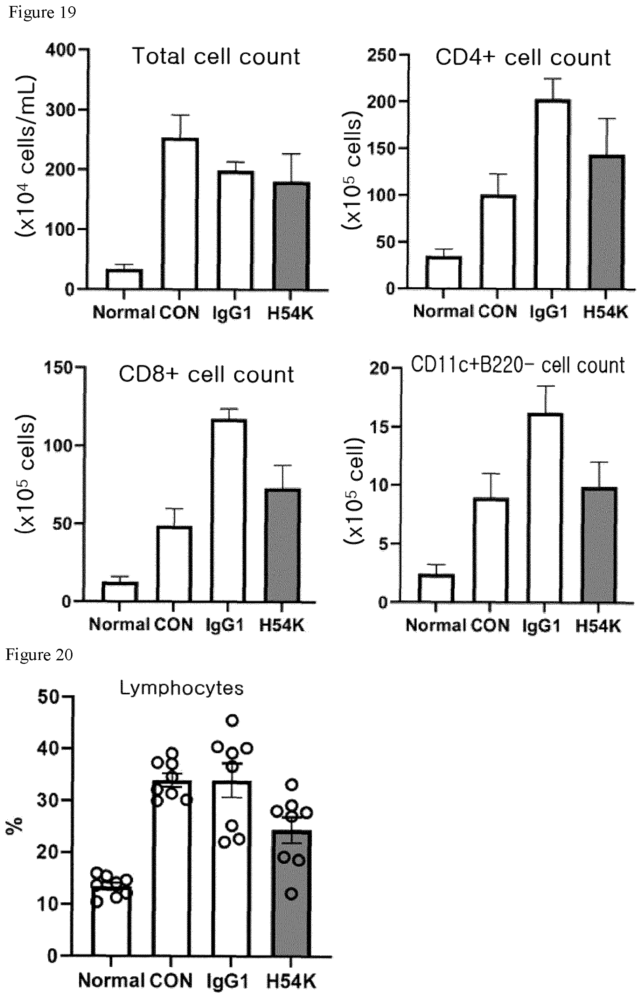

As a result, as shown in Figures 18a, 18b, and 20, the total number of cells infiltrated in the Isotype IgG1-administered group or the PBS-administered group and the number of lymphocytes (especially B lymphocytes, activated T lymphocytes), neutrophils, macrophages, and granulocytes had increased by approximately 3 to 100 times compared to the normal group. On the other hand, a decrease of up to about 1/2 was observed in the anti-WARS1 antibody (H54K) administered group compared to the Isotype IgG1-administered group or the PBS-administered group. All data was expressed as standard deviation (SEM) and analyzed using the ANOVA statistical program.

**Example 9.5. Analysis of immune cells in mediastinal lymph nodes**

Immune cells in the mediastinal lymph node obtained in the mouse experiment in Example 9.1 were analyzed in the same manner as Example 9.4.

Mediastinal lymph node samples were prepared as below. First, the chest was opened, and the mediastinal lymph nodes located in the superior vena cava near the thymus were removed using scissors and forceps, then passed through a 70 $\mu$m cell strainer to collect single cells.

Immunofluorescence staining was performed on the mediastinal lymph node cells prepared as above using the same antibody and method in Example 9.4. The flow cytometry values were CD3+, CD4+, CD8+, CD4+CD69+, CD8+CD69+, and CD11c+B220- as analyzed using the FlowJo program (FlowJo™ v7 Software, BD BioSciences), and each immune cell was classified and the distribution (%) of each cell was analyzed.

As a result, as shown in Figure 19, an increase in the total number of infiltrated cells was identified in the Isotype IgG1-administered group or the PBS-administered group compared to the normal group. In particular, CD4+T lymphocytes and CD8+T lymphocytes had increased by 5 times or more and activated in the IgG1-administered group. On the other hand, a decrease of about 1/2 or more was observed in the Isotype IgG1-administered group or the PBS-administered group in the anti-WARS1 antibody (H54K) administered group. All data was expressed as standard deviation (SEM) and analyzed using the ANOVA statistical program.

**Example 9.6. Analysis of immune cells in the blood**

The immune cells in the peripheral blood mononuclear cells (PBMC) obtained from the mouse experiment in Example 9.1 were analyzed in the same manner as Example 9.4. At this time, the PBMC sample was prepared by opening the chest, obtaining blood from the ventricle using a heparin (20 to 30 $\mu$l) syringe, then mixing the blood with an ACK solution (Thermos Fisher, A1049201) to remove red blood cells.

Immunofluorescence staining was performed on the immune cells in the PBMC prepared as above. At this time, a PE-anti-CD3e antibody (553064, BD Pharmingen), a FITC-anti-CD8 antibody (553031, BD Pharmingen), a PE-anti-CD4 antibody (553047, BD Pharmingen), a PE-anti-Gr-1 antibody (553128, BD Pharmingen), a FITC-anti-CD19 antibody (553785, BD Pharmingen), and a PerCP Cy5-anti-SiglecF antibody (565526, BD Pharmingen) were used as the antibody. Each cell was treated with the antibody and reacted in the dark at 4 °C for 30 minutes. After the reaction, the cells were washed with a FACS buffer (0.5% FBS, 0.02% NaN$_3$ in PBS), then the cells were analyzed using a flow cytometer. The analysis values were CD3+, CD19+, CD4+, and Gr-1+SiglecF-(neutrophils) as analyzed in the FlowJo program (FlowJo™

v7 Software, BD BioSciences), and each immune cell was classified and the distribution (%) of each cell was analyzed.

As a result, as shown in Figure 20, the distribution of lymphocytes in the blood had increased in the Isotype IgG1-administered group or the PBS-administered group compared to the normal group. On the other hand, a decrease of approximately 30% was observed in the anti-WARS1 antibody (H54K) administered group compared to the PBS or the Isotype IgG1-administered group. All data was expressed as standard deviation (SEM) and analyzed using the ANOVA statistical program.

**III. Verification of the effect of the anti-WARS1 antibody in improving inflammatory bowel disease**

**Example 10. Verification of the effect of anti-WARS1 antibody treatment in improving intestinal permeability in colon cancer cell lines**

To identify the effect of the anti-WARS1 antibody (H54K) in treating inflammatory bowel disease, the effect of improving intestinal permeability was identified by treatment with the anti-WARS1 antibody using intestinal epidermal cells.

At this time, human intestinal epidermal cell lines (Caco-2, ATCC, HTB-37) were used as the cells. The cells were cultured in a DMEM medium containing 10% FBS and 1% antibiotic/antifungal agent.

Specifically, the cell lines were inoculated into 6-well plates at $3 \times 10^5$ cells/well to be cultured for 24 hours in a DMEM containing 0.1% FBS. At this time, WARS1 (10 μg/ml) and the anti-WARS1 antibody (3C6, GKB101, H54K, 5 μg/ml) or PBS were mixed to be reacted for 1 hour at 37 °C. The WARS1/anti-WARS1 antibody mixture was treated in the prepared cells and cultured for an additional 16 hours. After 16 hours, the culture medium was removed and fixed with 4% paraformaldehyde (PFA) at room temperature for 5 minutes. The fixed cells were treated with 0.1% TritonX-100 and reacted for 5 minutes to increase the cell permeability of the antibody. After blocking with 2% BSA for 1 hour, the cells were treated with the anti-human ZO-1 (Invitrogen, 1A12) antibody diluted in 1% BSA to 1:500, and reacted overnight at 4 °C. Afterwards, the cells were treated with the secondary antibody conjugated with FITC (Jackson ImmunoResearch, 200-002-037) diluted in 1% BSA to 1:1000, then reacted at room temperature for 1 hour. The intracellular nuclei were stained by being treated with DAPI (4'-6-diamidino-2-phenylindole, Sigma-Aldrich, D8417) (1 μl). The fluorescently stained cells were identified under a confocal microscope (LSM700 confocal laser scanning microscope, Carl Zeiss). In addition, the cell image was analyzed using the Zen 3.0 software (blue edition).

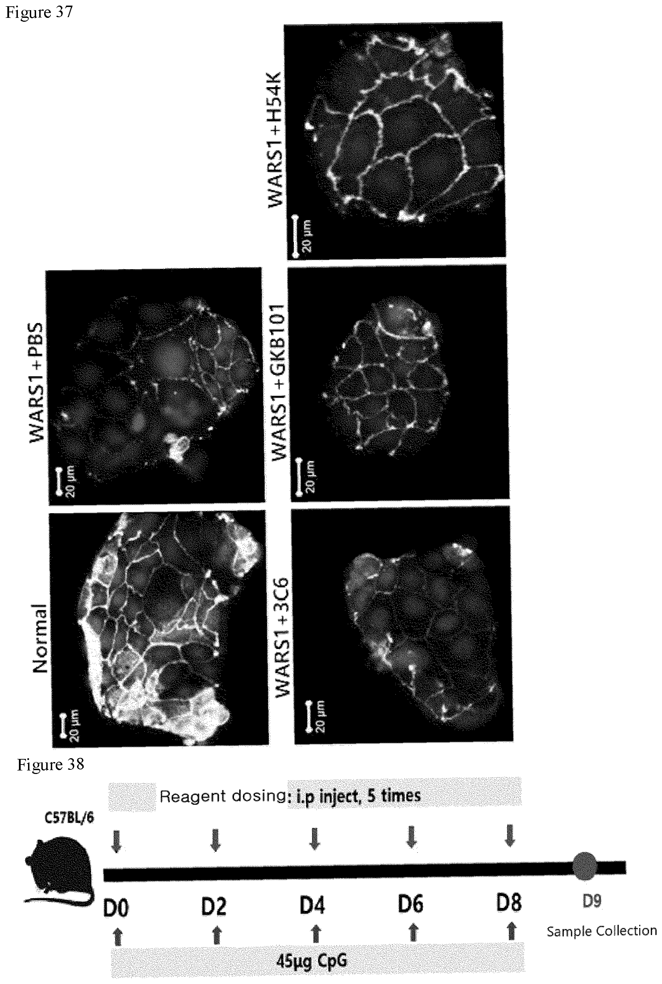

As a result, as shown in Figure 37, the expression of ZO-1 (tight junction) had decreased when the intestinal epithelial cells (Caco-2 cells) were treated with WARS1, but recovery was identified by treating with the anti-WARS1 antibody. In particular, the greatest recovery effect was observed in the H54K antibody-treated group.

Through this result, it was identified that intestinal permeability may be improved by the anti-WARS 1 antibody in inflammatory bowel disease.

**Example 11. Verification of the effect of the anti-WARS1 antibody in improving inflammatory bowel disease using a mouse model of inflammatory bowel disease**

**Example 11.1. Production of a mouse model of inflammatory bowel disease**

A DSS mouse model (dextran sulfate sodium-induced colitis model) was used as the mouse model of inflammatory bowel disease. 6 to 10-week-old female B57BL/6 mice (Orient Bio) were used, and they were raised in an SPF laboratory animal room maintained at a constant temperature of 21 to 23 °C and constant relative humidity of 40 to 45%. In addition, the number of experimental animals per cage was less than four, and the cages were replaced and feed was provided three times a week.

After dissolving DSS (MP biomedical, reagent-grade, MW 36-50 KDa, 160110) in sterilized water at a concentration of 2.5%, 100 ml of 2.5% DSS solution per mouse was supplied once every two days. Clinical tests were regularly performed until the end of the experiment to identify the progress of inflammation and to measure body weight. At this time, if the body weight decreased by more than 25% from the start date of the experiment, euthanasia was performed for ethical reasons. The experiment was ended at 7 to 8 days from the start of the experiment when it was determined that the clinical index of the control had reached its maximum.

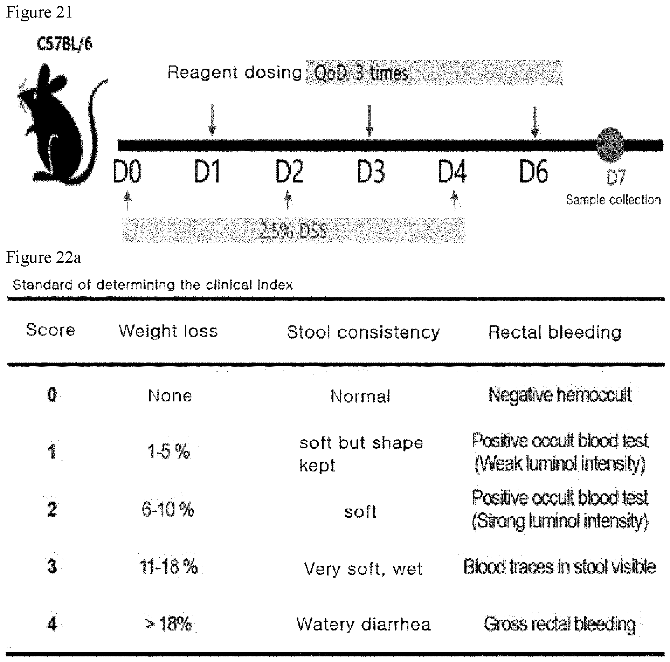

The test group was divided into four groups in total (Figure 21). The administration of DSS began from day 0, and the negative control material (vehicle, saline), Isotype IgG1 (5 mg/kg, Bio X cell, BP0297), and the anti-WARS1 antibody (GKB101, H54K, 5 pmk each) were each intraperitoneally administered from days 1, 3, and 5. At this time, saline, which was the vehicle, was orally administered.

From the start of the experiment to the end of the experiment, the occurrence and degree of inflammation were observed once a day to give a score of 0 to 4, and the sum was used as the disease activity index (DAI). In addition, body weight was measured once a day to give a score of 0 to 4, which was used to measure the DAI.

All data was expressed as standard deviation (SEM) and analyzed using the ANOVA statistical program.

**Example 11.2. Evaluation of the clinical index**

**Example 11.2.1. Evaluation of the disease activity index**

To identify the effect of the anti-WARS 1 antibody in improving inflammatory bowel disease in a DSS mouse model, the disease activity index (DAI) was quantitatively evaluated based on the occurrence of inflammatory symptoms and the change in body weight in the experimental mice of Example 11.1 (Figure 22a).

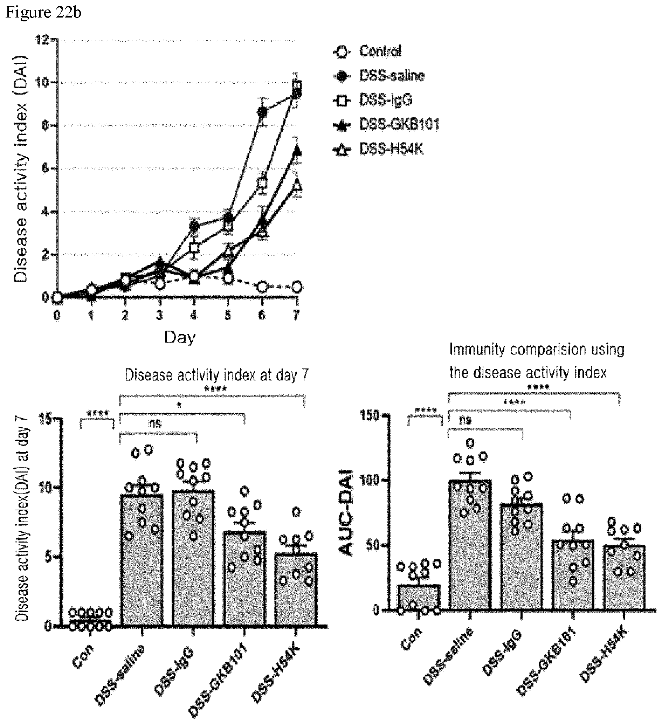

As a result, as shown in Figure 22b, the disease activity index had significantly decreased in the anti-WARS 1 antibody (GKB101, H54K) administered group compared to the control (saline), and in particular, had significantly decreased in the H54K-administered group (*p<0.05, ****p<0.0001).

The result of analyzing the AUC (area under the curve) of the clinical index also showed similar patterns as the result during the administration period of the clinical index.

Through the results above, it was identified that the anti-WARS1 antibody, especially H54K, exhibited an effect in improving inflammatory bowel disease.

**Example 11.2.2. Evaluation of body weight**

In addition, as a result of evaluating the body weight loss score, which was one of the items for DAI evaluation, weight loss was evident compared to the normal group (con), but when compared to the Isotype IgG1-administered group, there was no statistically significant difference in the anti-WARS 1 antibody-administered group.

**Example 11.2.3. Evaluation of rectal bleeding**

The rectal bleeding score was evaluated in the experimental mice in Example 11.1.

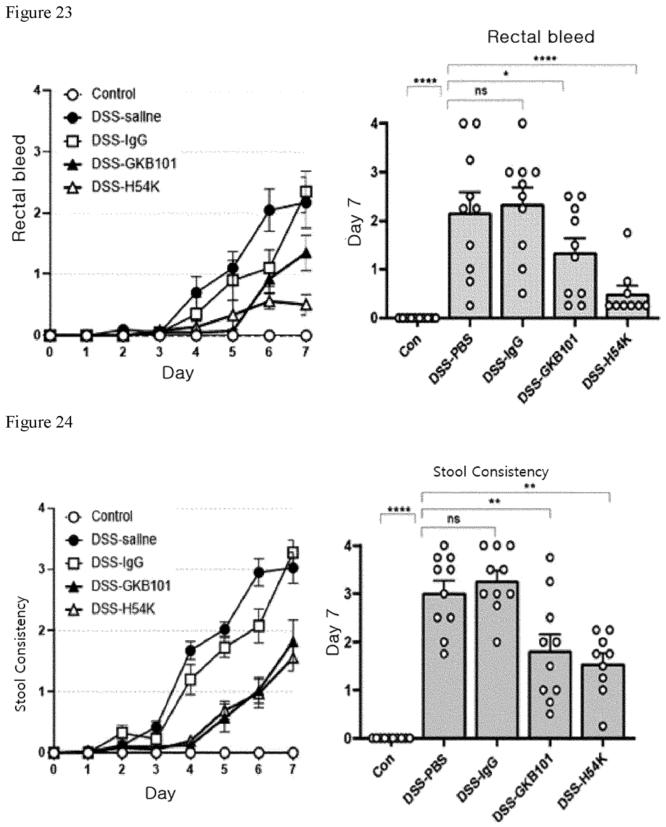

As a result, as shown in Figure 23, a significant decrease in rectal bleeding was identified in the anti-WARS 1 antibody (GKB101, H54K) administered group compared to the control (saline) (****p<0.0001). The result of analyzing the AUC of the rectal bleeding score also displayed a similar pattern as the result during the administration period.

**Example 11.2.4. Evaluation of the stool consistency score**

The stool consistency score was evaluated in the experimental mice in Example 11.1.

As a result, as shown in Figure 24, the stool consistency score had decreased in the anti-WARS1 antibody (GKB101, H54K) administered group compared to the control (saline), and in particular, a significant decrease was identified in the H54K-administered group (*p<0.05, **p<0.01, ***p<0.001, ****p<0.0001). The result of analyzing the AUC of the stool consistency score also displayed a similar pattern as the result during the administration period.

**Example 11.2.5. Measurement of the colon length**

Colonic tissue was extracted from the experimental mice in Example 11.1 and the colon length was measured.

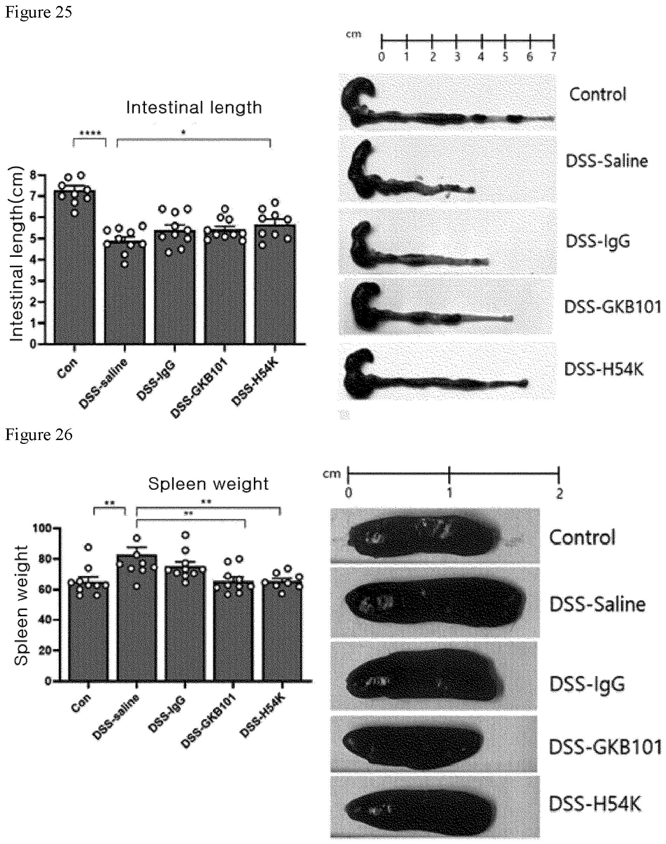

As a result, as shown in Figure 25, the colon length had decreased in the control (saline). On the other hand, damage to the colon had significantly decreased in the anti-WARS1 antibody (GKB101, H54K) administered group compared to the control, and in particular, a significant decrease was identified in the H54K-administered group (*p<0.05).

**Example 11.2.5. Evaluation of the spleen weight**

Spleen tissue was removed from the experimental mice in Example 11.1 and its weight was measured.

As a result, as shown in Figure 26, the spleen weight had increased in the control (PBS) compared to the untreated group. On the other hand, a significant decrease in the spleen weight was identified in the anti-WARS1 antibody (H54K) administered group (**p<0.01).

**Example 11.3. Identification of the immune factor expression in intestinal tissue**

Intestinal tissue was extracted from each region of the experimental mice in Example 11.1, protein and mRNA were separated, and the expression of immune factors were identified in each sample.

The protein samples were prepared using the method below.

First, the entire intestine or the distal part was removed, then a protein lysis buffer was added to dissolve the tissue. The lysate was centrifuged at 13,000 rpm, and only the supernatant was obtained and quantified to be used as an intestinal tissue sample.

The proteins separated as above were used to measure the concentrations of IL-1β (R&D systems, DY401), TNF-α (BioLegend, 430915), MCP-1 (R&D systems, DY479), IFN-γ (R&D systems, DY485), CXCL2/MIP2 (R&D systems, DY425), IL-6 (BioLegend, 431315), IL-17A (R&D Systems, DY421), and MIP1α (R&D systems, DY450).

In addition, the expression of WARS 1 was measured as below.

After coating a 96-well plate (Maxisorp, Thermo Fisher) with the WARS 1 monoclonal antibody at a concentration of 1 μg/ml, it was blocked with PBS at room temperature for 1 hour. Samples or recombinant WARS1 were each added to the coated well, reacted at room temperature for 1 hour, and then washed with PBST (PBS+Tween20) four times. The anti-WARS1 antibody (MirimGENE Co., Ltd.) was added to each well to be reacted at room temperature for 1 hour, then washed with PBST four times. After reacting with a secondary antibody (anti-rabbit IgG, HRP-conjugated antibody) conjugated with HRP, the well was reacted for 1 hour, and then washed with PBST, developed with a TMB substrate (BD), and the OD value was measured at 490 nm (VersaMax Microplate reader).

For the mRNA, Trizol (Invitrogen, 15596026) was added to the same intestinal area as the protein sample, then the RNA was extracted using a RNeasy RNA isolation kit (QIAGEN) according to the manufacturer's protocol. The RNA was used as a template to synthesize the cDNA using a cDNA EcoDry Premix (Random Hexamers) kit (Takara, 639546) and identify the expression of the immune factors through q-PCR. At this time, the primers used are shown in Table 22 below.

[Table 22]

| Gene Name | Forward primer (5' to 3') | Reverse primer (5' to 3') |
|---|---|---|
| FOXP3 | CCTGCCTTGGTACATTCGTG (SEQ ID NO: 118) | TGTTGTGGGTGAGTGCTTTG (SEQ ID NO: 119) |
| IFN-γ | ACAGCTCTCCGTCCTCGTAT (SEQ ID NO: 120) | CCTTAGAGTAGAAAGACCG (SEQ ID NO: 121) |
| IL-6 | CCTCTGGTCTTCTGGAGTAC (SEQ ID NO: 122) | GGAAATTGGGGTAGGAGG (SEQ ID NO: 123) |
| IL-10 | AGCCACATGCTCCTAGACC (SEQ ID NO: 124) | GCCTGGGGCATCACTTCTAC (SEQ ID NO: 125) |
| iNOS | GGAGCCTTTAGACCTCAACAGA (SEQ ID NO: 126) | TGAACGAGGAGGGTGGTG (SEQ ID NO: 127) |
| MCP-1 | GGGACGTTTGCTATGATGCC (SEQ ID NO: 128) | CCTACTCATTGGGATACTCTTGCT (SEQ ID NO: 129) |
| TGF-β | ACCATGCCAACTTCTGTCTG (SEQ | CGGGTTGTGTTGGTTGGTAGA |
| TNF-α | ID NO: 130 GCCTCTTCTCATTCCTGCTT (SEQ ID NO: 132) | (SEQ ID NO: 131 TGGGAACTTCTCATCCCTTTG (SEQ ID NO: 133) |
| WARS1 | CAGGATGTGTTCAATGTGCC (SEQ ID NO: 134) | TTGAAGGTGACGTGCTTCTG (SEQ ID NO: 135) |
| GAPD H | TCAACAGCAACTCCCACTCTTCCA (SEQ ID NO: 136) | ACCCTGTTGCTGTAGCCGTATTCA (SEQ ID NO: 137) |

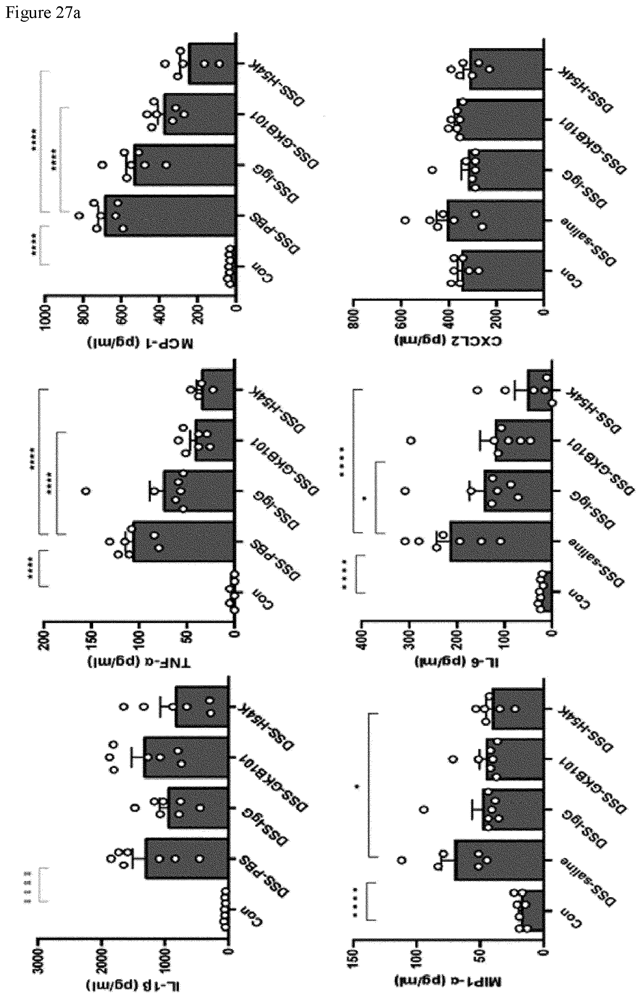

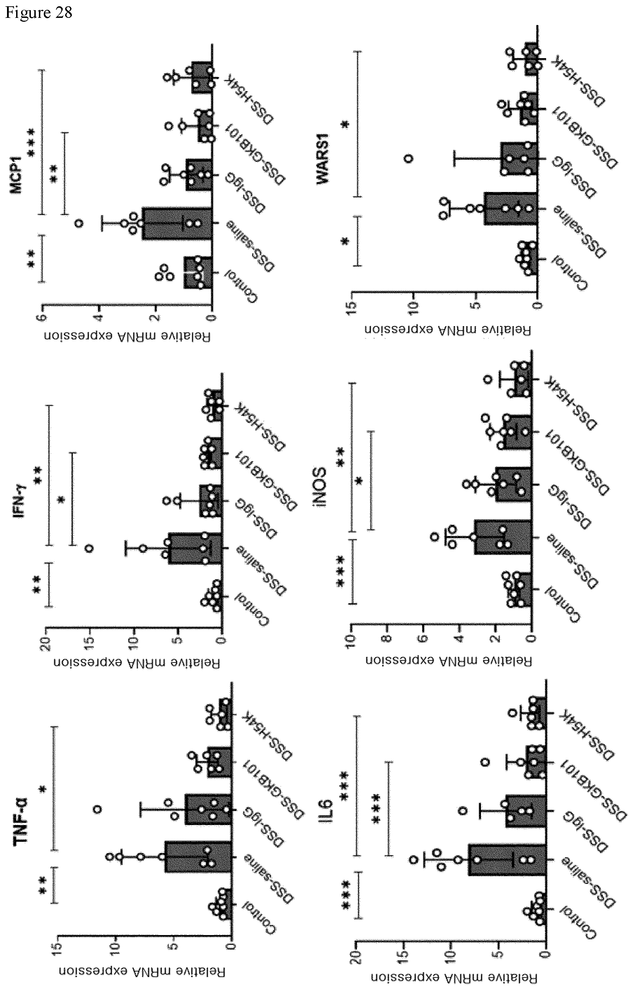

As a result, as shown in Figure 27, it was identified that the expression of protein in WARS1, TNF-α, IL-6, MIP1α, and MCP-1 had significantly decreased in the anti-WARS1 antibody (GKB101, H54K) administered group compared to the control (saline). In particular, a greater decrease was observed in the H54K antibody-administered group. In addition, the mRNA expression of the immune factors, such as TNF-α, IFN-y, MCP-1, IL-6, and iNOS, had significantly decreased in the anti-WARS1 antibody-administered group compared to the control, and in particular, the greatest decrease was identified in the H54K antibody-administered group (Figure 28). At this time, WARS1 expression had also significantly decreased in the intestinal tissue.

Through the results above, it was identified that the anti-WARS 1 antibody (GKB101, H54K) could suppress the expression of inflammatory immune factors in the intestinal tissue of an inflammatory bowel disease model, and in particular, the H54K variant antibody had better inhibitory activity compared to the GKB101 antibody.

**Example 11.4. Morphological evaluation of intestinal tissue**

Intestinal tissue was extracted from the experimental mice in Example 11.1, and H&E staining was performed in the same manner as Example 9.2.1 to observe the morphology of the intestinal tissue.

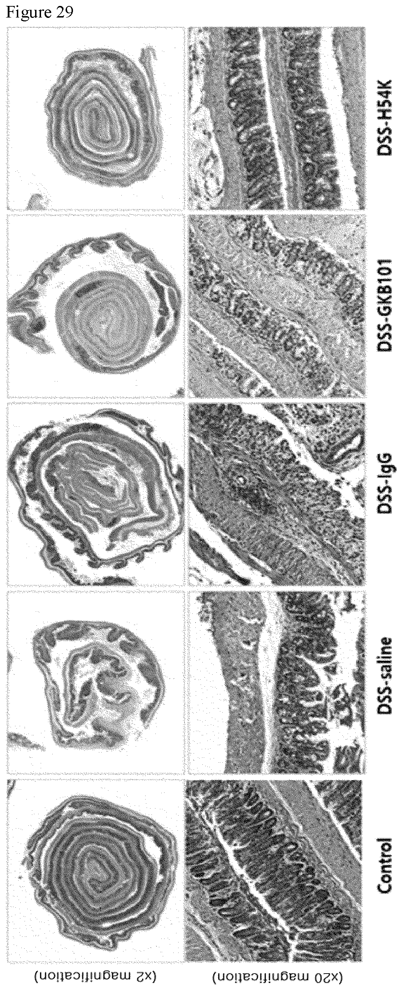

As a result, as shown in Figure 29, it was identified that damage to the intestinal tissue and the infiltration of the immune cells had decreased in the anti-WARS1 antibody-administered group compared to the control (saline). In particular, the effect of the H54K antibody-administered group was superior to that of the GKB101 antibody-administered group.

**IV. Verification of the effect of the anti-WARS1 antibody in improving rheumatoid arthritis**

**Example 12. Identification of the inflammatory activity of the anti-WARS1 antibody in a cell line related to rheumatoid arthritis**

To identify the effect of the anti-WARS1 antibody (H54K) in treating rheumatoid arthritis, the anti-inflammatory activity of the anti-WARS1 antibody was observed using cells involved in rheumatoid arthritis.

At this time, osteoclasts differentiated from human fibroblast-like synoviocyte (FLS) cell lines (cell applications, 408K-05a), J774A.1 cells, which were mouse macrophages, and RAW264.7 cells, which were mouse macrophages, were used as the cells.

Specifically, the cell lines above were each inoculated in 96-well plates at a concentration of $2 \times 10^4$ cells/well and cultured for 24 hours in a DMEM containing 0.1% FBS. At this time, the cell lines were prepared by mixing and reacting WARS1 (10 μg/ml) and the anti-WARS1 antibody (H54K, 10 μg/ml) in an incubator for 1 hour at 37 °C. The WARS 1/anti-WARS1 antibody mixture was treated in the prepared cells and cultured for an additional 16 hours. After 16 hours, the culture medium was used to measure the concentrations of CXCL2 (R&D systems, DY452), MMP3 (R&D systems, DY548), IL-6 (BioLegend, #431304), and TNF-α (BioLegend, #430904) using ELISA. For experiments with J774A.1 cells, IgG was used as the control for the anti-WARS 1 antibody. RAW264.7 cells were cultured for 5 days in a 10% FBS/DMEM medium containing 50 ng/ml of recombinant protein mouse RANKL (peprotech, 315-11) to induce the differentiation into osteoclasts. Afterwards, only the differentiated osteoclasts were collected to identify the anti-WARS1 antibody's ability of suppressing osteoclast activation under the same conditions as the WARS1 (10 μg/ml) and the anti-WARS 1 antibody (H54K, 10, 12.5, 25 μg/ml).

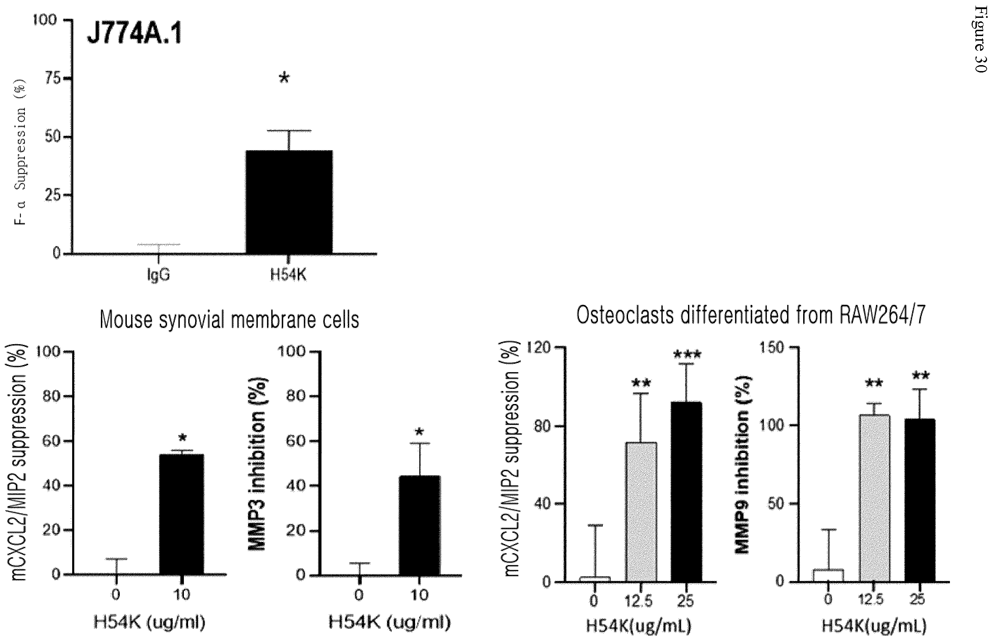

As a result, as shown in Figure 30, it was identified that inflammatory responses through cell activation by the treatment of WARS1 had decreased by anti-WARS1 antibody (H54K) treatment in osteoclasts derived from J774A.1 cells, FLS cells, and RAW264,7 cells.

**Example 13. Verification of the effect of the anti-WARS1 antibody in improving rheumatoid arthritis using a mouse model of rheumatoid arthritis**

**Example 13.1. Production of a mouse model of rheumatoid arthritis**

The effect of the anti-WARS 1 antibody (H54K) in improving inflammatory arthritis was identified using a CIA (Collagen-Induced Arthritis) mouse model, which was a rheumatoid arthritis model.

7-week-old male DBA/1J mice were used, and they were raised in an SPF laboratory animal room maintained at a constant temperature of 21 to 23 °C and constant relative humidity of 40 to 45% (IACUC No. 2021-0133).

In addition, the number of experimental animals per cage was less than six, and the cages were replaced and feed was provided two times a week. First, to prepare a CIA mouse model, the stimulant for inducing arthritis was prepared by dissolving bovine type II collagen in 10 mM acetic acid to a concentration of 2 mg/ml, then mixed with Complete Freund's adjuvant (CFA, 2 mg/ml) at a ratio of 1:1 (v/v). Primary immunization was performed by intradermally injecting 100 μl of collagen prepared using the method above into the tail of each mouse. 21 days after the primary immunization, IFA (incomplete Freund's adjuvant) and type II collagen were mixed at a ratio of 1:1 (v/v) to be emulsified, then 100 μl was intradermally injected into the tail of each mouse for secondary immunization. Mice that underwent secondary immunization were randomly divided into experimental groups for the experiment to be conducted. The experiment groups were divided into an Isotype IgG1-administere group (5 mg/kg) and an anti-WARS 1 antibody (H54K, 5 mg/kg) administered group. Administration was given intraperitoneally once every two days from the day of secondary immunization (day 21) for a total of 10 times.

**Example 13.2. Evaluation of the arthritis clinical index**

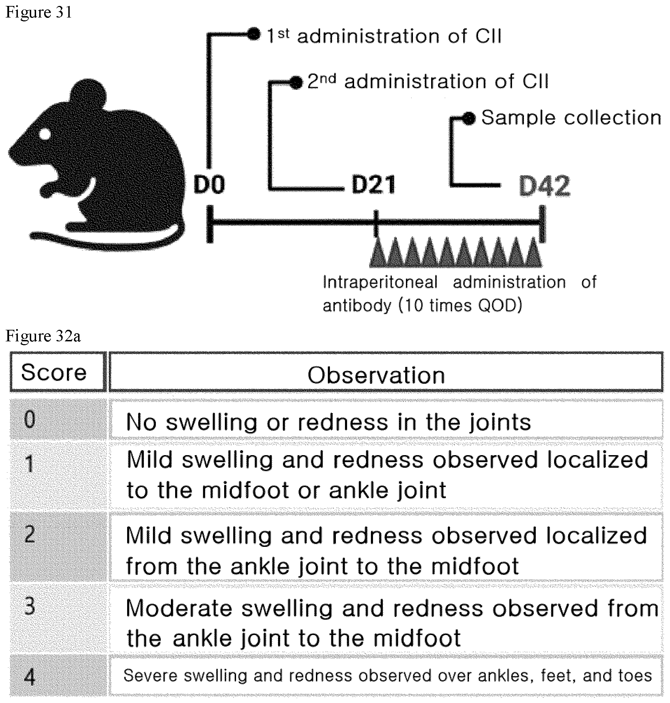

From starting the administration of the experimental substances to the experimental mice in Example 13.1, the clinical evaluation of the occurrence and severity of arthritis in the paws were evaluated according to the standards of the clinical index (Figure 32a).

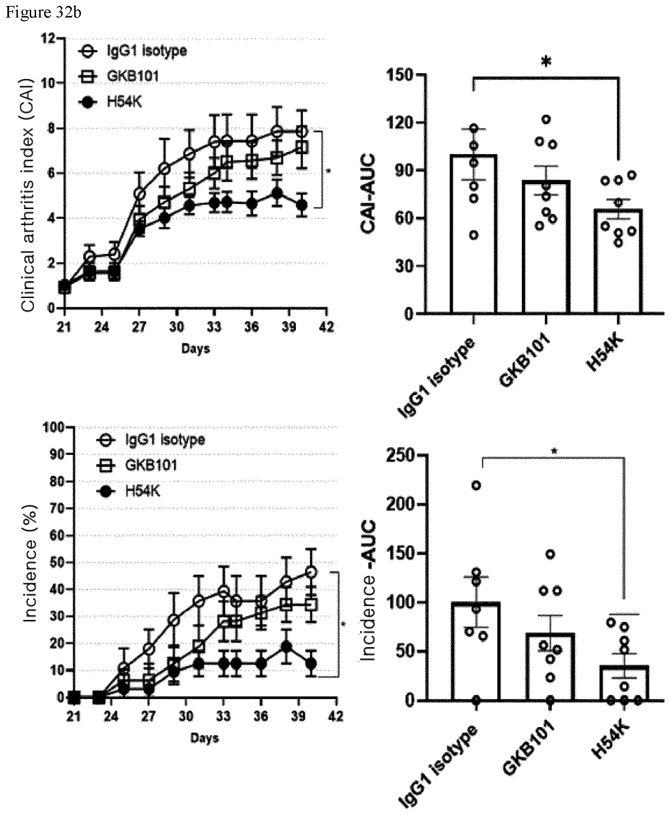

As a result, as shown in Figure 32b, it was identified that the severity of arthritis had decreased in the anti-WARS 1 antibody (GKB101, H54K) administered group compared to the Isotype IgG1-administered group, and in particular, a significant decrease was identified in the H54K antibody-administered group (IgG1-administered group vs. anti-WARS1 antibody-administered group (H54K), 7.85±0.319 vs. 4.59±0.182, *p<0.05). The result of analyzing the AUC of the clinical severity of arthritis (clinical arthritis index, CAI) also showed similar patterns with the result during the administration period.

Arthritis incidence had also decreased in the anti-WARS1 antibody (GKB101, H54K) administered group compared to the Isotype IgG1-administered group, and in particular, a significant decrease was identified in the H54K antibody-administered group (Isotype IgG1 vs. anti-WARS1 antibody (H54K), 46.42±7.945 vs. 12.5±3.689, **p<0.01). The result of analyzing the AUC of arthritis incidence also showed a significant decrease in the anti-WARS 1 antibody (H54K) administered group compared to the Isotype IgG1-administered group (Isotype IgG1 vs. anti-WARS1 antibody (H54K), 99.99±9.817 vs.35±3.931, *p<0.05).

Through the results above, it was identified that the administration of the anti-WARS 1 antibody had a therapeutic effect in the rheumatoid arthritis model, and in particular, the H54K antibody had a superior effect than the GKB101 antibody.

**Example 13.3 Analysis of inflammatory and osteoclast factors concentrations in serum and paw joints**

The concentrations of inflammatory cytokines and chemokines were measured by obtaining serum and paw joints from the experimental mice in Example 13.1.

Specifically, from the protein in the paw joints, the skin tissue in the paw joints of the mice was removed, and then a protein lysis buffer was added to dissolve the tissue. The lysate was centrifuged at 13,000 rpm, and only the supernatant was collected and quantified for use as joint tissue protein.

Using the proteins, the WARS1, and the inflammatory cytokines and chemokines in the serum and paw joints were measured using a mouse CXCL2/MIP-2 ELISA (R&D systems) kit, a mouse IL-6 ELISA (BioLegend) kit, a mouse MIP1-$\alpha$ ELISA (R&D systems) kit, MMP3 (R&D systems, DY548), IL-6 (BioLegend, #431304), TNF-$\alpha$ (BioLegend, #430904), IL-17A, IL-17F, MMP9, RANKL, and IL-1$\beta$ kits through ELISA, and the method was carried out according to the manufacturer's protocol.

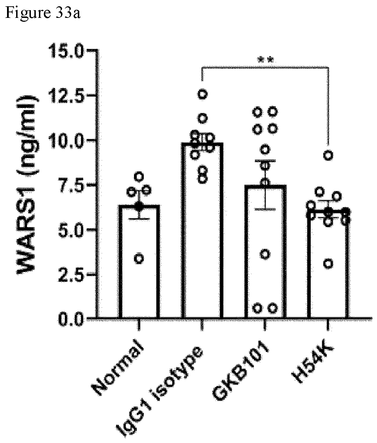

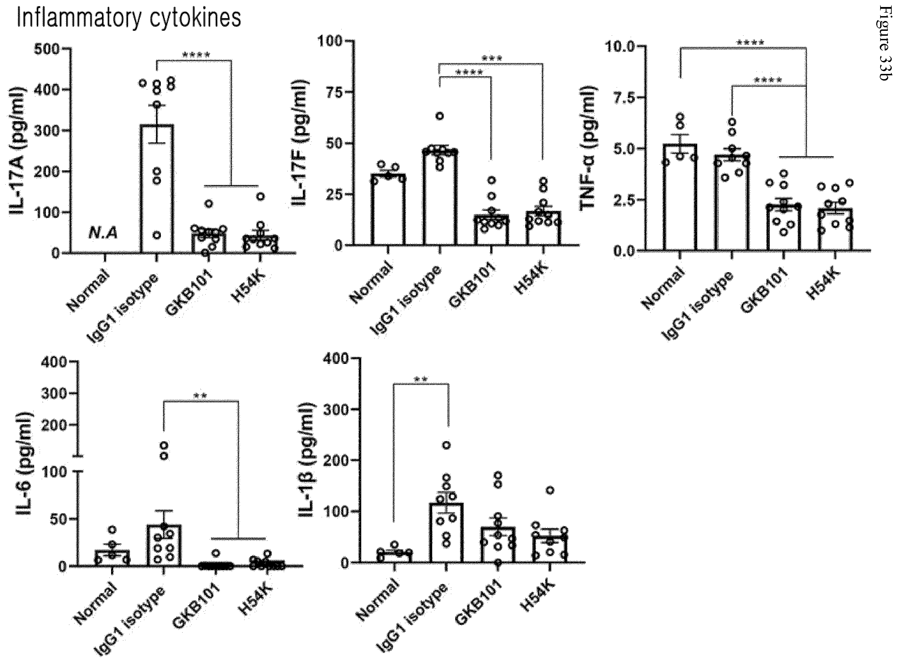

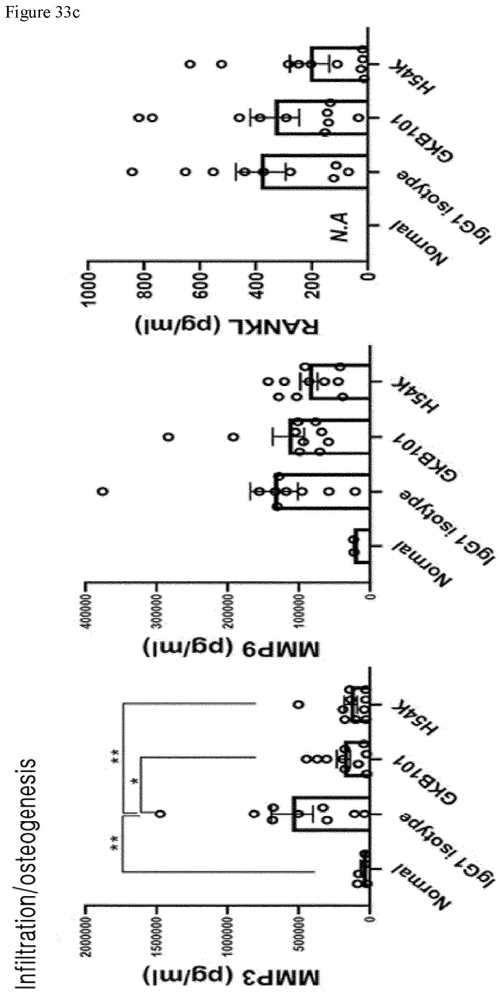

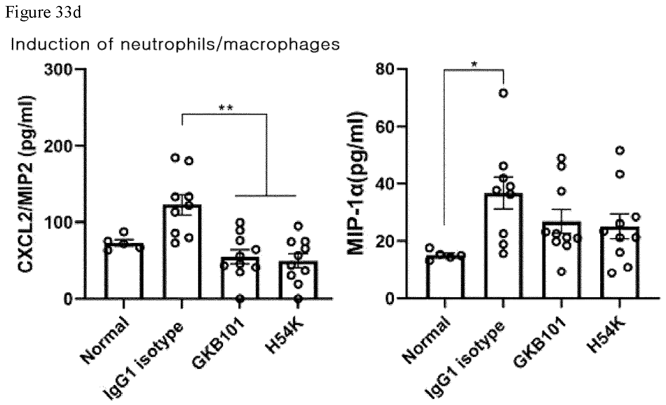

As a result, as shown in Figures 33a to 33d, it was observed that the concentrations of WARS1, IL-17A, IL-17F, TNF-$\alpha$, IL-6, MIP1$\alpha$, CXCL2/MIP2 (IL-8 homologue), IL-1$\beta$, and MMP3 had significantly decreased in the anti-WARS1 antibody (H54K) administered group compared to the Isotype IgG1-administered group (Figures 33a to 33d). At this time, all data was expressed as standard deviation (SEM) and analyzed using the ANOVA statistical program.

Through the results above, it was identified that the anti-WARS 1 antibody (GKB101, H54K) not only suppresses the expression of inflammatory cytokines and chemokines, but also suppresses the expression of chondrocyte destructive factor/osteoclast differentiation factor in a rheumatoid arthritis model.

**V. Verification of the effect of the anti-WARS1 antibody in improving dermatitis**

**Example 14. Identification of the anti-inflammatory activity of the anti-WARS1 antibody in human skin cell lines**

To identify the target mechanism of the anti-WARS1 antibody (H54K) for the anti-inflammatory activity, cell migration, and proliferation suppression in atopic dermatitis, human dermal fibroblast (adult) (HDFa, ATCC, PCS-201-012) and human keratinocytes (HaCat) were used. The cells were cultured in a DMEM containing 10% FBS and 1% antibiotic/antifungal agent.

Specifically, to identify the anti-inflammatory activity, the HDFa and HaCat cells were inoculated in a 96-well plate at $2 \times 10^4$ cells/well and cultured in a DMEM containing 0.1% FBS for 24 hours. To secrete WARS1, *Staphylococcus aureus* (Antibiotic Resistant Strain Bank, CCARM2307) was treated with a multiplicity of infection (MOI) of 10 and treated with PBS (vehicle) or 1.25 $\mu$g/ml of the anti-WARS 1 antibody (H54K), respectively. After an additional 16 hours of culture, each of the cell culture was collected and the concentrations of IL-8 (BioLegend, #431504), MCD1 (R&D systems, DY336), TARC (R&D systems, DY364), and WARS1 in the cell culture were measured using ELISA. At this time, the concentration of WARS1 in the cell culture were measured in the same manner as Example 11.3.

In addition, to identify the effect of suppressing cell migration and proliferation, HDFa and HaCat cells were inoculated in a 24-well plate at $1 \times 10^5$ cells/well and cultured in a DMEM containing 0.1% FBS for 24 hours. Afterwards, a micro-tip was used to scrape the culture bottom of the well to form an even space without cells. Also, WARS1 (20 $\mu$g/ml) and the anti-WARS1 antibody (H54K, 20 $\mu$g/ml) were mixed and reacted at 37 °C for 1 hour. The mixed solution was treated in the well and cultured for an additional 16 hours. After 16 hours, each well was observed under an optical microscope, and the migration and proliferation of cells were recorded in photographs.

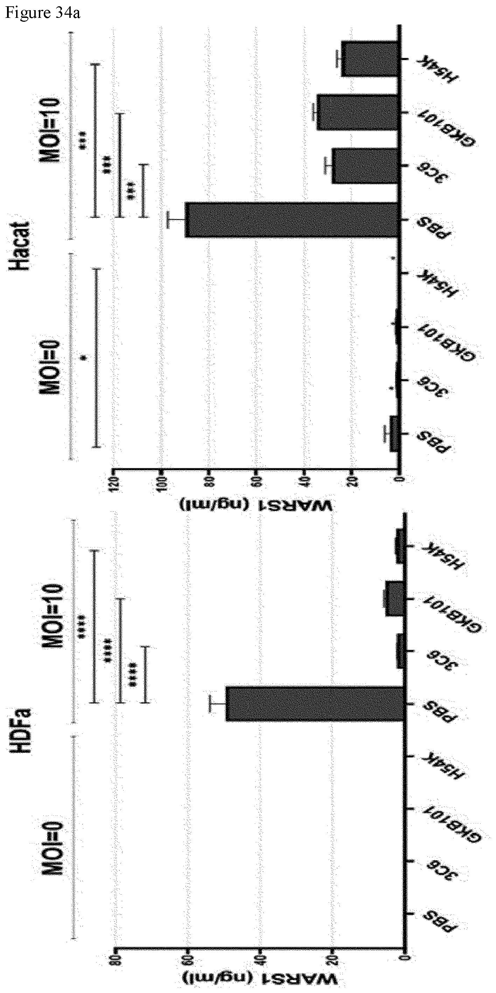

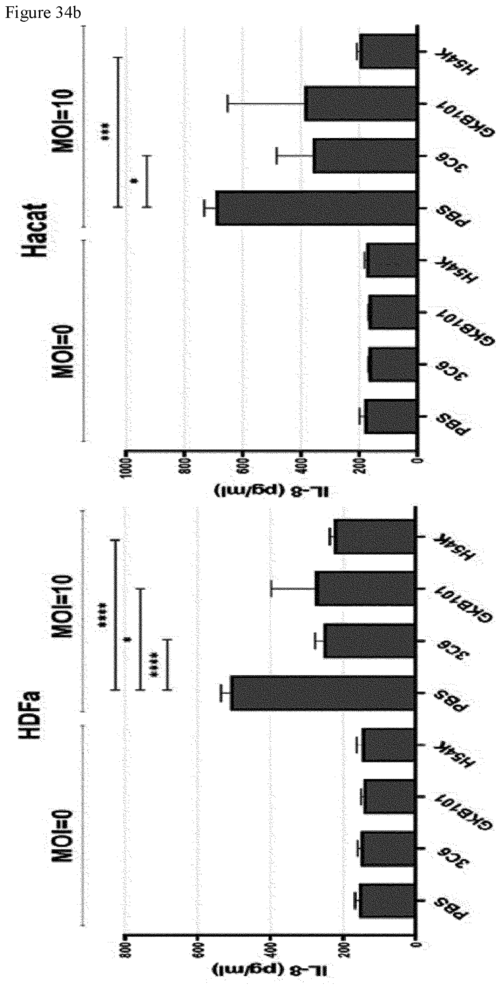

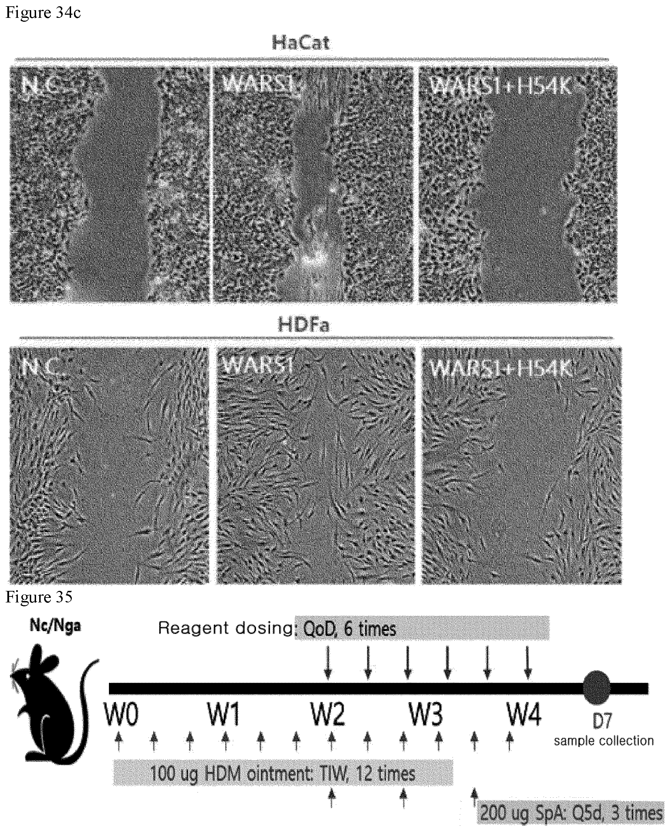

As a result, as shown in Figures 34a to 34c, it was identified that the secretion of WARS1 had increased by treating HDFa cells and HaCat cells with *Staphylococcus aureus,* whereas the secretion of WARS 1 and IL-8 had significantly decreased by treating the cells with the anti-WARS1 antibody (H54K). In addition, it was identified that the migration and proliferation of cells had also decreased by treating the cells with the anti-WARS 1 antibody.

## Example 15. Verification of the effect of the anti-WARS1 antibody in improving atopic dermatitis in a mouse model of atopy

### Example 15.1. Production of a mouse model of atopy

8-week-old female NC/Nga mice were used, and they were raised in an SPF laboratory animal room maintained at a constant temperature of 21 to 23 °C and constant relative humidity of 40 to 45%. In addition, the number of experimental animals per cage was less than three, and the cages were replaced and feed was provided once a week.

First, to produce a mouse model of atopy, one day before the start of the experiment, hair was removed as much as possible up to the upper back of the NC/Nga mice and a 4% SDS aqueous solution was sprayed on the area to be applied to remove fat components from the skin. After completely drying for about 1 hour, 100 mg of Biostir AD (atopic dermatitis-inducing reagent, house dust mite) ointment was evenly applied to the shaved area using a flat stick. The Biostir AD ointment was applied three times a week over 4 weeks (a total of 12 times). 200 μg of SpA (*Staphylococcus* protein A, Sigma Aldrich) was applied to the dorsal area per animal for a total of three times in five-day intervals starting from day 14. The anti-WARS 1 antibody (H54K) (10 mg/kg) or Isotype IgG1 (10 mg/kg) were administered intraperitoneally three times a week, six times in total. At this time, Isotype IgG1 was used as the control, and dexamethasone was used as the positive control. Dexamethasone was administered intraperitoneally at a concentration of 10 mg/kg, three times a week for a total of six times (Figure 35).

### Example 15.2. Evaluation of the clinical index

To identify the effect of the anti-WARS1 antibody (H54K) in improving atopic dermatitis, the expression of atopic dermatitis was clinical evaluated by administering Isotype IgG1, dexamethasone, or the anti-WARS1 antibody (H54K) in a mouse model of atopy, and observing skin erythema/bleeding, wounds/dryness, edema, exfoliation, and the like.

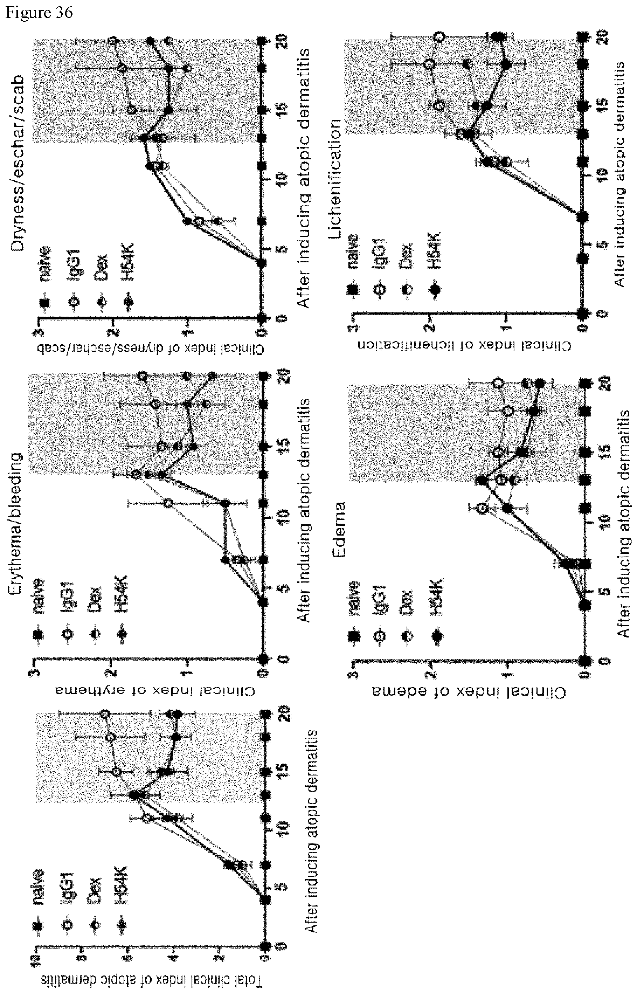

As a result, as shown in Figure 36, the dermatitis score in the Isotype IgG1-administered group had significantly increased in the four indices compared to the normal group (naive, group in which dermatitis was not induced). On the other hand, the dermatitis score had significantly decreased in the anti-WARS1 antibody-administered group compared to the Isotype IgG1-administered group.

## VI. Verification of the effect of the anti-WARS1 antibody in improving macrophage activation syndrome

## Example 16. Identification of the anti-inflammatory activation of the anti-WARS1 antibody in a macrophage activation syndrome model using CpG

### Example 16.1. Production of a macrophage activation syndrome model using CpG

A mouse model of CpG (CpG oligodeoxynucleotides (ODN)-induced macrophage activation syndrome model) was used as a macrophage activation syndrome mouse model. 8-10-week-old female B57BL/6 mice were used, and they were raised in an SPF laboratory animal room maintained at a constant temperature of 21 to 23 °C and constant relative humidity of 40 to 45%. In addition, the number of experimental animals per cage was less than five, and the cages were replaced and feed was provided once a week.

CpG ODN (InvivoGen, Class B, tlrl-1826) was diluted in PBS at a concentration of 45 μg/200 μl and stored at -80 °C, then intraperitoneally administered once a day in an amount of 200 μl for each mouse for a total of five times (days 0, 2, 4, 6, and 8). 1 day after the final administration (on day 9), blood was collected from the heart of anesthetized mice, and then the liver and spleen were collected to identify the level of inflammatory indices and cell activity. The condition of the mice was identified once a day until the end of the experiment.

The test group was divided into six groups in total, and 2 hours after administering CpG on each day of CpG administration, PBS, which was the negative control, Isotype IgG (10 mg/kg, Bio X cell. BP0297), the anti-WARS1 antibody [(H54K, 5, 10, 15 mg/kg) or GKB101 (10 mg/kg)] was intraperitoneally administered in accordance with each concentration. All data was expressed as standard deviation (SEM) and analyzed using the ANOVA statistical program (Figure 38).

### Example 16.2. Analysis of the WARS1 and inflammatory cytokine expression in liver tissue

A portion of the liver tissue of the mice in Example 16.1 was collected and the liver tissue was dissolved using the gentleMACS™ Octo Dissociator (Miltenyi Biotec) and Trizol (Takara, 9109, 1 ml). After separating RNA and synthesizing cDNAin the dissolved liver tissue in the same manner as Example 11.3, the primers in Tables 22 and 23 were used to identify the gene expression through q-PCR.

[Table 23]

| Gene Name | Forward primer (5' to 3') | Reverse primer (5' to 3') |
|---|---|---|
| IFN-γ | GAACTGGCAAAAGGATGGTGA (SEQ ID NO: 138) | TGTGGGTTGTTGACCTCAAAC (SEQ ID NO: 139) |
| IL-6 | TACCACTTCACAAGTCGGAGGC (SEQ ID NO: 140) | CTGCAAGTGCATCGTTGTTC (SEQ ID NO: 141) |
| TNF-α | GGTGCCTATGTCTCAGCCTCTT (SEQ ID NO: 142) | GCCATAGAACTGATGAGAGGGAG (SEQ ID NO: 143) |
| CXCL9 | ATGAAGTCCGCTGTTCTTTTCC (SEQ ID NO: 144) | GTCTCTTATGTAGTCTTCCTTG (SEQ ID NO: 145) |

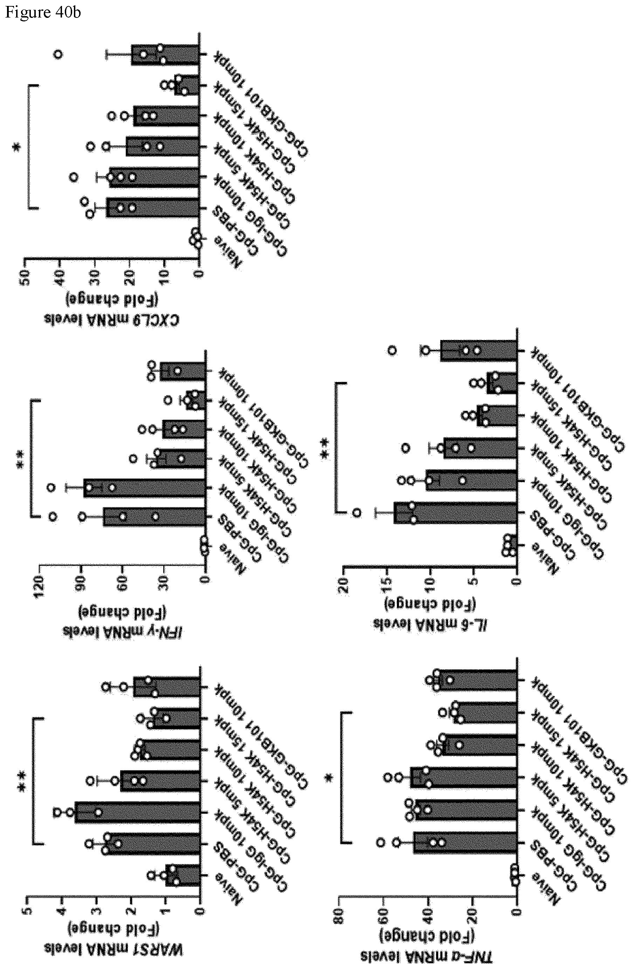

As a result, as shown in Figure 40b, the expression of WARS1 and inflammatory cytokines and chemokines had decreased in the anti-WARS1 antibody (GKB101, H54K) administered group compared to the PBS-administered group or the Isotype IgG-administered group. In particular, in the H54K-administered group, the expression of WARS1 and inflammatory cytokines and chemokines had decreased in a concentration-dependent manner. To identify the effect of the anti-WARS 1 antibody (GKB101, H54K) in decreasing WARS 1 in liver tissue in a CpG mouse model, a portion of the liver tissue of the mice was collected and a protein lysis buffer was added, and then the liver tissue was inserted into M tubes (Miltenyi Biotec, 130-093-236) and homogenized using gentleMACS™ Octo Dissociation (Miltenyi Biotec). The process afterwards was performed in the same manner as Example 11.3 to measure WARS1.

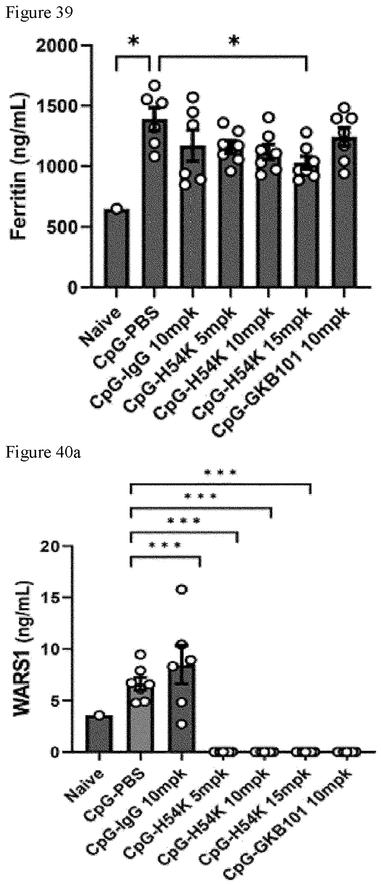

As a result, as shown in Figure 40a, a decrease in the expression of WARS 1 in liver tissue was identified in the anti-WARS1 antibody (GKB101, H54K) administered group compared to the PBS-administered group or the Isotype IgG1-administered group. In particular, the expression of WARS1 had decreased in the H54K antibody-administered group in a concentration-dependent manner.

## Example 16.3. Analysis of ferritin in blood

To identify the effect of the anti-WARS1 antibody (GKB101, H54K) in decreasing ferritin in plasma in a CpG mouse model, blood was collected from the heart of the mice, then transferred to a heparin tube and centrifuged at 2,000 rpm for 20 minutes at room temperature to prepare the plasma. Ferritin was measured using an ELISA kit (ferritin kit, Abcam, ab 157713) according to the manufacturer's protocol.

As a result, as shown in Figure 39, it was identified that the ferritin level in serum had significantly decreased in the H54K antibody-administered group (15 mg/kg) compared to the PBS-administered group or the Isotype IgG1-administered group.

## Example 16.4. Analysis of immune cell distribution in the spleen

To identify changes in the distribution (%) of T cells and natural killer cells (NK cells) by the anti-WARS 1 antibody in a CpG mouse model, the spleen of the mice was removed and passed through a 70 μm cell strainer to obtain single cells. Immunofluorescence staining was performed on the cells. The antibodies used at this time are shown in Table 24 below.

[Table 24]

| Antibody | Manufacturer | Cat No. |
|---|---|---|
| BD Pharmingen™ APC Rat Anti-Mouse CD45 | BD Pharmingen | 559864 |
| BD Pharmingen™ FITC Mouse Anti-Mouse NK-1.1 | BD Pharmingen | 553164 |
| BD Horizon™ V450 Rat anti-CD11b | BD Pharmingen | 560455 |
| BD Pharmingen™ PE Hamster Anti-Mouse CD27 | BD Pharmingen | 558754 |
| BD Pharmingen™ PE Hamster Anti-Mouse CD3e | BD Pharmingen | 553064 |
| .BD Pharmingen™ FITC Rat Anti-Mouse CD8 | BD Pharmingen | 561966 |

(continued)

| Antibody | Manufacturer | Cat No. |
|---|---|---|
| BD Pharmingen™ PE anti-mouse IFN gamma | BD Pharmingen | 554412 |

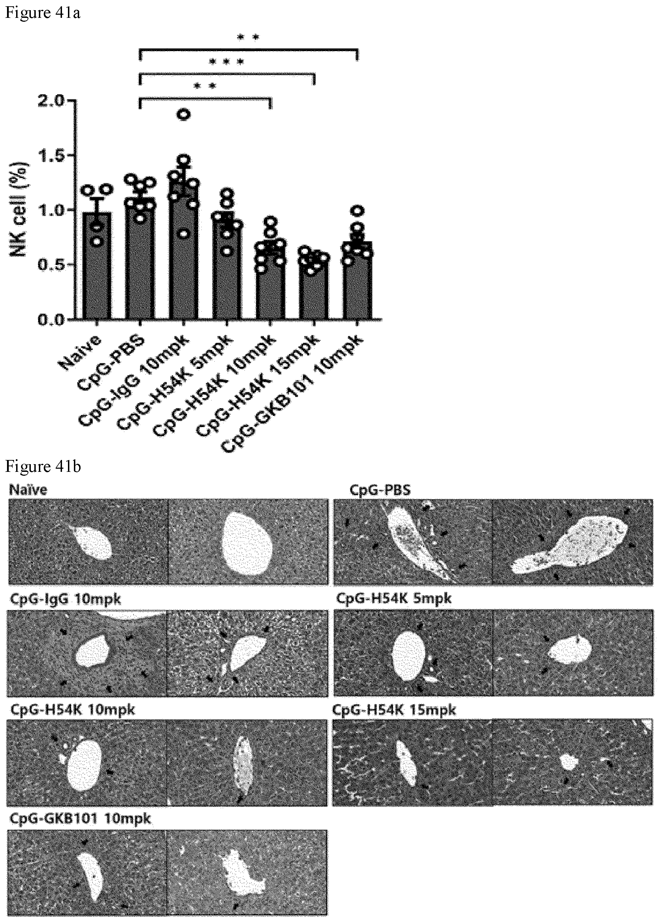

The antibodies above excluding the anti-mouse IFN-y antibody were each treated in a corresponding cell sample tube and reacted for 20 minutes, then washed with a FACS buffer. To identify the distribution of the activated Th1 cells, the corresponding tube sample was fixed/permeabilized using a fix/perm buffer (Invitrogen, 00-5523-00). The sample tube was reacted with a PE anti-mouse IFN-y antibody, then washed with a FACS buffer and flow cytometry was performed. The analysis values were CD45+CD11b+CD27-NK1.1+(actiated NK cells), CD45+CD3+CD8+IFNy+(activated Th1 cells) as analyzed in the FlowJo program (FlowJo™ v7 Software, BD BioSciences), and each immune cell was classified and the distribution (%) of each cell was analyzed. As a result, as shown in Figure 41a, the distribution (%) of the NK cells had increased in the PBS-administered group or the Isotype IgG1-administered group compared to the normal group. On the other hand, in the anti-WARS1 antibody (GKB101, H54K) administered group, the distribution of the NK cells had decreased in a concentration-dependent manner compared to the PBS-administered group or the Isotype IgG1-administered group. In particular, the distribution (%) of the NK cells had decreased in the H54K antibody-administered group in a concentration-dependent manner.

**Example 17. Analysis of immune cell infiltration in liver tissue**

To identify the effect of the anti-WARS 1 antibody (GKB101, H54K) on the infiltration of immune cells in liver tissue in a CpG mouse model, one lobe of the excised mouse liver was extracted and fixed in 10% formalin solution (Sigma, HT501128) for about 1 day. H&E staining was performed on the fixed tissue in the same manner as Example 11.1 for observation.

As a result, as shown in Figure 41b, a decrease in the infiltration of the immune cells was identified in the liver tissue in the anti-WARS1 antibody (H54K, GKB101) administered group compared to the PBS-administered group or the Isotype IgG1-administered group.

**Claims**

1. An antibody-WARS 1 antibody or a fragment thereof, comprising:

   a heavy chain variable region comprising H-CDR1 comprising the amino acid sequence of SEQ ID NO: 3, H-CDR2 comprising the amino acid sequence of SEQ ID NO: 5, and H-CDR3 comprising the amino acid sequence of SEQ ID NO: 7; and
   a light chain variable region comprising L-CDR1 comprising the amino acid sequence of SEQ ID NO: 12, L-CDR2 comprising the amino acid sequence of SEQ ID NO: 95 below, and L-CDR3 comprising the amino acid sequence of SEQ ID NO: 96 below:

   N terminus-Ala Asn Xaa1 Xaa2 His Arg Pro Ser-C terminus (SEQ ID NO: 95),
   wherein Xaa1 is Ala, Val, Ile, Leu, Met, Phe, Tyr, Trp, Lys, Arg, His, Asp, Ser, Thr, Glu, Asn, Gln, Cys, Gly, or Pro,
   Xaa2 is Ala, Val, Ile, Leu, Met, Phe, Tyr, Trp, Lys, Arg, His, Asp, Ser, Thr, Glu, Asn, Gln, Cys, Gly, or Pro,
   wherein when Xaa1 is Ser, Xaa2 is not His,
   N terminus-Gly Ala Trp Asp Asp Ser Xaa3 Ser Ala Tyr Val-C terminus (SEQ ID NO: 96),
   wherein Xaa3 is Ala, Val, Ile, Leu, Met, Phe, Tyr, Trp, Lys, Arg, His, Asp, Ser, Thr, Glu, Asn, Gln, Cys, Gly, or Pro.

2. The anti-WARS1 antibody or a fragment thereof according to claim 1, wherein the antibody or a fragment thereof specifically binds to WARS1.

3. The anti-WARS1 antibody or a fragment thereof according to claim 1, wherein the antibody or a fragment thereof specifically binds to WARS1.

4. The anti-WARS1 antibody or a fragment thereof according to claim 1, wherein the Xaa3 is Leu or Asn.

**EP 4 516 812 A1**

5. The anti-WARS1 antibody or a fragment thereof according to claim 1, wherein the light chain variable region consisting of: L-CDR1 comprising the amino acid sequence of SEQ ID NO: 12; L-CDR2 comprising an amino acid sequence of any one selected from the group consisting of SEQ ID NOs: 20, 22, 24, 26, 28, 30, 32, 34, 36, 38, 40, 42, and 44; L-CDR3 comprising the amino acid sequence of SEQ ID NO: 16 or SEQ ID NO: 46.

6. The anti-WARS1 antibody or a fragment thereof according to claim 1, wherein the heavy chain variable region comprises the amino acid sequence of SEQ ID NO: 9.

7. The anti-WARS1 antibody or a fragment thereof according to claim 1, wherein the light chain variable region comprises an amino acid sequence of any one selected from the group consisting of SEQ ID NOs: 21, 23, 25, 27, 29, 31, 33, 35, 37, 39, 41, 43, 45, 47, 48, and 49.

8. The anti-WARS1 antibody or a fragment thereof according to claim 1, wherein the antibody comprises a Fc region.

9. A polynucleotide encoding the anti-WARS 1 antibody or a fragment thereof according to any one of claims 1 to 8.

10. An expression vector comprising the polynucleotide of claim 9.

11. A transformed cell into which the expression vector of claim 10 has been introduced.

12. A method for producing an anti-WARS 1 antibody or a fragment thereof comprising:

    culturing the transformed cell of claim 11; and
    obtaining an anti-WARS 1 antibody or a fragment thereof.

13. A pharmaceutical composition for use in preventing or treating an inflammatory disease comprising the anti-WARS1 antibody or a fragment thereof according to any one of claims 1 to 8 as an active ingredient.

14. The pharmaceutical composition for use in preventing or treating an inflammatory disease according to claim 13, wherein the inflammatory disease is any one selected from the group consisting of: peritonitis, osteomyelitis, cellulitis, meningitis, encephalitis, pancreatitis, trauma-induced shock, cystic fibrosis, stroke, Lyme disease, polyarteritis nodosa, hypersensitivity angiitis, Lou Gehrig's granulomatosis, articular cell arteritis, bursitis, tenosynovitis, epicondylitis (Tennis elbow), Henoch-Schönlein purpura, multicentric reticulohistiocytoma, surcoilosis, hemochromatosis, sickle cell disease and other hemoglobinopathies, hyperlipoproteinemia, hypogammaglobulinemia, familial Mediterranean fever, relapsing fever, sepsis, septic shock, multi-organ dysfunction syndrome, broncho-pulmonary dysplasia, inflammatory bowel disease, chronic obstructive pulmonary disease (COPD), macrophage activation syndrome (MAS), chronic systemic lupus erythematosus, scleroderma, atopic dermatitis, psoriasis, anaphylaxis, dermatitis, diabetic retinopathy, retinitis, macular degeneration, uveitis, conjunctivitis, arthritis, rheumatoid arthritis, ankylosing spondylitis, osteoarthritis, osteoporosis, allergy, diabetes, diabetic nephropathy, pyelonephritis, nephritis, Sjögren's syndrome, autoimmune pancreatitis, periodontal disease, asthma, graft-versus-host disease, chronic pelvic inflammatory disease, endometritis, rhinitis, transplant rejection, and chronic prostatitis.

15. Use of the antibody or a fragment thereof of claim 1 for preventing or treating an inflammatory disease.

16. A method for preventing or treating an inflammatory disease comprising administering the antibody or a fragment thereof of claim 1 to a subject.

Figure 1

pUC origin

Heavy chain vector
pOptiVec-GKB101
5833bp

$Amp^R$

$C_H$

CMV
Promotor

$V_H$

Figure 2

pUC origin

Light chain vector
pcDNA3.3-GKB101
6226bp

$Amp^R$

$Neo^R/Kan^R$

CMV
Promotor

$V_L$

$C_L$

Figure 3a

Figure 3b

Figure 4a

Figure 4b

Pig WARS1

Rat WARS1

Figure 5

Figure 6a

Figure 6b

Figure 6c

Figure 7

Figure 8

Figure 9a

```
                  10          20          30          40          50          60          70
WT      MPNSEPASLL  ELFNSIATQG  ELVRSLKAGN  ASKDEIDSAV  KMLVSLKMSY  KAAAGEDYKA  DCPPGNPAPT  (SEQ ID NO: 115)
S08A    · · · · · · · A · ·   · · · · · · · · · ·   · · · · · · · · · ·   · · · · · · · · · ·   · · · · · · · · · ·   · · · · · · · · · ·   · · · · · · · · · ·
L09A    · · · · · · · · A ·   · · · · · · · · · ·   · · · · · · · · · ·   · · · · · · · · · ·   · · · · · · · · · ·   · · · · · · · · · ·   · · · · · · · · · ·
L10A    · · · · · · · · · A   · · · · · · · · · ·   · · · · · · · · · ·   · · · · · · · · · ·   · · · · · · · · · ·   · · · · · · · · · ·   · · · · · · · · · ·
E11A    · · · · · · · · · ·   A · · · · · · · · ·   · · · · · · · · · ·   · · · · · · · · · ·   · · · · · · · · · ·   · · · · · · · · · ·   · · · · · · · · · ·
L12A    · · · · · · · · · ·   · A · · · · · · · ·   · · · · · · · · · ·   · · · · · · · · · ·   · · · · · · · · · ·   · · · · · · · · · ·   · · · · · · · · · ·
F13A    · · · · · · · · · ·   · · A · · · · · · ·   · · · · · · · · · ·   · · · · · · · · · ·   · · · · · · · · · ·   · · · · · · · · · ·   · · · · · · · · · ·
N14A    · · · · · · · · · ·   · · · A · · · · · ·   · · · · · · · · · ·   · · · · · · · · · ·   · · · · · · · · · ·   · · · · · · · · · ·   · · · · · · · · · ·
S15A    · · · · · · · · · ·   · · · · A · · · · ·   · · · · · · · · · ·   · · · · · · · · · ·   · · · · · · · · · ·   · · · · · · · · · ·   · · · · · · · · · ·
I16A    · · · · · · · · · ·   · · · · · A · · · ·   · · · · · · · · · ·   · · · · · · · · · ·   · · · · · · · · · ·   · · · · · · · · · ·   · · · · · · · · · ·
T18A    · · · · · · · · · ·   · · · · · · · A · ·   · · · · · · · · · ·   · · · · · · · · · ·   · · · · · · · · · ·   · · · · · · · · · ·   · · · · · · · · · ·
Q19A    · · · · · · · · · ·   · · · · · · · · A ·   · · · · · · · · · ·   · · · · · · · · · ·   · · · · · · · · · ·   · · · · · · · · · ·   · · · · · · · · · ·
R20A    · · · · · · · · · ·   · · · · · · · · · A   · · · · · · · · · ·   · · · · · · · · · ·   · · · · · · · · · ·   · · · · · · · · · ·   · · · · · · · · · ·
E21A    · · · · · · · · · ·   · · · · · · · · · ·   A · · · · · · · · ·   · · · · · · · · · ·   · · · · · · · · · ·   · · · · · · · · · ·   · · · · · · · · · ·
L22A    · · · · · · · · · ·   · · · · · · · · · ·   · A · · · · · · · ·   · · · · · · · · · ·   · · · · · · · · · ·   · · · · · · · · · ·   · · · · · · · · · ·
V23A    · · · · · · · · · ·   · · · · · · · · · ·   · · A · · · · · · ·   · · · · · · · · · ·   · · · · · · · · · ·   · · · · · · · · · ·   · · · · · · · · · ·
R24A    · · · · · · · · · ·   · · · · · · · · · ·   · · · A · · · · · ·   · · · · · · · · · ·   · · · · · · · · · ·   · · · · · · · · · ·   · · · · · · · · · ·
S25A    · · · · · · · · · ·   · · · · · · · · · ·   · · · · A · · · · ·   · · · · · · · · · ·   · · · · · · · · · ·   · · · · · · · · · ·   · · · · · · · · · ·
L26A    · · · · · · · · · ·   · · · · · · · · · ·   · · · · · A · · · ·   · · · · · · · · · ·   · · · · · · · · · ·   · · · · · · · · · ·   · · · · · · · · · ·
K27A    · · · · · · · · · ·   · · · · · · · · · ·   · · · · · · A · · ·   · · · · · · · · · ·   · · · · · · · · · ·   · · · · · · · · · ·   · · · · · · · · · ·
K33A    · · · · · · · · · ·   · · · · · · · · · ·   · · · · · · · · · ·   · · · · · · · · · ·   · · · · · · · · · ·   · · · · · · · · · ·   · · · · · · · · · ·
D34A    · · · · · · · · · ·   · · · · · · · · · ·   · · · · · · · · · ·   · · A · · · · · · ·   · · · · · · · · · ·   · · · · · · · · · ·   · · · · · · · · · ·
E35A    · · · · · · · · · ·   · · · · · · · · · ·   · · · · · · · · · ·   · · · A · · · · · ·   · · · · · · · · · ·   · · · · · · · · · ·   · · · · · · · · · ·
I36A    · · · · · · · · · ·   · · · · · · · · · ·   · · · · · · · · · ·   · · · · A · · · · ·   · · · · · · · · · ·   · · · · · · · · · ·   · · · · · · · · · ·
D37A    · · · · · · · · · ·   · · · · · · · · · ·   · · · · · · · · · ·   · · · · · A · · · ·   · · · · · · · · · ·   · · · · · · · · · ·   · · · · · · · · · ·
S38A    · · · · · · · · · ·   · · · · · · · · · ·   · · · · · · · · · ·   · · · · · · A · · ·   · · · · · · · · · ·   · · · · · · · · · ·   · · · · · · · · · ·
V40A    · · · · · · · · · ·   · · · · · · · · · ·   · · · · · · · · · ·   · · · · · · · · A   · · · · · · · · · ·   · · · · · · · · · ·   · · · · · · · · · ·
K41A    · · · · · · · · · ·   · · · · · · · · · ·   · · · · · · · · · ·   · · · · · · · · · ·   A · · · · · · · · ·   · · · · · · · · · ·   · · · · · · · · · ·
M42A    · · · · · · · · · ·   · · · · · · · · · ·   · · · · · · · · · ·   · · · · · · · · · ·   · A · · · · · · · ·   · · · · · · · · · ·   · · · · · · · · · ·
I43A    · · · · · · · · · ·   · · · · · · · · · ·   · · · · · · · · · ·   · · · · · · · · · ·   · · A · · · · · · ·   · · · · · · · · · ·   · · · · · · · · · ·
V44A    · · · · · · · · · ·   · · · · · · · · · ·   · · · · · · · · · ·   · · · · · · · · · ·   · · · A · · · · · ·   · · · · · · · · · ·   · · · · · · · · · ·
S45A    · · · · · · · · · ·   · · · · · · · · · ·   · · · · · · · · · ·   · · · · · · · · · ·   · · · · A · · · · ·   · · · · · · · · · ·   · · · · · · · · · ·
I46A    · · · · · · · · · ·   · · · · · · · · · ·   · · · · · · · · · ·   · · · · · · · · · ·   · · · · · A · · · ·   · · · · · · · · · ·   · · · · · · · · · ·
K47A    · · · · · · · · · ·   · · · · · · · · · ·   · · · · · · · · · ·   · · · · · · · · · ·   · · · · · · A · · ·   · · · · · · · · · ·   · · · · · · · · · ·
K51A    · · · · · · · · · ·   · · · · · · · · · ·   · · · · · · · · · ·   · · · · · · · · · ·   · · · · · · · · · ·   A · · · · · · · · ·   · · · · · · · · · ·
```

Figure 9b

Figure 9c

Figure 10

Figure 11

▼ Cecal slurry administration (*i.p.*)

▽ Isotype IgG 10 mpk administration (*i.p.*)

▼ Anti−WARS1 antibody administration (*i.p.*)

(2.5 mpk or 5 mpk or 10 mpk of each)

Figure 12

Figure 13

**pOptiVec Topo (Thermo fisher Cat # 12744017)**

```
AAAGTGCCACCTGACGTCGACGGATCGGGAGATCAGTTGACATTGATTATTGACTAGTTATTAATAGTAA
TCAATTACGGGGTCATTAGTTCATAGCCCATATATGGAGTTCCGCGTTACATAACTTACGGTAAATGGCC
CGCCTGGCTGACCGCCCAACGACCCCCGCCCATTGACGTCAATAATGACGTATGTTCCCATAGTAACGCC
AATAGGGACTTTCCATTGACGTCAATGGGTGGAGTATTTACGGTAAACTGCCCACTTGGCAGTACATCAA
GTGTATCATATGCCAAGTACGCCCCCTATTGACGTCAATGACGGTAAATGGCCCGCCTGGCATTATGCCC
AGTACATGACCTTATGGGACTTTCCTACTTGGCAGTACATCTACGTATTAGTCATCGCTATTACCATGGT
GATGCGGTTTTGGCAGTACATCAATGGGCGTGGATAGCGGTTTGACTCACGGGGATTTCCAAGTCTCCAC
CCCATTGACGTCAATGGGAGTTTGTTTTGGCACCAAAATCAACGGGACTTTCCAAAATGTCGTAACAACT
CCGCCCCATTGACGCAAATGGGCGGTAGGCGTGTACGGTGGGAGGTCTATATAAGCAGAGCTCGTTTAGT
GAACCGTCAGATCGCCTGGAGACGCCATCCACGCTGTTTTGACCTCCATAGAAGACACCGGGACCGATCC
AGCCTCCGGACTCTAGAGGATCCAACCCTTAAGGGTTGGATCCCTACCGGTGCTGCGGCGCGCAGTTAA
CGCCGCCCCTCTCCCTCCCCCCCCCCTAACGTTACTGGCCGAAGCCGCTTGGAATAAGGCCGGTGTGCGT
TTGTCTATATGTTATTTTCCACCATATTGCCGTCTTTTGGCAATGTGAGGGCCCGGAAACCTGGCCCTGT
CTTCTTGACGAGCATTCCTAGGGGTCTTTCCCCTCTCGCCAAAGGAATGCAAGGTCTGTTGAATGTCGTG
AAGGAAGCAGTTCCTCTGGAAGCTTCTTGAAGACAAACAACGTCTGTAGCGACCCTTTGCAGGCAGCGGA
ACCCCCCACCTGGCGACAGGTGCCTCTGCGGCCAAAAGCCACGTGTATAAGATACACCTGCAAAGGCGGC
ACAACCCCAGTGCCACGTTGTGAGTTGGATAGTTGTGGAAAGAGTCAAATGGCTCTCCTCAAGCGTATTC
AACAAGGGGCTGAAGGATGCCCAGAAGGTACCCCATTGTATGGGATCTGATCTGGGGCCTCGGTACACAT
GCTTTACATGTGTTTAGTCGAGGTTAAAAAAACGTCTAGGCCCCCCGAACCACGGGGACGTGGTTTTCCT
TTGAAAAACACGATGATAATATGGCCACAAGATCTGCCACCATGGTTCGACCATTGAACTGCATCGTCGC
CGTGTCCCAAAATATGGGGATTGGCAAGAACGGAGACCTACCCTGGCCTCCGCTCAGGAACGAGTTCAAG
TACTTCCAAAGAATGACCACAACCTCTTCAGTGGAAGGTAAACAGAATCTGGTGATTATGGGTAGGAAAA
CCTGGTTCTCCATTCCTGAGAAGAATCGACCTTTAAAGGACAGAATTAATATAGTTCTCAGTAGAGAACT
CAAAGAACCACCACGAGGAGCTCATTTTCTTGCCAAAAGTTTGGATGATGCCTTAAGACTTATTGAACAA
CCGGAATTGGCAAGTAAAGTAGACATGGTTTGGATAGTCGGAGGCAGTTCTGTTTACCAGGAAGCCATGA
ATCAACCAGGCCACCTCAGACTCTTTGTGACAAGGATCATGCAGGAATTTGAAAGTGACACGTTTTTCCC
AGAAATTGATTTGGGGAAATATAAACTTCTCCCAGAATACCCAGGCGTCCTCTCTGAGGTCAGGAGGAA
AAAGGCATCAAGTATAAGTTTGAAGTCTACGAAGAAGAAAGACTAAAACCGGTTAGTAATGAGTTTAAACG
GGGGAGGCTAACTGAAACACGGAAGGAGACAATACCGGAAGGAACCCGCGCTATGACGGCAATAAAAAGA
CAGAATAAAACGCACGGGTGTTGGGTCGTTTGTTCATAAACGCGGGGTTCGGTCCCAGGGCTGGCACTCT
GTCGATACCCCACCGAGACCCCATTGGGGCCAATACGCCCGCGTTTCTTCCTTTTCCCCACCCCACCCCC
CAAGTTCGGGTGAAGGCCCAGGGCTCGCAGCCAACGTCGGGGCGGCAGGCCCTGCCATTACCGTCGACCT
CTAGCTAGAGCTTGGCGTAATCATGGTCATAGCTGTTTCCTGTGTGAAATTGTTATCCGCTCACAATTCC
ACACAACATACGAGCCGGAAGCATAAAGTGTAAAGCCTGGGGTGCCTAATGAGTGAGCTAACTCACATTA
ATTGCGTTGCGCTCACTGCCCGCTTTCCAGTCGGGAAACCTGTCGTGCCAGCTGCATTAATGAATCGGCC
AACGCGCGGGGAGAGGCGGTTTGCGTATTGGGCGCTCTTCCGCTTCCTCGCTCACTGACTCGCTGCGCTC
GGTCGTTCGGCTGCGGCGAGCGGTATCAGCTCACTCAAAGGCGGTAATACGGTTATCCACAGAATCAGGG
GATAACGCAGGAAAGAACATGTGAGCAAAAGGCCAGCAAAAGGCCAGGAACCGTAAAAAGGCCGCGTTGC
TGGCGTTTTTCCATAGGCTCCGCCCCCCTGACGAGCATCACAAAAATCGACGCTCAAGTCAGAGGTGGCG
AAACCCGACAGGACTATAAAGATACCAGGCGTTTCCCCCTGGAAGCTCCCTCGTGCGCTCTCCTGTTCCG
ACCCTGCCGCTTACCGGATACCTGTCGGCCTTTCTCCCTTCGGGAAGCGTGGCGCTTTCTCATAGCTCAC
GCTGTAGGTATCTCAGTTCGGTGTAGGTCGTTCGCTCCAAGCTGGGCTGTGTGCACGAACCCCCCGTTCA
GCCCGACCGCTGCGCCTTATCCGGTAACTATCGTCTTGAGTCCAACCCGGTAAGACACGACTTATCGCCA
CTGGCAGCAGCCACTGGTAACAGGATTAGCAGAGCGAGGTATGTAGGCGGTGCTACAGAGTTCTTGAAGT
GGTGGCCTAACTACGGCTACACTAGAAGAACAGTATTTGGTATCTGCGCTCTGCTGAAGCCAGTTACCTT
CGGAAAAAGAGTTGGTAGCTCTTGATCCGGCAAACAAACCACCGCTGGTAGCGGTTTTTTTGTTTGCAAG
CAGCAGATTACGCGCAGAAAAAAAGGATCTCAAGAAGATCCTTTGATCTTTTCTACGGGGTCTGACGCTC
AGTGGAACGAAAACTCACGTTAAGGGATTTTGGTCATGAGATTATCAAAAAGGATCTTCACCTAGATCCT
TTTAAATTAAAAATGAAGTTTTAAATCAATCTAAAGTATATATGAGTAAACTTGGTCTGACAGTTACCAA
TGCTTAATCAGTGAGGCACCTATCTCAGCGATCTGTCTATTTCGTTCATCCATAGTTGCCTGACTCCCCG
TCGTGTAGATAACTACGATACGGGAGGGCTTACCATCTGGCCCCAGTGCTGCAATGATACCGCGAGACCC
ACGCTCACCGGCTCCAGATTTATCAGCAATAAACCAGCCAGCCGGAAGGGCCGAGCGCAGAAGTGGTCCT
GCAACTTTATCCGCCTCCATCCAGTCTATTAATTGTTGCCGGGAAGCTAGAGTAAGTAGTTCGCCAGTTA
ATAGTTTGCGCAACGTTGTTGCCATTGCTACAGGCATCGTGGTGTCACGCTCGTCGTTTGGTATGGCTTC
ATTCAGCTCCGGTTCCCAACGATCAAGGCGAGTTACATGATCCCCCATGTTGTGCAAAAAAGCGGTTAGC
TCCTTCGGTCCTCCGATCGTTGTCAGAAGTAAGTTGGCCGCAGTGTTATCACTCATGGTTATGGCAGCAC
TGCATAATTCTCTTACTGTCATGCCATCCGTAAGATGCTTTTCTGTGACTGGTGAGTACTCAACCAAGTC
ATTCTGAGAATAGTGTATGCGGCGACCGAGTTGCTCTTGCCCGGCGTCAATACGGGATAATACCGCGCCA
CATAGCAGAACTTTAAAAGTGCTCATCATTGGAAAACGTTCTTCGGGGCGAAAACTCTCAAGGATCTTAC
CGCTGTTGAGATCCAGTTCGATGTAACCCACTCGTGCACCCAACTGATCTTCAGCATCTTTTACTTTCAC
CAGCGTTTCTGGGTGAGCAAAAACAGGAAGGCAAAATGCCGCAAAAAAGGGAATAAGGGCGACACGGAAA
TGTTGAATACTCATACTCTTCCTTTTTCAATATTATTGAAGCATTTATCAGGGTTATTGTCTCATGAGCG

GATACATATTTGAATGTATTTAGAAAAATAAACAAATAGGGGTTCCGCGCACATTTCCCCGA
```

(SEQ ID NO: 114)

Figure 14

Initial human lung fibroblasts

Figure 15

Figure 16a

H&E staining/M–T staining for identifying immune cell infiltration and collagen deposition in lung tissue

Figure 16b

PAS staining for identifying mucus secretion in the airway

Figure 17a

Figure 17b

Figure 17c

Figure 18a

EP 4 516 812 A1

Figure 18b

Figure 19

Figure 20

Figure 21

**C57BL/6**

Reagent dosing: QoD, 3 times

D0    D1    D2    D3    D4    D6    D7

Sample collection

2.5% DSS

Figure 22a

Standard of determining the clinical index

| Score | Weight loss | Stool consistency | Rectal bleeding |
|-------|-------------|-------------------|-----------------|
| 0 | None | Normal | Negative hemoccult |
| 1 | 1-5 % | soft but shape kept | Positive occult blood test (Weak luminol intensity) |
| 2 | 6-10 % | soft | Positive occult blood test (Strong luminol intensity) |
| 3 | 11-18 % | Very soft, wet | Blood traces in stool visible |
| 4 | > 18% | Watery diarrhea | Gross rectal bleeding |

Figure 22b

Figure 23

Figure 24

Figure 25

Figure 26

Figure 27a

Figure 28

Figure 29

Figure 30

Figure 31

Figure 32a

| Score | Observation |
|-------|-------------|
| 0 | No swelling or redness in the joints |
| 1 | Mild swelling and redness observed localized to the midfoot or ankle joint |
| 2 | Mild swelling and redness observed localized from the ankle joint to the midfoot |
| 3 | Moderate swelling and redness observed from the ankle joint to the midfoot |
| 4 | Severe swelling and redness observed over ankles, feet, and toes |

Figure 32b

Figure 33a

Figure 33b

Figure 33c

Figure 33d

Induction of neutrophils/macrophages

Figure 34a

Figure 34b

Figure 34c

HaCat

HDFa

Figure 35

Figure 36

Figure 37

Figure 38

Figure 39

Figure 40a

Figure 40b

Figure 41a

Figure 41b

<table>
<tr><td colspan="2"><strong>INTERNATIONAL SEARCH REPORT</strong></td><td>International application No.<br><br><strong>PCT/KR2023/005828</strong></td></tr>
</table>

**A.    CLASSIFICATION OF SUBJECT MATTER**

**C07K 16/40**(2006.01)i; **A61P 29/00**(2006.01)i; **A61K 39/00**(2006.01)i

According to International Patent Classification (IPC) or to both national classification and IPC

**B.    FIELDS SEARCHED**

Minimum documentation searched (classification system followed by classification symbols)

C07K 16/40(2006.01); A23L 1/305(2006.01); A61K 38/17(2006.01); A61K 38/53(2006.01); A61K 39/00(2006.01); A61P 29/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 트립토파닐 티알엔에이 합성효소(tryptophanyl-tRNA synthetase 1, WARS1), 염증성 질환(inflammatory disease)

**C.    DOCUMENTS CONSIDERED TO BE RELEVANT**

| Category* | Citation of document, with indication, where appropriate, of the relevant passages | Relevant to claim No. |
|---|---|---|
| A | KR 10-2021-0010236 A (MEDICINAL BIOCONVERGENCE RESEARCH CENTER) 27 January 2021 (2021-01-27)<br>        See claims 1-14; and paragraph [0040]. | 1-15 |
| A | KR 10-1899591 B1 (MEDICINAL BIOCONVERGENCE RESEARCH CENTER et al.) 17 September 2018 (2018-09-17)<br>        See entire document. | 1-15 |
| A | KR 10-1729547 B1 (THE INDUSTRY & ACADEMIC COOPERATION IN CHUNGNAM NATIONAL UNIVERSITY (IAC)) 25 April 2017 (2017-04-25)<br>        See entire document. | 1-15 |
| A | JIN, M. Unique roles of tryptophanyl-tRNA synthetase in immune control and its therapeutic implications. Experimental & Molecular Medicine. vol. 51, no. 1, pp. 1-10, 2019.<br>        See entire document. | 1-15 |

☑ Further documents are listed in the continuation of Box C.          ☑ See patent family annex.

| | |
|---|---|
| *    Special categories of cited documents:<br>"A"    document defining the general state of the art which is not considered to be of particular relevance<br>"D"    document cited by the applicant in the international application<br>"E"    earlier application or patent but published on or after the international filing date<br>"L"    document which may throw doubts on priority claim(s) or which is cited to establish the publication date of another citation or other special reason (as specified)<br>"O"    document referring to an oral disclosure, use, exhibition or other means<br>"P"    document published prior to the international filing date but later than the priority date claimed | "T"    later document published after the international filing date or priority date and not in conflict with the application but cited to understand the principle or theory underlying the invention<br>"X"    document of particular relevance; the claimed invention cannot be considered novel or cannot be considered to involve an inventive step when the document is taken alone<br>"Y"    document of particular relevance; the claimed invention cannot be considered to involve an inventive step when the document is combined with one or more other such documents, such combination being obvious to a person skilled in the art<br>"&"    document member of the same patent family |

| Date of the actual completion of the international search | Date of mailing of the international search report |
|---|---|
| **07 August 2023** | **07 August 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.

**PCT/KR2023/005828**

**C.  DOCUMENTS CONSIDERED TO BE RELEVANT**

| Category* | Citation of document, with indication, where appropriate, of the relevant passages | Relevant to claim No. |
|---|---|---|
| A | YU, C. Y. et al. Aminoacyl-tRNA synthetases and amino acid signaling. BBA-Molecular Cell Research. vol. 1868, no. 1: 118889, pp. 1-11, 2021.<br>See entire document. | 1-15 |

Form PCT/ISA/210 (second sheet) (July 2022)

**INTERNATIONAL SEARCH REPORT**

International application No.

**PCT/KR2023/005828**

Box No. I          Nucleotide and/or amino acid sequence(s) (Continuation of item 1.c of the first sheet)

1.  With regard to any nucleotide and/or amino acid sequence disclosed in the international application, the international search was carried out on the basis of a sequence listing:

    a.  ☑ forming part of the international application as filed.

    b.  ☐ furnished subsequent to the international filing date for the purposes of international search (Rule 13*ter*.1(a)),

        ☐ accompanied by a statement to the effect that the sequence listing does not go beyond the disclosure in the international application as filed.

2.  ☐ With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

**INTERNATIONAL SEARCH REPORT**

International application No.

**PCT/KR2023/005828**

| Box No. II | Observations where certain claims were found unsearchable (Continuation of item 2 of first sheet) |
|---|---|

This international search report has not been established in respect of certain claims under Article 17(2)(a) for the following reasons:

1. ☑ Claims Nos.: **16**
   because they relate to subject matter not required to be searched by this Authority, namely:

   Claim 16 pertains to a method for treatment of the human body, and thus pertains to a subject matter on which the International Searching Authority is not required to carry out an international search under the provisions of PCT Article 17(2)(a)(i) and PCT Rule 39.1(iv).

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

**INTERNATIONAL SEARCH REPORT**
Information on patent family members

International application No.

**PCT/KR2023/005828**

| Patent document cited in search report | | | Publication date (day/month/year) | Patent family member(s) | | | Publication date (day/month/year) |
|---|---|---|---|---|---|---|---|
| KR | 10-2021-0010236 | A | 27 January 2021 | None | | | |
| KR | 10-1899591 | B1 | 17 September 2018 | EP | 3292872 | A1 | 14 March 2018 |
| | | | | EP | 3292872 | B1 | 07 June 2023 |
| | | | | KR | 10-2016-0104587 | A | 05 September 2016 |
| | | | | US | 11207389 | B2 | 28 December 2021 |
| | | | | US | 2018-0064790 | A1 | 08 March 2018 |
| | | | | US | 2021-0205423 | A1 | 08 July 2021 |
| | | | | WO | 2016-137280 | A1 | 01 September 2016 |
| KR | 10-1729547 | B1 | 25 April 2017 | None | | | |

Form PCT/ISA/210 (patent family annex) (July 2022)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **YOUNG HA AHN et al.** *Nature Microbiology*, 2016, vol. 2, 16191
- **ENGELS** ; **UHLMANN**. *Angew Chem IntEd Engl.*, 1988, vol. 37, 73-127
- **GILLIES et al.** *J. Immunol. Meth*, 1989, vol. 125, 191-202
- **SAKANO et al.** *Nature*, 1980, vol. 286, 676-683
- **WATSON et al.** *Nucleic Acid Research*, 1984, vol. 12, 5145-5164
- Remington's Pharmaceutical Sciences. 1995